# EUROPEAN PATENT APPLICATION

(11) **EP 3 783 104 A1**
(43) Date of publication of application: **24.02.2021**
(21) Application number: 19192490.1
(22) Date of filing: 20.08.2019
(51) Int. Cl.: C12N 15/10, C12N 15/62, C12N 9/22, C12N 9/78, C12N 15/85, C12N 5/10, A61K 31/70, A61K 48/00

(54) **COILED-COIL MEDIATED TETHERING OF CRISPR-CAS AND EXONUCLEASES FOR ENHANCED GENOME EDITING**

(71) Applicant: Kemijski Institut, 1000 Ljubljana (SI)
(72) Inventor: JERALA, Roman, 1000 Ljubljana (SI); LAINSCEK, Dusko, 1000 Ljubljana (SI)
(74) Representative: Weickmann & Weickmann PartmbB

(57) **Abstract**

The invention provides a method for enhanced genome engineering using CRISPR/Cas system tethered to exonucleases via different systems. By connecting Cas9 and exonucleases via heterodimeric peptides that form coiled-coils or via heterodimerization systems, greater indel mutations at desired genomic region occurs, resulting in higher genome editing rate. This novel improved CRISPR/Cas system can be exploited in different cell origins and organisms in various fields, where genome engineering is required and desired.

## Description

### FIELD OF THE INVENTION

Heterodimerization of Cas9 protein, part of CRISPR/Cas system, with exonucleases via peptides that form coiled-coils increases the rate of occurrence of indel mutations in genome region of interests in cells resulting in higher degree of genome engineering that is exploited in all areas where genome editing is required.

### BACKGROUND OF THE INVENTION

The CRISPR (Clustered Regularly Interspaced Short Palindromic Repeats)/Cas (CRISPR-associated) system that is used as a tool for genome editing and derives from bacteria and archaea as a part of their adaptive immune system to fight infectious viruses. When prokaryotes are invaded with viruses, short viral DNA segments are integrated into CRISPR locus, which is the key for CRISPR immunity. Upon second accounter with viruses, RNA that includes viral sequence, from CRISPR locus is transcribed. That RNA is complimentary to viral DNA and mediates targeting of Cas9 protein to the sequence in genome. This specific targeting results in DNA cleavage and thereby silencing the viral target,

This specific targeting and DNA cleavage at desired genome region made CRISPR/Cas system as a potent novel tool for genome editing. Cas9 protein or its variants are guided by gRNA (guide RNA) to its complimentary specific site or region of interest (ROI) within the genome. The only necessity for Cas9 recognition is short PAM (protospacer adjacent motif) sequence presence within the genome. Depending on the Cas9 origin, different PAM sequences are recognized. After specific Cas9 targeting to ROI site-specific double strand breaks (DSBs) occur, which vary in length and in frequency. Repair of DSBs and their manipulation lead to knockout or knockin of gene of interests (GOI).

DSBs are then repaired with cell repair mechanisms, mostly with error-prone classical-Non-homologous end joining (c-NHEJ), which is DNA resection independent. Other repair mechanisms, like alt-EJ (alternative end joining), HR (homologous recombination), SSA (single-strand annealing) work in DNA resection dependent manner. C-NHEJ mainly introduces few nucleotides long insertion-deletion (indel) mutations that can lead to functional gene inactivation by either frameshift or deletions resulting in certain percentage or degree of knockout of GOI. C-NHEJ repairs Cas9 mediated blunt-end DSB via simple blunt end sequence-homology independent ligation process. Alternatively, DSBs can be resected, leaving 3' or 5' single-stranded DNA (ssDNA) overhangs. When DSBs via HR are repaired a certain DNA template as a source for repair is needed. Usually sister chromatid get strand invaded in typically error-free process, resulting in site-specific integration. This is often exploited for knockin production where included exogenous DNA reacts as a template. ssDNA overhangs can be also repaired via mutagenic repair mechanism, namely SSA, where annealing at large homologies occurs, resulting in larger deletions with no insertions. Alt-EJ uses annealing at microhomologies, resulting in mutagenic indel mutations.

Depending on the size and frequency of indel mutations, higher rates of knockout can be achieved. Greater genome rearrangements occurs when additional DNA resection after DSBs are carried out. DNA resection and subsequent DNA repair is used for elevated genome editing using CRISPR/Cas regarding the efficiency. Coexpression of certain exonucleases (e.g. human EX01, TREX2) with CRISPR/Cas system can increase the size of mutations resulting in higher frequency of knockin or knockout of GOI.

### SUMMARY OF THE INVENTION

The present invention provides an implementation for CRISPR/Cas system for greater efficiency regarding genome modification and editing based on tethering of Cas9 protein with exonucleases via different heterodimerization systems. Tethering of CRISPR/Cas system and exonucleases result in higher percentage or degree of genome modification for GOI or recisse DNA to the extent to allow higher homologies for greater percentage of knockin.

This disclosure provides new system of improved genome editing by the use of CRISPR/Cas system. Genome editing herein encompasses DNA cleavage and sequential repair. DSBs are formed by protein Cas9 or its variants or Cas9 nickase (HNH-mutant nickase or RuvC-mutant nickase) that are guided via one or two gRNA to specific genome sites. This system for enhanced genome editing can be used in living mammalian and non-mammalian (e.g. plant) cells.

Heterodimerization of CRISPR/Cas system and certain exonucleases is achieved by the use of heterodimerizing peptides that form coiled-coils (CC) that are expressed with Cas9 protein at its C- or N- terminus and with certain exonucleases at its C- or N-terminus thus Cas9 gets tethered with certain exonucleases. Tethering of Cas9 with certain exonucleases brings exonucleases into close proximity of DSB to influence DNA recession. Collocation of certain exonuclease at DSB improves DNA recession thus making longer 3' and/or 5' DNA overhangs. Longer DNA overhangs generates greater non-homologies between upper and lower DNA strands thus making larger indel mutations after DSB repairs at higher degree. Heterodimerization of Cas9 protein or its variants or Cas9 nickase and certain exonucleases is achieved due to the dimer forming peptides. Heterodimerizing peptides express coiled coil structural motif, a combination of hydrophobic and electrostatic interactions between amino acid residues within heptads of alpha-helices.

This disclosure presents tethering of CRISPR/Cas system and certain exonucleases via additional heterodimerization systems. System is based on the selection of heterodimerization (HD) domains (different protein-protein partners; HD system), which allows regulated expression in cells upon presence of one or more inductors that can provide external regulator signals. Inductors for heterodimerization can be light (e.g. Blue light, red light etc.) or chemical nature depending on the use of protein-protein partner pair. Such chemical inductors can be small molecules, such as rapalog, gibberellin and/or abscisic acid.

Protein partners of the HD system dimerize into a functional form when the appropriate heterodimerizing signal is present. One partner of the HD system is connected to a Cas9 protein or its variants or Cas9 nickase. The other partner of the selected heterodimerization system is coupled with certain exonuclease. By the addition of an external light at the appropriate wavelength or small chemical inductor or regulator, respectively, (for example a rapalog, gibberellin or abscisic acid) that acts to promote heterodimerization, two partners of an HD system dimerize into a heterodimer. Heterodimerization of two protein partners of HD systems allows close proximity of certain exonuclease at the point of DSB, which is CRISPR/Cas system mediated. Juxtaposition of certain exonuclease at DSB enhances DNA recession thus making longer 3' and/or 5' DNA overhangs. Longer DNA overhangs generates greater non-homologies between upper and lower DNA strands thus making larger indel mutations after DSB repairs at higher degree.

The invention provides modified CRSIPR/Cas system based on the use of HD with certain exonucleases. Enhanced genome editing includes knockout and knockin for desired GOI at determined gene locus in mammalian and non-mammalian cells.

### Figure legends

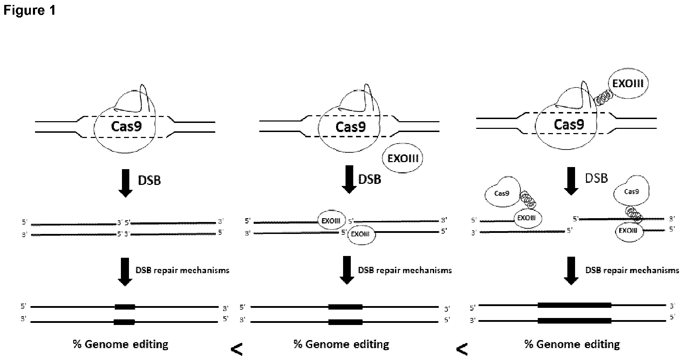

**Figure** 1: A schematic diagram of action of the method for enhanced genome engineering using CRISPR/Cas system according to the invention, tethered to exonucleases via heterodimeric peptides that form coiled-coils. A) By using simple CRISPR/Cas system that consists out of Cas9 protein and gRNA DSB are formed, which are then repaired via cell repair mechanisms, resulting in certain degree or percentage of genome editing. B) By using cooexpresion of simple CRISPR/Cas system and certain exonuclease (e.g. EXOIII) DSBs are formed. The exposed DNA strands are then additionally recised via certain exonucleases, creating DNA overhangs in 5' or 3' manner. Again, DSBs with established DNA overhangs are repaired via cell repair mechanisms, resulting in higher degree or percentage of genome editing compared to simple CRISPR/Cas system. C) By using CRISPR/Cas system tethered to certain exonuclease (e.g. EXOIII) via heterodimeric peptides that form coiled-coils DSBs are formed. The exposed DNA strands are then additionally recised via certain exonucleases, creating even larger DNA overhangs compared to overhangs, created in system, where CRISPR/Cas and exonucleases are coexpressed. Again, DSBs with established DNA overhangs are repaired via cell repair mechanisms, resulting in even higher degree or percentage of genome editing compared to simple CRISPR/Cas system or system, where CRISPR/Cas and exonucleases are coexpressed.

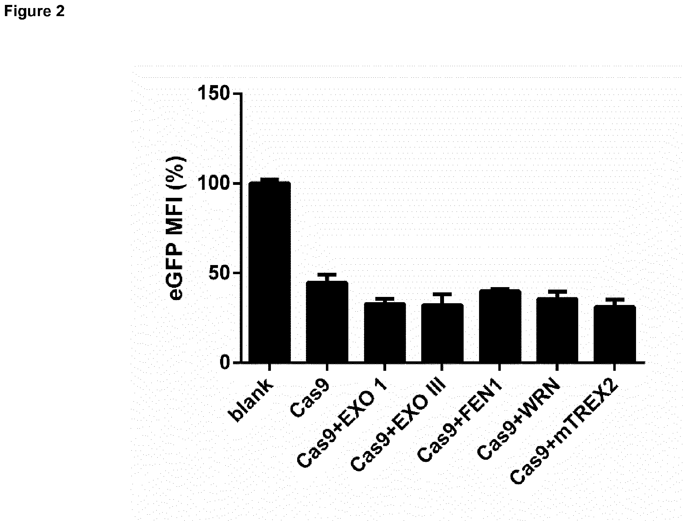

**Figure 2****:** Genome editing in eGFP genome region. By coexpression of Cas9 and gRNA, directed towards eGFP genomic region eGFP genome region is modified. Greater genome editing occurred when Cas9, gRNA were coeexpressed with exonucleases, wherein EX01, EXOIII, FEN1, WRN or mTREX2 is at least one of the exonuclease used. eGFP genome editing resulted in eGFP fluorescence decrease.

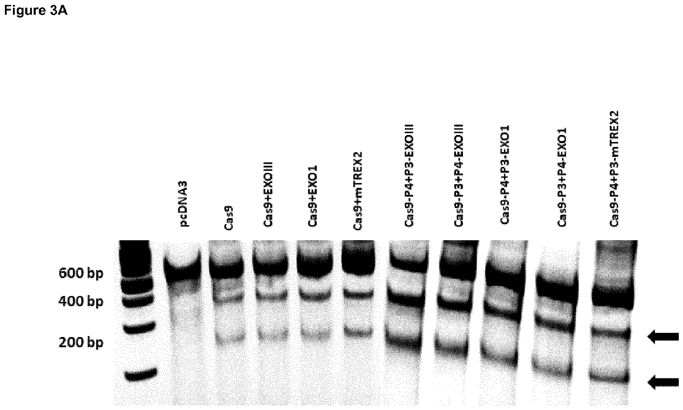

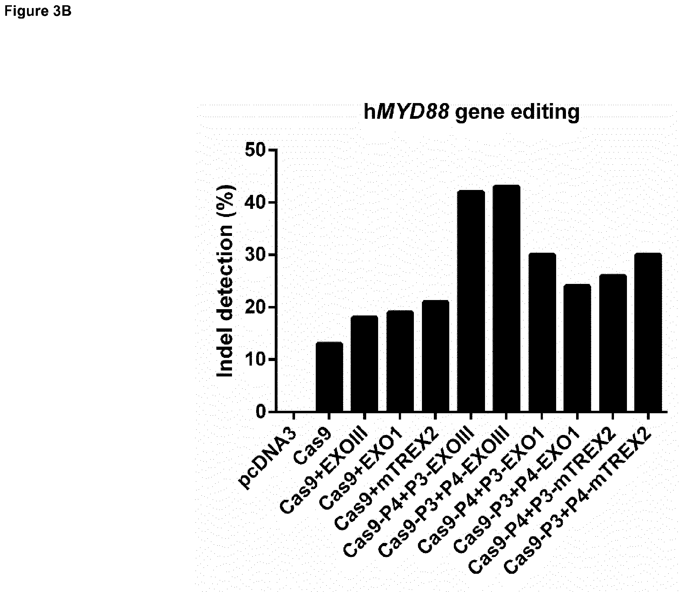

**Figure 3****:** Genome editing in human *MYD88* genome region. By coexpression of Cas9 and gRNA, directed towards human *MYD88* genomic region human *MYD88* genome region is modified. Greater genome editing occurred when Cas9, gRNA were coeexpressed with exonucleases, wherein EX01, EXOIII, or mTREX2 is at least one of the exonuclease used. Bigger genome editing occurred when CRISPR/Cas system tethered to certain exonucleases via heterodimeric coiled-coil forming peptides were used, wherein Cas9-P3 or Cas9-P4 are one of the Cas9 variants and P3-EX01, P4-EX01, P3-EXOIII, P4-EXOIII, P3-mTREX2 or P4-mTREX2 are one of the variants of expressed exonucleases. A) Human *MYD88* genome editing resulted in indel mutations, detected via T7E1 assay. B) Indel detection (%) of *MYD88* genome region engineering.

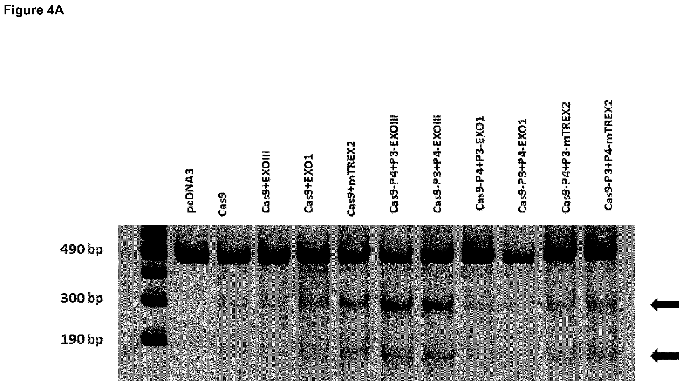

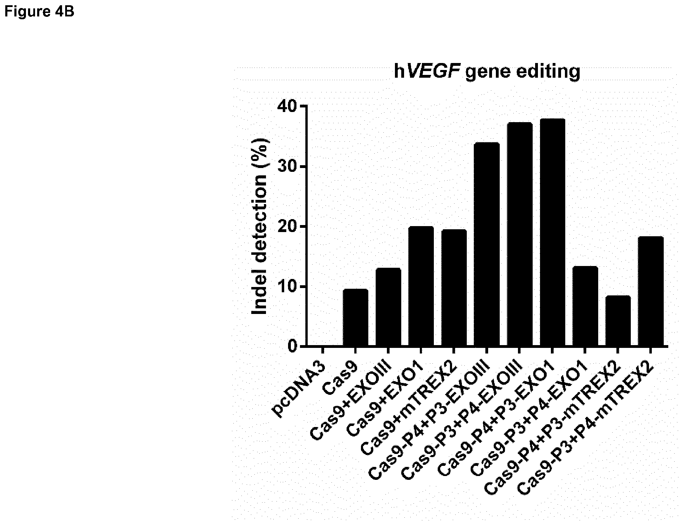

**Figure 4****:** Genome editing in human *VEGF* genome region. By coexpression of Cas9 and gRNA, directed towards human *VEGF* genomic region human *VEGF* genome region is modified. Greater genome editing occurred when Cas9, gRNA were coeexpressed with exonucleases, wherein EX01, EXOIII, or mTREX2 is at least one of the exonuclease used. Bigger genome editing occurred when CRISPR/Cas system tethered to certain exonucleases via heterodimeric coiled-coil forming peptides were used, wherein Cas9-P3 or Cas9-P4 are one of the Cas9 variants and P3-EX01, P4-EX01, P3-EXOIII, P4-EXOIII, P3-mTREX2 or P4-mTREX2 are one of the variants of expressed exonucleases. A) Human *VEGF* genome editing resulted in indel mutations, detected via T7E1 assay. B) Indel detection (%) of *VEGF* genome region engineering.

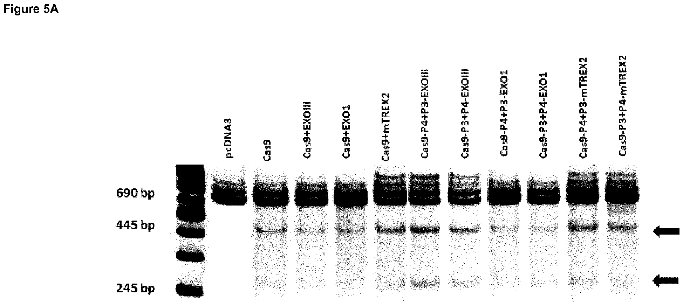

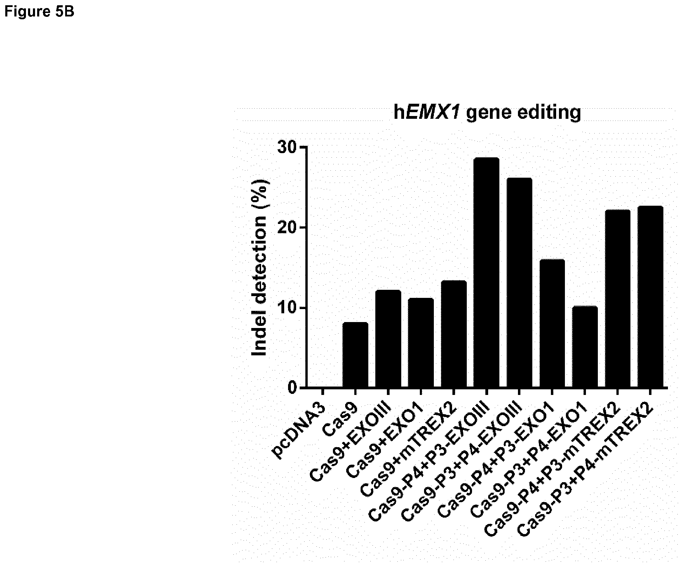

**Figure 5****:** Genome editing in human *EMX1* genome region. By coexpression of Cas9 and gRNA, directed towards human *EMX1* genomic region human *EMX1* genome region is modified. Greater genome editing occurred when Cas9, gRNA were coeexpressed with exonucleases, wherein EX01, EXOIII, or mTREX2 is at least one of the exonuclease used. Bigger genome editing occurred when CRISPR/Cas system tethered to certain exonucleases via heterodimeric coiled-coil forming peptides were used, wherein Cas9-P3 or Cas9-P4 are one of the Cas9 variants and P3-EX01, P4-EX01, P3-EXOIII, P4-EXOIII, P3-mTREX2 or P4-mTREX2 are one of the variants of expressed exonucleases. A) Human *EMX1* genome editing resulted in indel mutations, detected via T7E1 assay. B) Indel detection (%) of *EMX1* genome region engineering.

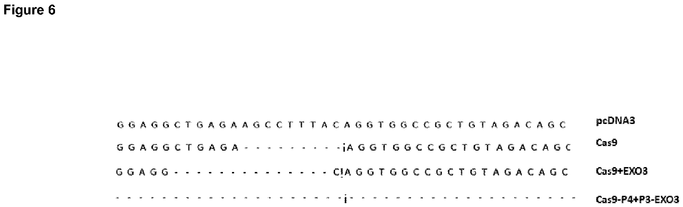

**Figure 6****:** Equivalent Cas9 protein expression. Cas9 protein is expressed in the same manner when only Cas9 is expressed or cooexpressed with exonucleases, wherein EX01, EXOIII, or mTREX2 is at least one of the exonuclease used. The same Cas9 expression is observed when CRISPR/Cas system tethered to certain exonucleases via heterodimeric coiled-coil forming peptides were used, wherein Cas9-P3 or Cas9-P4 are one of the Cas9 variants and P3-EX01, P4-EX01, P3-EXOIII, P4-EXOIII, P3-mTREX2 or P4-mTREX2 are one of the variants of expressed exonucleases. Cas9 expression is detected via Western blot and immunodetection.

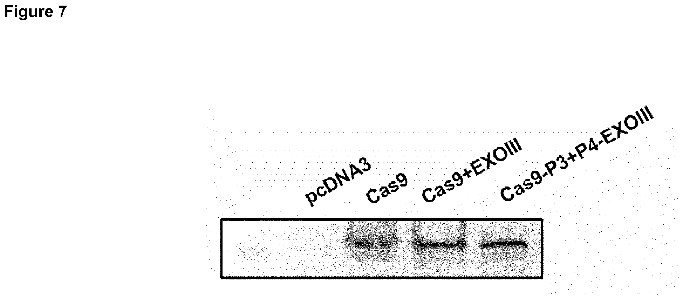

**Figure 7****:** Genome editing in human *MYD88* genome region. By coexpression of Cas9 and gRNA, directed towards human *MYD88* genomic region human *MYD88* genome region is modified. Greater genome editing occurred when Cas9, gRNA were coeexpressed with exonucleases, wherein EX01, EXOIII, or mTREX2 is at least one of the exonuclease used. Bigger genome editing occurred when CRISPR/Cas system tethered to certain exonucleases via heterodimeric coiled-coil forming peptides were used, wherein Cas9-P3 or Cas9-P4 are one of the Cas9 variants and P3-EX01, P4-EX01, P3-EXOIII, P4-EXOIII, P3-mTREX2 or P4-mTREX2 are one of the variants of expressed exonucleases. Next generation sequencing was performed to detect the size of edited genome region.

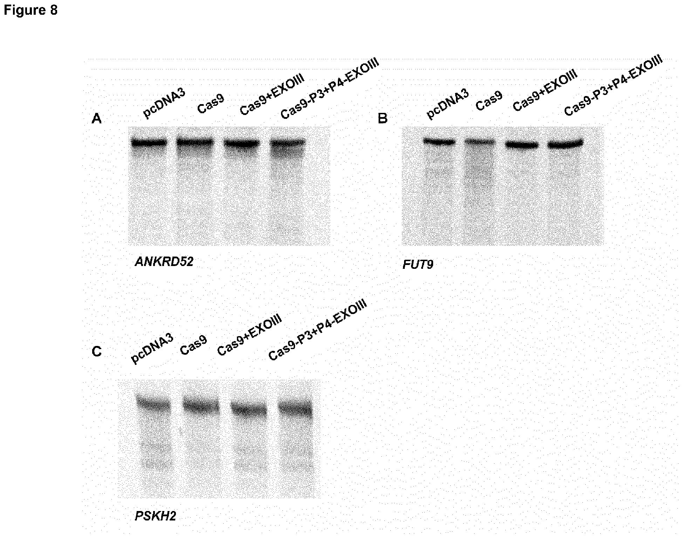

**Figure 8****:** Absence of human *MYD88* "off target" genome editing due to increased CRISPR/Cas action. By coexpression of Cas9 and gRNA, directed towards human *MYD88* genomic region human *MYD88* genome region is modified. Greater genome editing occurred when Cas9, gRNA were coeexpressed with exonucleases, wherein EX01, EXOIII, or mTREX2 is at least one of the exonuclease used. Bigger genome editing occurred when CRISPR/Cas system tethered to certain exonucleases via heterodimeric coiled-coil forming peptides were used, wherein Cas9-P3 or Cas9-P4 are one of the Cas9 variants and P3-EX01, P4-EX01, P3-EXOIII, P4-EXOIII, P3-mTREX2 or P4-mTREX2 are one of the variants of expressed exonucleases. Three predicted coding regions of human *MYD88* gene targeting gRNA "off-target" sites were screened via T7E1 assay. A) Predicted "off-target" site 1 (human *ANKRD52* gene) genome editing did not result in indel mutations, detected via T7E1 assay. B) Predicted "off-target" site 2 (human *FUT9* gene) genome editing did not result in indel mutations, detected via T7E1 assay. C) Predicted "off-target" site 3 (human *PSKH2* gene) genome editing did not result in indel mutations, detected via T7E1 assay.

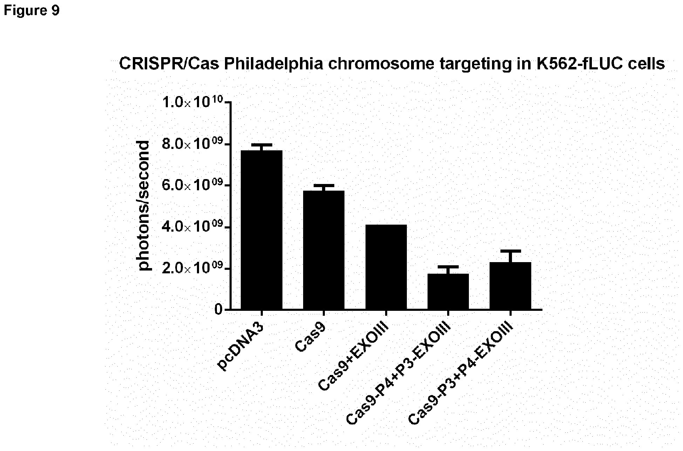

**Figure 9****:** Reduction in bioluminescence signal (photons/second) of K562-fLUC cells. gRNA targeting BCR-ABL chromosomal translocation was designed. CRISPR/Cas genome editing at BCR-ABL chromosomal translocation resulted in cell death, detected via drop of bioluminescence signal. By coexpression of Cas9 and gRNA, directed towards human BCR-ABL chromosomal translocation genomic region human *BCR-ABL* genome region is modified. Greater genome editing occurred when Cas9, gRNA were coeexpressed with exonucleases, wherein EX01, EXOIII, or mTREX2 is at least one of the exonuclease used. Bigger genome editing occurred when CRISPR/Cas system tethered to certain exonucleases via heterodimeric coiled-coil forming peptides were used, wherein Cas9-P3 or Cas9-P4 are one of the Cas9 variants and P3-EX01, P4-EX01, P3-EXOIII, P4-EXOIII, P3-mTREX2 or P4-mTREX2 are one of the variants of expressed exonucleases.

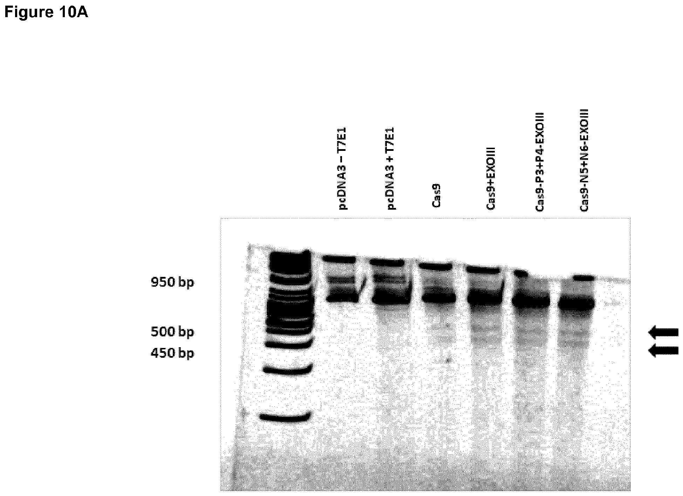

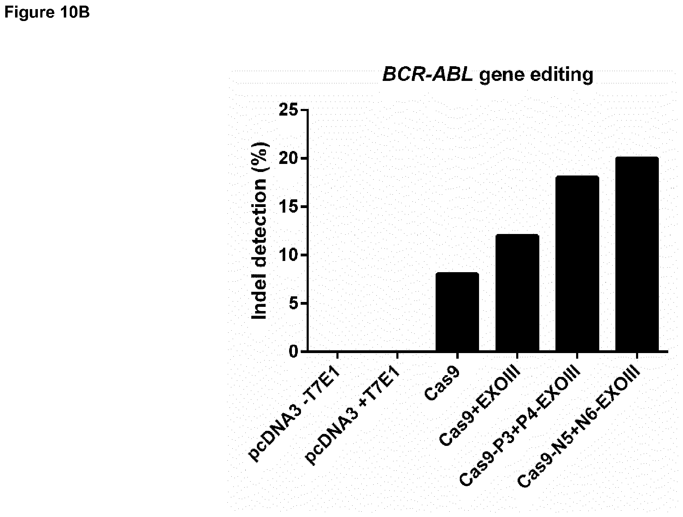

**Figure 10****:** Genome editing in human *BCR-ABL* genome region. By coexpression of Cas9 and gRNA, directed towards human *BCR-ABL* genomic region human *BCR-ABL8* genome region is modified. Greater genome editing occurred when Cas9, gRNA were coeexpressed with exonucleases, wherein EXOIII is at least one of the exonuclease used. Bigger genome editing occurred when CRISPR/Cas system tethered to certain exonucleases via heterodimeric coiled-coil forming peptides were used, wherein Cas9-P3 are one of the Cas9 variants and P4-EXOIII are one of the variants of expressed exonucleases. Even greater genome editing occurred when Cas9-N5 and N6-EXOIII variants were used, showing stronger heterodimerization affinity of peptides that form coiled-coils.

### Definitions

The term CRISPR/Cas system, as used herein relates to one or more gRNA that guides catalytically active Cas9 or its variants or inactive nickase Cas9 or its variants protein to the desired genomic site where DSB formations are formed through specific Cas9 action after appropriate PAM recognition. Cas9 protein in this invention is fused with heterodimerization system at N- or C-terminus that allows tethering with certain exonucleases.

Cas protein, as used herein relates to Cas9 and its variants and homologous derived from *Streptococcus pyogenes* that recognizes NGG or NAG PAM. This invention relies also on all other DNA catalytically active or inactive endonucleases (dCas9) or nickases derived from all types of CRISPR system.

APOBEC polypeptide, as used herein relates to proteins that have deaminase activity and are connected with Cas9 and its variants and causes single base editing.

Genome editing, as used herein relates to any modification to genomic and/or non-genomic DNA due to CRISPR/Cas system and its efficiency to cause DSBs. Sequentially single base editing, knockin or knockout cells and/or organisms are processed.

Organism, as used herein relates to any living organism.

The term exonucleases as used herein, relates to any sort of enzymes that are able to cleave nucleotides from polynucleotide chain in 3' and/or 5' direction and are connected to heterodimerization system at N- or C-terminus.

The term "heterodimerization system", as used herein, refers to a pair of peptides or peptide domains that form coiled-coils or that connect in the presence of an inductor or a signal for dimerization. The term "heterodimerization", as used herein, refers to protein domains or peptide pairs that connect to other domains of a different type through covalent or non-covalent interactions. The term "constitutive dimerization domains", as used herein, refers to dimerization domains that connect themselves independently, such as, for example, coiled coils. The term "inducible heterodimerization domain", as used herein, refers to heterodimerization domains that merge only in the presence of a heterodimerization inductor.

The term "heterodimerization inductor" refers to a signal, a chemical or non-chemical ligand, which cause the heterodimerization of protein domains that do not have intrinsic affinity with each other and cannot be connected independently in the absence of a heterodimedimerization ligand or signal. A heterodimerization inductor according to the invention can be a small molecule, such as rapalog, abscisic acid, or gibberellin. According to this invention, a heterodimerization inductor can also be light with certain wavelength.

Light induced heterodimerization system, as used herein, refers to protein partners CIB1 or its homologous and CRY2PHR, derived from *Arabidopsis thaliana* or are produced synthetically and heterodimerize in the presence of blue light with certain wavelength, approximately 450 nm. Another blue light induced heterodimerization system is LOVpep and ePDZB protein partners, derived from *Avena sativa.* This invention also relates to 650 nm wavelength red light induced heterodimerization system, where protein partners PIF and PHYB or its synthetic homologous are used. Another light induced heterodimerization system that this invention relies is heterodimerization of FKF1 and GI protein partners from *Gigantea* sp.

The term "chemical ligand", as used herein, refers to a small molecule, such as rapamycin and its rapalogs that can bind to proteins, preferably to FKBP-FRB heterodimerization domains; to plant hormones, such as abscisic acid and giberellin that can bind to proteins, preferably to ABI-PYL1 and GID-GAI1 heterodimerization domains.

The term peptide pair that form coiled coils, used herein refers to any peptide pair, heterodimerizing or homodimerizing peptides that express coiled coil structural motif, a combination of hydrophobic and electrostatic interactions between amino acid residues within heptads of alpha-helices that results in heterodimerization or homodimerization.

The term "orthogonal", as used herein, refers to the characteristic of the components of the engineered CRISPR/Cas system tethered to certain exonucleases via different heterodimerization systems to take place separately, i.e., that the individual parts of different heterodimerization systems do not interact with parts of other heterodimerization systems of endogenous or exogenous signaling pathways. The main feature of the orthogonal system is that the input signal of each system leads to the heterodimerization independently of the presence or absence of other systems or their input and output signals.

The term "cell", as used herein, refers to an eukaryotic or prokaryotic cell, a cellular or multicellular organism (cell line) cultured as a single cell entity that has been used as a recipient of nucleic acids and includes the daughter cells of the original cell that has been genetically modified by the inclusion of nucleic acids. The term refers primarily to cells of higher developed eukaryotic organisms, preferably vertebrates, preferably mammals. This invention relies also on non-vertebrates cells, preferably plant cells.

The term "cells" also refers to human cell lines and plant cells. Naturally, the descendants of one cell are not necessarily completely identical to the parents in morphological form and its DNA complement, due to the consequences of natural, random or planned mutations. A "genetically modified host cell" (also "recombinant host cell") is a host cell into which the nucleic acid has been introduced. The eukaryotic genetically modified host cell is formed in such a way that a suitable nucleic acid or recombinant nucleic acid is introduced into the appropriate eukaryotic host cell. The invention hereafter includes host cells and organisms that contain a nucleic acid according to the invention (transient or stable) bearing the operon record according to the invention. Suitable host cells are known in the field and include eukaryotic cells. It is known that proteins can be expressed in cells of the following organisms: human, rodent, cattle, pork, poultry, rabbits and the like. Host cells may include cultured cell lines of primary or immortalized cell lines.

The term "nucleic acids", as used herein, refers to a polymeric form of nucleotides (ribonucleotides or deoxyribonucleotides) of any length and is not limited to single, double or higher chains of DNA or RNA, genomic DNA, cDNA, DNA-RNA hybrids, or polymers with a phosphorothioate polymer backbone made from purine and pyrimidine bases or other natural, chemical or biochemically modified, synthetic or derived nucleotide bases.

The term "protein", as used herein, refers to the polymeric form of amino acids of any length, which expresses any function, for instance localizing to a specific location, localizing to specific DNA sequence, facilitating and triggering chemical reactions, transcription regulation.

The term "recombinant", as used herein, means that a particular nucleic acid (DNA or RNA) is a product of various combinations of cloning, restriction and / or ligation leading to a construct having structurally coding or non-coding sequences different from endogenous nucleic acids in a natural host system.

The insertion of the vectors into the host cells is carried out by conventional methods known from the field of science, and the methods relate to transformation or transfection and include: chemically induced insertion, electroporation, micro-injection, DNA lipofection, cellular sonication, gene bombardment, viral DNA input, as well as other methods. The entry of DNA may be of transient or stable. Transient refers to the insertion of a DNA with a vector that does not incorporate the DNA of the invention into the cell genome. A stable insertion is achieved by incorporating DNA of the invention into the host genome. The insertion of the DNA of the invention, in particular for the preparation of a host organism having stably incorporated a nucleic acid, e.g. a DNA, of the invention, can be screened by the presence of markers. The DNA sequence for markers refers to resistance to antibiotics or chemicals and may be included on a DNA vector of the invention or on a separate vector.

The insertion of the CRISPR/Cas tethered with certain exonucleases via different heterodimerization systems are delivered to the cell as plasmid DNA or as mRNA or a proteins or as RNA:protein complexes where different parts of CRISPR/Cas system tethered with certain exonucleases via different heterodimerization systems can be as DNA or RNA or protein.

### DETAILED DESCRIPTION OF THE INVENTION

The invention relates to an enhanced CRISPR/Cas system that is made of a combination of two orthogonal protein partners of different heterodimerization (HD) systems or coiled-coil forming peptide pairs, connected with a Cas protein and certain exonucleases that reconstitute upon addition of an inductor of heterodimerization or via intrinsic affinity of peptide pairs that form coiled-coils. Functionally, enhanced CRISPR/Cas system influences the higher rate of genome editing based on larger insertion and/or deletion mutations that arise in higher percentage of genetically modified cells or organisms.

Chemical and non-chemical control over the activation of enhanced CRISPR/Cas system tethered to certain exonucleases via different heterodimerization systems of gene modified cells or organisms of this invention presents a novel way of gene editing in different research area, e.g. cancer therapy, new model organism production, new drug development etc.

The invention provides a combination comprising a first component (a) and a second component (b), wherein
a) the first component comprises an endonuclease capable of inducing a double stranded DNA break or a nickase capable of inducing a DNA nick, preferably a Cas protein or mutant thereof, fused with a first protein or protein domain, and
b) the second component comprises an exonuclease, fused to a second protein or protein domain,
wherein the first and the second components are capable of heterodimerizing with each other via interaction of the first protein or protein domain and the second protein or protein domain.

The combination of the invention represents a modified CRISPR/Cas system. The endonuclease of the first component (a) can in particular be a Cas protein, a Cas nickase or dCas9 protein. The second component (b) can in particular be an exonuclease or cytidine deaminase, in particular APOBEC protein.

According to a particular aspect of the invention, heterodimerization of the first protein or protein domain and the second protein or domain is inducible via a chemical or non-chemical signal. The (at least one) first protein or protein domain of the first component can be a single protein partner of a selected chemically or non-chemically inducible heterodimerization system (e.g. ABI-PYL1, FKBP-FRB or DmrC-DmrA, GAI-GID1) or non-chemically (e.g. CIBN-CRY2PHR, LOvpep-ePDZb, PIF-PHYB, FKF1-GI) that is genetically fused to a Cas protein or its variants; for example: CIBN or LOVpep or PIF or FKF1 or ABI or FKBP or GAI can be connected to a Cas protein or its variants (e.g. Cas9, Cas9nickase, Cpf1).

The second protein or protein domain of the second component can be a second protein partner of a selected heterodimerization system (e.g. ABI-PYL1, FKBP-FRB or DmrC-DmrA, GAI-GID1, CIBN-CRY2PHR, LOvpep-ePDZb, PIF-PHYB, FKF1-GI) that is genetically fused to a certain exonuclease (e.g. human EX01, EXOIII E.coli, TREX2 etc.).

Addition of a chemical inductor of heterodimerization (HD) can trigger heterodimerization of the first component and the second component by heterodimerization of two protein partners (i.e. the at least one protein or protein domain and the second protein or protein domain), thereby resulting in a functional modified CRISPR/Cas system tethered with certain exonucleases, which can influence the genome modification of any GOI.

A chemical inductor for triggering heterodimerization of the at least one first protein or protein domain of the a) first component and the second protein or protein domain of the b) second component can be, e.g. rapalog (e.g. rapamycin) for triggering heterodimerization of a FKBP-FRB HD system (the at least one first protein domain is FKBP, the second protein domain is FRB), abscisic acid for triggering heterodimerization of a ABI-PYL1 HD system (the at least one first protein domain is ABI, the second protein domain is PYL1), gibberellin for triggering heterodimerization of a GID-GA1 HD system (the at least one first protein domain is GID, the second protein domain is GA1) or any other relevant physiological chemical signal.

Addition of a non-chemical inductor of heterodimerization (HD) can trigger heterodimerization of the first component and the second component by heterodimerization of two protein partners (i.e. the at least one protein or protein domain and the second protein or protein domain), thereby resulting in a functional modified CRISPR/Cas system tethered with certain exonucleases, which can influence the genome modification of any GOI.

A non-chemical inductor for triggering heterodimerization of the at least one first protein or protein domain of the a) first component and the second protein or protein domain of the b) second component can be light with appropriate wave length, e.g. blue light (e.g. 450 nm wave length) for triggering heterodimerization of a CIBN-CRY2PHR HD system (the at least one first protein domain is CIBN, the second protein domain is CRY2PHR) or LOVpep-ePDZb HD system (the at least one first protein domain is LOVpep, the second protein domain is ePDZb). Other inductor for light induced heterodimerization is red light (e.g. 650 nm wave length) for triggering heterodimerization of a PIF-PHYB HD system (the at least one first protein domain is PIF, the second protein domain is PHYB) or for triggering heterodimerization of a FKF1-GI HD system (the at least one first protein domain is FKF1, the second protein domain is GI) or any other relevant physiological non-chemical signal.

In a further embodiment of the invention, the heterodimerization between endonuclease or nickase (e.g. Cas protein or variant or mutant thereof) and certain exonuclease can be achieved using peptide pairs that form coiled-coils due to the intrinsic affinity and electrostatic interactions and thus bringing certain exonucleases into close proximity of DSB allowing exonuclease to additionally recise DNA in 3' and/or 5' manner. In this embodiment, enhancing the function of regular CRISPR/Cas system through additional DNA recizing with certain exonucleases can result in greater percentage of genome modification compared to normal CRISPR/cas system or system where CRISPR/Cas and certain exonucleases are cooexpressed in tested cell or organism (see, e.g. Fig. 1).

The first component of peptide pair coiled-coil induced heterodimerization system can be at least one peptide or peptide domain from peptide pair that form coiled-coils of a selected peptide pairs (e.g. P3-P4, N5-N6, P3S-P4S etc) that is genetically fused to a Cas protein or its variants; for example: P3, N5 or P3S can be connected to Cas9 protein or its variants (e.g. Cas9, Cas9nickase, Cpf1 etc.).

The second component of peptide pair coiled-coil induced heterodimerization system can be at least one peptide or peptide domain from peptide pair that form coiled-coils of a selected peptide pairs (e.g. P3-P4, N5-N6, P3S-P4S etc.) that is genetically fused to a certain exonucleases (human EX01, EXOIII E.coli, TREX2 etc).

In a further embodiment of the invention, the selected pair of peptide pair that form coiled-coils are chosen based on their physical properties (e.g. Kd values, their orthogonality properties etc.). Peptide pairs that form coiled-coils can have strong heterodimerization properties: for example, peptide pair N5-N6 exhibit stronger heterodimerization affinity than peptide pair P3S-P4S. From this follows that percentage of genome editing in N5-N6 used heterodimerizing peptide pair that form coiled-coils for enhanced CRISPR/Cas system tethered with certain exonucleases is greater than for the example where P3S-P4S heterodimerizing peptide pair that form coiled-coils is used, that exhibit poor heterodimerization properties.

In a further embodiment, heterodimerization of peptide pairs, which form coiled-coils, can be obtained by using peptide pairs that heterodimerize in parallel (e.g. N5-N6, P3-P4 etc.) or anti-parallel (e.g.P3-AP4) manner.

In a further embodiment the use of heterodimerization systems for tethering CRISPR/Cas system with certain exonucleases, additional DNA recession with certain exonucleases and sequential improvement in genome editing does not enhance possible off-target activities of CRISPR/Cas system.

In a further embodiment, the modified CRISPR/Cas system tethered to certain exonucleases via different heterodimerization systems according to the invention comprises several repeats of the at least one first protein domain and/or the second protein domain, e.g. 10 repeats of a coiled coil. In this embodiment, the genome editing can be further enhanced. Several repeats (for example 10 repeats of coiled coil of a peptide pair) of the at least one first protein domain can then bind to several repeats (for example 10 repeats of coiled coil) of the second protein domain, which is fused to certain exonuclease. The first component comprising the at least one first protein domain having several repeats then heterodimerizes with the second component comprising the second protein domain having several repeats after the appropriate chemical inductor is added. In this embodiment, the repeats can act as constitutive dimerization domains that assemble upon the heterodimerization signal by the chemical inductor as orthogonal coiled coil pairs.

In a further embodiment, the at least one first protein domain of the chemical signal-inducible heterodimerization protein complex of the first component is selected from the group consisting of the pairs:
i) FKBP and FRB;
ii) FKBP and calcineurin catalytic subunit A (CnA);
iii) FKBP and cyclophilin;
iv) gyrase B (GyrB) and GyrB;
v) DmrA and DmrC;
vi) ABI and PYL1; and
vii) GAI and GID1, and
the second protein domain of the second component is selected from the group consisting of the pairs:
i) FKBP and FRB;
ii) FKBP and calcineurin catalytic subunit A (CnA);
iii) FKBP and cyclophilin;
iv) gyrase B (GyrB) and GyrB;
v) DmrA and DmrC;
vi) ABI and PYL1; and
vii) GAI and GID1.

In a further embodiment, the at least one first protein domain of the light signal-inducible heterodimerization protein complex of the first component is selected from the group consisting of the pairs:
i) CIB1 and CRY2;
ii) LOV and PDZ;
iii) PIF and PHYB;
iv) FKF1-GI
v) UVR8-COP1,
and the second protein domain of the second component is selected from the group consisting of the pairs:
i) CIB1 and CRY2;
ii) LOV and PDZ;
iii) PIF and PHYB;
iv) FKF1-GI
v) UVR8-COP1.

In a further embodiment of the invention, the at least one first protein domain and the second protein domain can be from two different proteins and can have orthologous properties.

The invention further provides an isolated nucleic acid molecule, or a combination of isolated nucleic acid molecules, comprising a first nucleic acid sequence encoding the first component (a) and a second nucleic acid sequence encoding the second component (b) of the combination according to the invention as defined above. The invention further provides a vector, preferably an expression vector, encoding the nucleic acid molecule(s) of the invention.

In particular, the invention provides isolated nucleic acid molecules comprising sequences comprising or consisting of SEQ ID NOs: 1-18, as per the enclosed sequence listing. The invention further provides vectors comprising nucleic acid sequences comprising or consisting of SEQ ID NOs: 1-18, as per the enclosed sequence listing.

The invention further provides a vector encoding the isolated nucleic acids for protein isolation of the invention.
The invention further provides a cell or organism comprising the combination, the nucleic acid molecule(s) or the vector according to the invention as defined above, provided that the cell is not a human germ line cell.

The invention further provides the combination, the nucleic acid molecule(s) or the vector according to the invention as defined above for use in medicine, or for use as a medicament. The invention further provides the combination, the nucleic acid molecule(s) or the vector according to the invention as defined above for use in a method of treating cancer. In one embodiment of the invention, the aforementioned use comprises the use of a chemical inductor of heterodimerization. Preferably, the cells for use in a method of treating cancer are modified T cells. Preferably, the types of cancer to be treated according to the aforementioned use according to the invention include, CML, B-cell lymphoma, acute lymphoblastic leukemia, chronic lymphocytic leukemia, Hodgkins and Non-Hodgkins Lymphomas, mezotelioma, ovarian cancer, prostate cancer etc..

The invention further provides a method for making knockout cells or organisms in more efficient manner where invention provides the combination, the nucleic acid molecule(s) or the vector according to the invention as defined above where genome editing is enhanced by the tethered exonucleases.

The invention further provides a method for making knockin cells or organisms in more efficient manner where invention provides the combination, the nucleic acid molecule(s) or the vector according to the invention as defined above where genome editing is enhanced by the tethered exonucleases and by coaddition of template DNA.

The invention further provides the combination, the nucleic acid molecule(s) or the vector according to the invention as defined above for use as a tool for drug new sources of fuel development etc.
The examples described in more detail below are designed to best describe the invention. These examples do not limit the scope of the invention, but are merely intended to provide a better understanding of the invention and its use.

### Example 1: Preparation of constructs for modified CRISPR/Cas system tethered with certain exonucleases via different heterodimerization systems

*Preparation of DNA coding for modified CRISPR*/*Cas system tethered with certain exonucleases via different heterodimerization systems.* In order to prepare DNA constructs, the inventors used molecular biology methods such as: chemical transformation of competent E. *coli* cells, plasmid DNA isolation, polymerase chain reaction (PCR), reverse transcription - PCR, PCR linking, nucleic acid concentration determination, DNA agarose gel electrophoresis, isolation of fragments of DNA from agarose gels, chemical synthesis of DNA, DNA restriction with restriction enzymes, cutting of plasmid vectors, ligation of DNA fragments, purification of plasmid DNA in large quantities. The exact course of experimental techniques and methods are well known to experts in the field and are described in the manuals of molecular biology.

All the work was performed using sterile techniques, which are also well known to the experts in the field. All plasmids, completed constructs and partial constructs were transformed into the bacteria *Escherichia coli* by chemical transformation. Plasmids for transfection into cell lines (animal or human) have been isolated using a DNA isolation kit that removes endotoxins.

In the described cases, the Cas9 protein was selected from all types of CRISPR system. Exonucleases were selected among known exonucleases. The Cas9 was connected via a peptide linker with different protein domains of different heterodimerization systems or with peptide from peptide pairs that form coiled-coils at C-terminus. Exonucleases were connected via a peptide linker with different protein domains of different heterodimerization systems or with peptide from peptide pairs that form coiled-coils at N-terminus.

The sequences of Cas9 and certain exonucleases according to the invention are listed in **Table 1.** All operons were prepared by techniques according to methods known to experts in the field. The operons were inserted into plasmids suitable for eukaryotic systems. The suitability of the nucleotide sequence was confirmed by the inventors by sequencing and restriction analysis.

**Table 1: Fusion proteins of components of exemplary artificial engineered NFAT2 transcription factors.**

| | | | |
|---|---|---|---|
| 1 | Cas9-P4 | pcDNA3 | Cas9 from *Streptococcus pyogenes* fused with P4 peptide, that form coiled-coil |
| 2 | Cas9-P3 | pcDNA3 | Cas9 from *Streptococcus pyogenes* fused with P3 peptide, that form coiled-coil |
| 3 | Cas9-N5 | pcDNA3 | Cas9 from *Streptococcus pyogenes* fused with N5 peptide, that form coiled-coil |
| 4 | Cas9-N6 | pcDNA3 | Cas9 from *Streptococcus pyogenes* fused with N6 peptide, that form coiled-coil |
| 5 | Cas9-P3S | pcDNA3 | Cas9 from *Streptococcus pyogenes* fused with P3S peptide, that form coiled-coil |
| 6 | Cas9-P4S | pcDNA3 | Cas9 from *Streptococcus pyogenes* fused with P4S peptide, that form coiled-coil |
| 7 | Cas9-AP4 | pcDNA3 | Cas9 from *Streptococcus pyogenes* fused with AP4 peptide, that form coiled-coil |
| 8 | P4-EXOIII | pcDNA3 | Exonuclease EXOIII, derived from *Escherichia coli* fused with P4 peptide, that form coiled-coil |
| 9 | P3-EXOIII | pcDNA3 | Exonuclease EXOIII, derived from *Escherichia coli* fused with P3 peptide, that form coiled-coil |
| 10 | N5-EXOIII | pcDNA3 | Exonuclease EXOIII, derived from *Escherichia coli* fused with N5 peptide, that form coiled-coil |
| 11 | N6-EXOIII | pcDNA3 | Exonuclease EXOIII, derived from *Escherichia coli* fused with N6 peptide, that form coiled-coil |
| 12 | P3S-EXOIII | pcDNA3 | Exonuclease EXOIII, derived from *Escherichia coli* fused with P3S peptide, that form coiled-coil |
| 13 | P4S-EXOIII | pcDNA3 | Exonuclease EXOIII, derived from *Escherichia coli* fused with P4S peptide, that form coiled-coil |
| 14 | AP4-EXOIII | pcDNA3 | Exonuclease EXOIII, derived from *Escherichia coli* fused with AP4 peptide, that form coiled-coil |
| 15 | P3-TREX2 | pcDNA3 | Exonuclease TREX2, derived from *Mus musculus* fused with P3 peptide, that form coiled-coil |
| 16 | P4-TREX2 | pcDNA3 | Exonuclease TREX2, derived from *Mus musculus* fused with P4 peptide, that form coiled-coil |
| 17 | P3-EXO1 | pcDNA3 | Exonuclease EXO1, derived from *Homo sapiens* fused with P3 peptide, that form coiled-coil |
| 18 | P4-EXO1 | pcDNA3 | Exonuclease EXO1, derived from *Homo sapiens* fused with P4 peptide, that form coiled-coil |

Methods and techniques of cultivating cell cultures are well known to experts in the field and are therefore briefly described in order to illustrate the implemented examples. Cells from the HEK293T cell line were grown at 37 ° C and 5% CO₂. For cultivation, a DMEM medium containing 10% FBS was used, containing all the necessary nutrients and growth factors. Cells K562 were grown in RPMI medium containing 10% FBS at 37 ° C and 5% CO₂. When the cell culture reached the appropriate density, the cells were grafted into a new breeding flask and / or diluted. For the use of cells in experiments, the number of cells was determined by a hemocytometer and seeded in density 1x10⁵ per hole into the microtiter plate with 24 holes 18-24 hours before transfection regarding HEK293 cells. The seeded plates were incubated at 37 ° C and 5% CO₂ until the cells were 50-70% confluent and ready for transfection by transfection reagent. The transfection was carried out according to the instructions of the transfection reagent manufacturer (e.g., JetPei, Lipofectamine 2000) and was adapted for the microtiter plate used. K562 (1x10⁴) were electroporated with the use of Neon electroporation system in 10 µl electroporation tips. Afterwards the cells were put in RPMI medium, containg 10% FBS in a well of 12 well plate. On the next day the inductor for heterodimerization were added if chemical and non-chemical induced heterodimerization system were used.48 hours after transfection genomic DNA was isolated and desired area PCR amplified. Next T7E1 (T7 endonuclese 1) assay was performed to determine the percentage of indel mutations.

### Example 2. Defining the most successful exonuclease cooexpressed with CRISPR/Cas system for enhacing genome editing at eGFP genomic region

To detect the most efficient exonuclease cooexpression of certain exonucleases with CRISPR/Cas system was used. HEK293 cell line that stably expresses eGFP was used.

HEK293T, stably expressing eGFP cells, seeded in 24-well plates, were transfected one day prior to the experiment with plasmids, which encode for one of the examined exonuclease and a separate plasmid that encodes CRISPR/Cas system; Cas9 protein from *Streptococcus pyogenes* and gRNA (targeting sequence in eGFP genomic region: *GGCGAGGGCGATGCCACCTA*)*.*

To analyze the genome editing activity of cooexpression of certain exonuclease and CRISPR/Cas system, cells were analyzed on flow cytometry 48 hours after transfection. We measured the fluorescence intensity in cell population respectively and presented results in % of MFI (mean fluorescence intensity)

### Results:

It is clear from Figure 2 that the cooexpression of certain exonucleases and CRISPR/Cas system did enhance genome editing in eGFP genomic region regarding diminished fluorescence in all cell samples, transfected with CRISPR/Cas system and certain exonuclease. Cooexpression of CRISPR/Cas system targeting *eGFP* GOI and EXOIII, derived from *E.coli* showed the most edited percentage of tested cells due to the lowest % of MOI.

### Example 3. Activity of modified CRISPR/Cas system tethered with certain exonucleases via heterodimerizing peptide pairs that form coiled-coils

To test the activity of modified CRISPR/Cas system tethered with certain exonucleases via heterodimerizing peptide pairs HEK293 cells were used to determine the activity of modified CRISPR/Cas system tethered with certain exonucleases via heterodimerizing peptide pairs. Cells were transfected with constructs that express CRISPR/Cas system tethered with certain exonucleases via heterodimerizing peptide pairs; for example Cas9-P3 or Cas9-P4 with combination of P3-EX01 or P4-EX01 or P3-EXOIII or P4-EXOIII or P3-mTREX2. For control, cells were transfected with regular CRISPR/Cas9 system or were cooexpressed with CRISPR/Cas9 system and certain exonuclease (e.g. EX01 or EXOIII or mTREX2). gRNA for human *EMX1* gene (targeting sequence: *GAGTCCGAGCAGAAGAAGAA*) or human *VEGF gene* (targeting sequence: *GGTGAGTGAGTGTGTGCGTG*) or human *MYD88 (GGCTGAGAAGCCTTTACAGG*) were used. 48 hours genomic DNA from transfected cells was isolated and genomic region around predicted DSB was PCR amplified. Next T7E1 assay was carried out. Presence and quantification of indel mutation was determined by Syber gold stained PAGE (polyacrylamide gel electrophoresis) gel analysis. Percentage of indel mutations was calculated by ImageJ software. Also next-generation sequencing (NGS) was performed from 150 base pair long amplicons.

### Result:

PAGE gel analysis of T7E1 treated samples from transfected cells with appropriate constructs in Figure 3A indicate that genome editing of human *MYD88* gene was highest among all tested samples when Cas9-P4 tethered to EXOIII via P3 peptide was used. A modest decrease in genome editing was observed when cells were transfected with constructs, expressing Cas9-P3 and P4-EXOIII. In all cases, where modified CRISPR/Cas system tethered with certain exonucleases (EX01 or EXOIII or mTREX2) via heterodimerizing peptide pairs (P3-P4) were used genome editing was higher compared to cell samples with cooexpression of Cas9, gRNA targeting human *MYD88* gene and certain exonucleases (e.g. EX01 or EXOIII or mTREX2). Genome editing, where modified CRISPR/Cas system tethered with certain exonucleases (EX01 or EXOIII or mTREX2) via heterodimerizing peptide pairs (P3-P4) were used was also higher when regular CRISPR/Cas system was used, where only Cas9 and gRNA targeting human *MYD88* gene were used. Figure 3B graphically shows percentage of indel presence, which were determined with PAGE analysis, depicted in figure 3A.

PAGE gel analysis of T7E1 treated samples from transfected cells with appropriate constructs in Figure 4A indicate that genome editing of human *VEGF* gene was highest among all tested samples when Cas9-P3 tethered to EXOIII via P4 heterodimerizing peptide was used. A modest decrease in genome editing was observed when cells were transfected with constructs, expressing Cas9-P4 and P3-EXOIII. Genome editing was higher compared to cell samples with cooexpression of Cas9, gRNA targeting human *VEGF* gene and certain exonucleases (e.g. EX01 or EXOIII or mTREX2). Genome editing, where modified CRISPR/Cas system tethered with certain exonucleases (EX01 or EXOIII) via heterodimerizing peptide pairs (P3-P4) were used was also higher when regular CRISPR/Cas system was used, where only Cas9 and gRNA targeting human *VEGF* gene were used. Figure 4B graphically shows percentage of indel presence, which were determined with PAGE analysis, depicted in figure 4A.

PAGE gel analysis of T7E1 treated samples from transfected cells with appropriate constructs in Figure 5A indicate that genome editing of human *EMX1* gene was highest among all tested samples when Cas9-P4 tethered to EXOIII via P3 peptide was used. A modest decrese in genome editing was observed when cells were transfected with constructs, expressing Cas9-P3 and P4-EXOIII. In all cases, where modified CRISPR/Cas system tethered with certain exonucleases (EX01 or EXOIII or mTREX2) via heterodimerizing peptide pairs (P3-P4) were used genome editing was higher compared to cell samples with cooexpression of Cas9, gRNA targeting human *EMX1* gene and certain exonucleases (e.g. EX01 or EXOIII or mTREX2). Genome editing, where modified CRISPR/Cas system tethered with certain exonucleases (EX01 or EXOIII or mTREX2) via heterodimerizing peptide pairs (P3-P4) were used was also higher when regular CRISPR/Cas system was used, where only Cas9 and gRNA targeting human *EMX1* gene were used. Figure 5B graphically shows percentage of indel presence, which were determined with PAGE analysis, depicted in figure 5A.

Figure 6 shows results from NGS. 150 base pair PCR amplicons from cells that were transfected with empty vector pcDNA3 or with regular Cas9 or with Cas9, cooexpressed with EXOIII or with Cas9-P4 and P3-EXOIII were sequenced. In all cases gRNA targeting human *MYD88* gene was used. No base pair deletion was observed in sample where only pcDNA3 was expressed. Nine base pair deletion was observed in sample where only Cas9 and gRNA was expressed. Fourteen base pair deletion was observed in sample where Cas9 and gRNA was coexpressed with EXOIII. Forty base pair deletion was observed in sample where Cas9-P4 tethered with P3-EXOIII and gRNA was expressed. Sign (i) depicts the predicted cleavage site for Cas9.

### Example 4: Genome editing of modified CRISPR/Cas system tethered with certain exonucleases via heterodimerizing peptide pairs is not due to the higher Cas9 protein expression

Higher percentage of genome editing for desired GOI can be enhanced due to higher cas9 protein expression. Genome editing of modified CRISPR/Cas system tethered with certain exonucleases via heterodimerizing peptide pairs is not due to the higher Cas9 protein expression.

To experimentally determine that higher genome editing of modified CRISPR/Cas system tethered with certain exonucleases via heterodimerizing peptide pairs is not due to Cas9 protein overexpression we visualized Cas9 protein content via western blot and anti-Cas9 specific immunodetection. To show the same Cas9 protein expression, HEK293 cells were transfected with constructs that express empty pcDNA3 plasmid, CRISPR/Cas system tethered with certain exonucleases via heterodimerizing peptide pairs; for example Cas9-P3 with combination of P4-EXOIII. For control, cells were transfected with regular CRISPR/Cas9 system or were cooexpressed with CRISPR/Cas9 system and certain exonuclese (e.g. EXOIII). gRNA for human *MYD88* (target sequence: *GGCTGAGAAGCCTTTACAGG*) was used. 48 hours cell lysates were prepared. The same amount of protein, determined by standard BCA assay, was analyzed on SDS PAGE gel analysis. Next, western blot transfer was performed and afterwards the membrane was stained with specific anti-Cas9 antibodies.

### Result:

Figure 7 shows that Cas9 protein content is equal in all tested samples. The band intensity of Cas9 protein is the same for cell lysates of cells, which were transfected with regular CRISPR/Cas9 system, where only Cas9 and gRNA are expressed or for cells that were used for cooexpression of Cas9 and certain exonuclease (e.g. EXOIII) and gRNA and for cells that were transfected with CRISPR/Cas system tethered with certain exonucleases via heterodimerizing peptide pairs; for example Cas9-P3 with combination of P4-EXOIII. In sample where only empty vector was transfected Cas9 protein band is absent.

### Example 5: Higher genome editing due to the modified CRISPR/Cas system tethered with certain exonucleases via heterodimerizing peptide pairs does not increase unwanted genome modification at off-target sites

When using CRISPR/Cas system with enhanced functionality there are some possibilities that increased on-target action of CRISPR/Cas bears also additional amplified off-target effect. To show that the modified CRISPR/Cas system tethered with certain exonucleases via heterodimerizing peptide pairs does not increase unwanted genome modification at off-target sites HEK293 cells where transfected with designated constructs for CRISPR/Cas. With standard T7E1 assay 48 hours after transfection potential three off-target sites for *MYD88* gRNA (target sequence: *GGCTGAGAAGCCTTTACAGG*) were analyzed. Due to bioinformatics tool for gRNA design and scoring three potential off-target sites for *MYD88* gRNA were predicted, for example: off-target site 1 was found in *ANKRD52* gene (target sequence: *ACTGTAAAGGCTGCTCTCCC*); off-target site 2 was predicted to be in *FUT9* gene (target sequence: *TGCAGAGGAGCCTTTACATG*) and off-target site 3 was determined in *PSKH2* gene (target sequence: *GCCAGACAAGGCTTTACAGG*)*.*

### Result:

PAGE gel analysis of T7E1 treated samples from transfected cells with appropriate constructs depicted in Figure 8 indicate that genome editing of three predicted possible off-target sites for human *MYD88* gene gRNA (target sequence: *GGCTGAGAAGCCTTTACAGG*) did not happen. In figure 8A PAGE gel analysis of T7E1 treated samples from transfected cells for off-target site 1 that was found in *ANKRD52* gene (target sequence: *ACTGTAAAGGCTGCTCTCCC*) is depicted. There is no indel mutation present due to the absence of DNA bands cleavage. There is no DNA bands cleavage in samples, which were transfected with empty (pcDNA3) vector or with regular CRISPR/Cas9 system, where only Cas9 and gRNA are expressed. No DNA bands cleavage is seen also in cells, that were used for cooexpression of Cas9 and certain exonuclease (e.g. EXOIII) and gRNA and for cells that were transfected with CRISPR/Cas system tethered with certain exonucleases via heterodimerizing peptide pairs; for example Cas9-P3 with combination of P4-EXOIII.

In figure 8B PAGE gel analysis of T7E1 treated samples from transfected cells for off-target site 2 that was found in *FUT9* gene (target sequence: *TGCAGAGGAGCCTTTACATG*) is depicted. There is no indel mutation present due to the absence of DNA bands cleavage. There is no DNA bands cleavage in samples, which were transfected with empty (pcDNA3) vector or with regular CRISPR/Cas9 system, where only Cas9 and gRNA are expressed. No DNA bands cleavage is seen also in cells, that were used for cooexpression of Cas9 and certain exonuclease (e.g. EXOIII) and gRNA and for cells that were transfected with CRISPR/Cas system tethered with certain exonucleases via heterodimerizing peptide pairs; for example Cas9-P3 with combination of P4-EXOIII.

In figure 8C PAGE gel analysis of T7E1 treated samples from transfected cells for off-target site 3 that was found in *PSKH2* gene (target sequence: *GCCAGACAAGGCTTTACAGG*) is depicted. There are DNA bands cleavage present, but there is also DNA bands cleavage in samples, which were transfected with empty (pcDNA3) vector or with regular CRISPR/Cas9 system, where only Cas9 and gRNA are expressed, thus suggesting presence of SNP in the tested region. DNA bands cleavage are seen also in cells, that were used for cooexpression of Cas9 and certain exonuclease (e.g. EXOIII) and gRNA and for cells that were transfected with CRISPR/Cas system tethered with certain exonucleases via heterodimerizing peptide pairs; for example Cas9-P3 with combination of P4-EXOIII, but the band intensities, which is the criterion for indel mutation determination, are the same in all samples.

### Example 6: Modified CRISPR/Cas system tethered with certain exonucleases via heterodimerizing peptide pairs poses anticancer therapeutical properties

Modified CRISPR/Cas system tethered with certain exonucleases via heterodimerizing peptide pairs poses anticancer therapeutical properties when the cause of cancer is in genomic rearrangements. CML (chronic myelogenous leukemia) patients in all cases bear Philadelphia chromosome or *BCR-ABL* fusion gene (t9, 22; q34;q11) between chromosome 9 and 22. This chromosome has the causative role for coding a hybrid kinase, a tyrosine kinase signaling protein. Constitutive signaling of this kinase is causing cells uncontrollably to divide and proliferate. To determine the possible anti-CML therapeutic option, K562 cells (stably expressing firefly luciferase; K562-fLUC), which carry *BCR-ABL* fusion gene were electroporated with plasmid DNA of designated CRISPR/Cas9 constructs. gRNA with targeting sequence *GACCTGTCTTTTAGACAGGC* for leading Cas9 to *BCR-ABL* fusion gene was used. 72 hours after electroporation of K562-fLUC cells, D-luciferin (Xenogen) was added and bioluminescence was measured by using IVIS® Lumina Series III (Perkin Elmer). Data were analyzed with Living Image® 4.5.2 (Perkin Elmer).

To test the activity of modified CRISPR/Cas system tethered with certain exonucleases via heterodimerizing peptide pairs K562 cells were used to determine the activity of modified CRISPR/Cas system tethered with certain exonucleases via heterodimerizing peptide pairs. Cells were electroporated with constructs that express CRISPR/Cas system tethered with certain exonucleases via heterodimerizing peptide pairs; for example Cas9-P3 or Cas9-P4 or Cas9-N5 with combination of P4-EXOIII or P3-EXOIII or N6-EXOIII. For control, cells were electroporated with regular CRISPR/Cas9 system or were cooexpressed with CRISPR/Cas9 system and certain exonuclease (e.g. EXOIII). gRNA for human *BCR-ABL* fusion gene (targeting sequence *GACCTGTCTTTTAGACAGGC*) was used. 48 hours later genomic DNA from cells was isolated and genomic region around predicted DSB was PCR amplified. Next T7E1 assay was carried out. Presence and quantification of indel mutation was determined by Syber gold stained PAGE (polyacrylamide gel electrophoresis) gel analysis. Percentage of indel mutations was calculated by ImageJ software.

### Result:

Figure 9 shows bioluminescence of cell population of K562-fLUC cells after CRISPR/Cas constructs electroporation. Bioluminescence reduction demonstrates cell death due to genomic editing at *BCR-ABL* fusion gene genomic area. Drop of bioluminescent signal is seen in cells that are electroporated with regular CRISPR/Cas9 system, where only Cas9 and gRNA are expressed, compared to cells, electroporated with empty pcDNA3 vector. Additional drop of bioluminescence is observed when cooexpression of Cas9 and certain exonuclease (e.g. EXOIII) and gRNA is used. The biggest loss of bioluminescent signal due to the highest genome editing at *BCR-ABL* fusion gene genomic area and consequently cell death is observed in cells that were electroporated with CRISPR/Cas system tethered with certain exonucleases via heterodimerizing peptide pairs; for example Cas9-P3 or Cas9-P4 with combination of P4-EXOIII or P3-EXOIII and corresponding gRNA.

PAGE gel analysis of T7E1 treated samples from transfected cells with appropriate constructs in Figure 10A indicate that genome editing of human *BCR-ABL* fusion gene gene was highest among all tested samples when Cas9-N5 tethered to EXOIII via N6 peptide was used. A modest decrease in genome editing was observed when cells were transfected with constructs, expressing Cas9-P3 and P4-EXOIII or in cell samples with cooexpression of Cas9, gRNA targeting human *BCR-ABL* gene and certain exonucleases (e.g. EXOIII), but nevertheless in all samples genome editing was higher when only Cas9 and gRNA were used. No gene editing occurred when cells were electroporated with empty vector. Figure 10B graphically shows percentage of indel presence, which were determined with PAGE analysis, depicted in figure 10A.

The Invention will be further described by the following items:
1. Modified CRISPR/Cas system tethered with certain exonucleases via heterodimerizing systems comprising two components:
   a) a first component comprising a Cas protein, fused with at least one first protein domain of a chemical and non-chemical signal-inducible heterodimerization protein complex, and
   b) a second component comprising a certain exonuclease, fused to a second protein domain that binds to the at least one first protein domain of the chemical and non-chemical signal-inducible heterodimerization protein complex.
2. Modified CRISPR/Cas system tethered with certain exonucleases via heterodimerizing peptide pairs that form coiled-coils comprising two components:
   a) a first component comprising a Cas protein, fused with at least one first peptide of peptide pair that form coiled-coils, and
   b) a second component comprising a certain exonuclease, fused to a second peptide of peptide pair that form coiled-coils.
3. Modified CRISPR/Cas system tethered with certain exonucleases via heterodimerizing systems of item 1, wherein the at least one first protein domain of the chemical and non-chemical signal-inducible heterodimerization protein complex of the first component is selected from the group consisting of the pairs:
   i) FKBP and FRB;
   ii) FKBP and calcineurin catalytic subunit A (CnA);
   iii) FKBP and cyclophilin;
   iv) gyrase B (GyrB) and GyrB;
   v) DmrA and DmrC;
   vi) ABI and PYL1;
   vii) GAI and GID1;
   viii) CIB1 and CRY2
   ix) LOV and PDZ;
   x) PIF and PHYB;
   xi) FKF1-GI
   xii) UVR8-COP1.
   wherein the second protein domain of the second component is selected from the group consisting of the pairs:
   i) FKBP and FRB;
   ii) FKBP and calcineurin catalytic subunit A (CnA);
   iii) FKBP and cyclophilin;
   iv) gyrase B (GyrB) and GyrB;
   v) DmrA and DmrC;
   vi) ABI and PYL1;
   vii) GAI and GID1;
   viii) CIB1 and CRY2
   ix) LOV and PDZ;
   x) PIF and PHYB;
   xi) FKF1-GI
   xii) UVR8-COP1.
4. Modified CRISPR/Cas system tethered with certain exonucleases via heterodimerizing peptide pairs of item 2, wherein peptide pairs are selected from any peptides natural and non-natural origin that form coiled-coils.
5. The Modified CRISPR/Cas system tethered with certain exonucleases via heterodimerizing systems of items 1 and 3 or the modified CRISPR/Cas system tethered with certain exonucleases via heterodimerizing peptide pairs that form coiled-coils of items 2 and 4, uses endonuclease or one or more nickase from any CRISPR type systems.
6. The Modified CRISPR/Cas system tethered with certain exonucleases via heterodimerizing systems of items 1 and 3 and 5 or the modified CRISPR/Cas system tethered with certain exonucleases via heterodimerizing peptide pairs that form coiled-coils of items 2 and 4-5, uses endonuclease or one or more nickase from any CRISPR type systems that cause one or more DSBs or one or more DNA nicks.
7. The Modified CRISPR/Cas system tethered with certain exonucleases via heterodimerizing systems of items 1 and 3 and 5-6 or the modified CRISPR/Cas system tethered with certain exonucleases via heterodimerizing peptide pairs that form coiled-coils of items 2 and 4-6, uses any enzyme that recise DNA strands in 3' and/or 5' manner.
8. The Modified CRISPR/Cas system tethered with certain exonucleases via heterodimerizing systems of items 6 and 7 or the modified CRISPR/Cas system tethered with certain exonucleases via heterodimerizing peptide pairs that form coiled-coils of items 6 and 7, influence genome editing at any selected genomic regions.
9. The Modified CRISPR/Cas system tethered with certain exonucleases via heterodimerizing systems of items 8 or the modified CRISPR/Cas system tethered with certain exonucleases via heterodimerizing peptide pairs that form coiled-coils of items 8, influence genome editing at any selected genomic regions with any designed one or more gRNA.
10. The modified CRISPR/Cas system tethered with certain exonucleases via heterodimerizing systems of items 8 and 9 or the modified CRISPR/Cas system tethered with certain exonucleases via heterodimerizing peptide pairs that form coiled-coils of items 8 and 9, influence genome editing in prokaryotic and eukaryotic cells.
11. DSBs developed after function of modified CRISPR/Cas system tethered with certain exonucleases via heterodimerizing systems of items 10 or the modified CRISPR/Cas system tethered with certain exonucleases via heterodimerizing peptide pairs that form coiled-coils of items 10 repair in any cell-repair mechanisms.
12. The modified CRISPR/Cas system tethered with certain exonucleases via heterodimerizing systems of items 11 or the modified CRISPR/Cas system tethered with certain exonucleases via heterodimerizing peptide pairs that form coiled-coils of items 11 is used for knockin and knockout cell or organisms production.
13. For knockin production modified CRISPR/Cas system tethered with certain exonucleases via heterodimerizing systems of items 11 or the modified CRISPR/Cas system tethered with certain exonucleases via heterodimerizing peptide pairs that form coiled-coils of items 11 additional DNA template is present.
14. DNA template of item 13 is present as a ssDNA or dsDNA.
15. The modified CRISPR/Cas system tethered with certain exonucleases via heterodimerizing systems of items 1 ad 4 and 6-14 comprises a chemical and non-chemical inductor of heterodimerization capable of inducing functional reconstitution of the modified CRISPR/Cas system tethered with exonucleases.
16. The regulatory system of items 1 and 3, wherein the at least one first protein domain and the second protein domain are from two different proteins and have orthologous properties.
17. The peptide pairs of item 2 and 4 form coiled-coils in parallel and anti-parallel formation.
18. The heterodimerizing system of items 1 and 4 and 6-16, wherein the first and second component form a heterodimer in the presence of a chemical inductor of heterodimerization to form the chemical signal-inducible heterodimerization protein complex, wherein the chemical inductor preferably is a small molecule.
19. The heterodimerizing system of items 1 and 4 and 6-16, wherein the first and second component form a heterodimer in the presence of a non-chemical inductor of heterodimerization to form the non-chemical signal-inducible heterodimerization protein complex, wherein the non-chemical inductor preferably is a light with designated wavelength, appropriate for heterodimerizing system protein partners.
20. The modified CRISPR/Cas system tethered with certain exonucleases via heterodimerizing systems of items 12 or the modified CRISPR/Cas system tethered with certain exonucleases via heterodimerizing peptide pairs that form coiled-coils of items 12 edits BCR-ABL fusion gene, thereby resulting in decreased deviation and proliferation of cells.
21. An isolated nucleic acid comprising a nucleic acid encoding a) the first component and/or b) the second component of the with certain exonucleases via heterodimerizing systems of items 1-20 or the modified CRISPR/Cas system tethered with certain exonucleases via heterodimerizing peptide pairs that form coiled-coils of items 1-20.
22. A vector encoding the isolated nucleic acid(s) of item 21.
23. Delivery of modified CRISPR/Cas system tethered with certain exonucleases via heterodimerizing systems of items 1-20 or the modified CRISPR/Cas system tethered with certain exonucleases via heterodimerizing peptide pairs that form coiled-coils of items 1-20 as CasRNPs.
24. A cell or organism comprising the system of items 1-23.
25. The cell or organism from item 24 for use as a medicament, disese model, drug development model, biofuel production, new food discoveries, method for treating cancer, method for treating genomic mutation derived diseases.
26. The heterodimerization systems of item 3 and heterodimerizing peptide pairs that form coiled-coils from calim 4 can be used for tethering dCas9 or Cas9 nickase with cytidine deaminase APOBEC.

## Claims

1. Combination comprising a first component (a) and a second component (b), wherein
a) the first component comprises an endonuclease capable of inducing a double stranded DNA break or a nickase capable of inducing a DNA nick, preferably a Cas protein or mutant thereof, fused with a first protein or protein domain, and
b) the second component comprises an exonuclease or a cytidine deaminase, preferably APOBEC protein, fused to a second protein or protein domain,
wherein the first and the second components are capable of heterodimerizing with each other via interaction of the first protein or protein domain and the second protein or protein domain.

2. The combination according to claim 1, wherein heterodimerization of the first protein or protein domain and the second protein or domain is inducible via a chemical or non-chemical signal.

3. The combination according to claim 2, wherein the chemical inducing heterodimerization is a small molecule such as rapalog, gibberellin or abscisic acid or any known small molecule.

4. The combination according to claim 2, wherein the non-chemical signal inducing heterodimerization is light with a predetermined wave length.

5. The combination according to any one of the preceeding claims, wherein the first protein or protein domain in combination with the second protein or domain forms a coiled-coil structure, in particular a coiled-coil structure in parallel or antiparallel orientation.

6. The combination according to any one of claims 1-4, wherein the first protein or protein domain and the second protein or domain are selected from
i) FKBP and FRB;
ii) FKBP and calcineurin catalytic subunit A (CnA);
iii) FKBP and cyclophilin;
iv) gyrase B (GyrB) and GyrB;
v) DmrA and DmrC;
vi) ABI and PYL1;
vii) GAI and GID1;
viii) CIB1 and CRY2;
ix) LOV and PDZ;
x) PIF and PHYB;
xi) FKF1-GI; and
xii) UVR8-COP1.

7. The combination according to any one of the preceeding claims, further comprising a donor DNA molecule, which is a single-stranded or double-stranded DNA molecule, particularly a single-stranded DNA molecule, wherein the donor DNA molecule may carry a desired mutation.

8. The combination according to any one of claims 1-7, wherein the first protein or protein domain in combination with the second protein or protein domain forms a coiled-coil structure, wherein the first component comprises Cas9 or Cas9 nickase and the second component comprises an exonuclease, or wherein the first component comprises dCas9 and the second component comprises a cytidine deaminase, preferably APOBEC.

9. A nucleic acid molecule, or a combination of nucleic acid molecules, comprising a first nucleic acid sequence encoding the first component (a) and a second nucleic acid sequence encoding the second component (b) of the combination according to any one of claims 8.

10. A vector comprising the nucleic acid molecule(s) according to claim 9.

11. A cell or organism comprising the combination according to any one of claims 1-8, the nucleic acid molecule(s) according to claim 8 or the vector according to claim 10, provided that the cell is not a human germ line cell.

12. A combination according to any one of claims 1-8, a nucleic acid molecule according to claim 9, a vector according to claim 10, or a cell or organism according to claim 11, for use in medicine, in particular in a method comprising genome editing in a eukaryotic target cell or in a eukaryotic target organism.

13. The combination, nucleic acid molecule, vector, cell or organism for the use according to claim 12, wherein the target cell is a vertebrate target cell, in particular a mammalian target cell, preferably a human target cell, for example a stem cell including an induced or embryonic pluripotent stem cell of an eukaryotic target organism, particularly a human induced or embryonic pluripotent stem cell, provided that the cell is not a human germ line cell, or wherein the target organism is a mammalian target organism, particularly a human.

14. The combination, nucleic acid molecule, vector, cell or organism for the use according to claim 12 or 13, wherein the genome editing comprises introducing a donor DNA molecule optionally carrying a desired mutation, which is a single-stranded or double-stranded DNA molecule, particularly a single-stranded DNA molecule, into the target cell or target organism.

15. A method for editing the genome of a eukaryotic target cell or eukaryotic target organism comprising introducing a combination as defined in any one of claims 1- 8, a nucleic acid molecule according to claim 9, or a vector according to claim 10 into the target cell or target organism, provided that the cell is not a human germ line cell.

16. In vitro use of a combination as defined in any one of claims 1-8, a nucleic acid molecule according to claim 9, or a vector according to claim 10, for genome editing in a eukaryotic target cell, particularly in a mammalian target cell, more particularly in a human target cell, provided that the cell is not a human germ line cell.


SEQUENCE LISTING

<110>  National Institute of Chemistry

<120>  Coiled-coil mediated tethering of CRISPR/CAS and exonucleases for
       enhanced genome editing

<130>  69116P EP

<160>  18

<170>  PatentIn version 3.5

<210>  1
<211>  4308
<212>  DNA
<213>  Artificial Sequence

<220>
<223>  Cas9-P4

<400>  1
atggccccaa agaagaagcg gaaggtcggt atccacggag tcccagcagc cgacaagaag      60

tacagcatcg gcctggacat cggcaccaac tctgtgggct gggccgtgat caccgacgag     120

tacaaggtgc ccagcaagaa attcaaggtg ctgggcaaca ccgaccggca cagcatcaag     180

aagaacctga tcggagccct gctgttcgac agcggcgaaa cagccgaggc cacccggctg     240

aagagaaccg ccagaagaag atacaccaga cggaagaacc ggatctgcta tctgcaagag     300

atcttcagca acgagatggc caaggtggac gacagcttct tccacagact ggaagagtcc     360

ttcctggtgg aagaggataa gaagcacgag cggcacccca tcttcggcaa catcgtggac     420

gaggtggcct accacgagaa gtaccccacc atctaccacc tgagaaagaa actggtggac     480

agcaccgaca aggccgacct gcggctgatc tatctggccc tggcccacat gatcaagttc     540

cggggccact tcctgatcga gggcgacctg aaccccgaca acagcgacgt ggacaagctg     600

ttcatccagc tggtgcagac ctacaaccag ctgttcgagg aaaaccccat caacgccagc     660

ggcgtggacg ccaaggccat cctgtctgcc agactgagca agagcagacg gctggaaaat     720

ctgatcgccc agctgcccgg cgagaagaag aatggcctgt tcggaaacct gattgccctg     780

agcctgggcc tgacccccaa cttcaagagc aacttcgacc tggccgagga tgccaaactg     840

cagctgagca aggacaccta cgacgacgac ctggacaacc tgctggccca gatcggcgac     900

cagtacgccg acctgtttct ggccgccaag aacctgtccg acgccatcct gctgagcgac     960

atcctgagag tgaacaccga gatcaccaag gcccccctga gcgcctctat gatcaagaga 1020

tacgacgagc accaccagga cctgaccctg ctgaaagctc tcgtgcggca gcagctgcct 1080

gagaagtaca aagagatttt cttcgaccag agcaagaacg gctacgccgg ctacattgac 1140

ggcggagcca gccaggaaga gttctacaag ttcatcaagc ccatcctgga aaagatggac 1200

ggcaccgagg aactgctcgt gaagctgaac agagaggacc tgctgcggaa gcagcggacc 1260

ttcgacaacg gcagcatccc ccaccagatc cacctgggag agctgcacgc cattctgcgg 1320

cggcaggaag atttttaccc attcctgaag gacaaccggg aaaagatcga gaagatcctg 1380

accttccgca tcccctacta cgtgggccct ctggccaggg gaaacagcag attcgcctgg 1440

atgaccagaa agagcgagga aaccatcacc ccctggaact tcgaggaagt ggtggacaag 1500

ggcgcttccg cccagagctt catcgagcgg atgaccaact tcgataagaa cctgcccaac 1560

gagaaggtgc tgcccaagca cagcctgctg tacgagtact tcaccgtgta taacgagctg 1620

accaaagtga aatacgtgac cgagggaatg agaaagcccg ccttcctgag cggcgagcag 1680

aaaaaggcca tcgtggacct gctgttcaag accaaccgga aagtgaccgt gaagcagctg 1740

aaagaggact acttcaagaa aatcgagtgc ttcgactccg tggaaatctc cggcgtggaa 1800

gatcggttca acgcctccct gggcacatac cacgatctgc tgaaaattat caaggacaag 1860

gacttcctgg acaatgagga aaacgaggac attctggaag atatcgtgct gaccctgaca 1920

ctgtttgagg acagagagat gatcgaggaa cggctgaaaa cctatgccca cctgttcgac 1980

gacaaagtga tgaagcagct gaagcggcgg agatacaccg gctggggcag gctgagccgg 2040

aagctgatca acggcatccg ggacaagcag tccggcaaga caatcctgga tttcctgaag 2100

tccgacggct tcgccaacag aaacttcatg cagctgatcc acgacgacag cctgaccttt 2160

aaagaggaca tccagaaagc ccaggtgtcc ggccagggcg atagcctgca cgagcacatt 2220

gccaatctgg ccggcagccc cgccattaag aagggcatcc tgcagacagt gaaggtggtg 2280

gacgagctcg tgaaagtgat gggccggcac aagcccgaga acatcgtgat cgaaatggcc 2340

agagagaacc agaccaccca gaagggacag aagaacagcc gcgagagaat gaagcggatc 2400

gaagagggca tcaaagagct gggcagccag atcctgaaag aacaccccgt ggaaaacacc 2460

cagctgcaga acgagaagct gtacctgtac tacctgcaga atgggcggga tatgtacgtg 2520

gaccaggaac tggacatcaa ccggctgtcc gactacgatg tggaccatat cgtgcctcag 2580

agctttctga aggacgactc catcgacaac aaggtgctga ccagaagcga caagaaccgg 2640

ggcaagagcg acaacgtgcc ctccgaagag gtcgtgaaga agatgaagaa ctactggcgg 2700

cagctgctga acgccaagct gattacccag agaaagttcg acaatctgac caaggccgag 2760

agaggcggcc tgagcgaact ggataaggcc ggcttcatca agagacagct ggtggaaacc 2820

cggcagatca caaagcacgt ggcacagatc ctggactccc ggatgaacac taagtacgac 2880

gagaatgaca agctgatccg ggaagtgaaa gtgatcaccc tgaagtccaa gctggtgtcc 2940

gatttccgga aggatttcca gttttacaaa gtgcgcgaga tcaacaacta ccaccacgcc 3000

cacgacgcct acctgaacgc cgtcgtggga accgccctga tcaaaaagta ccctaagctg 3060

gaaagcgagt tcgtgtacgg cgactacaag gtgtacgacg tgcggaagat gatcgccaag 3120

agcgagcagg aaatcggcaa ggctaccgcc aagtacttct tctacagcaa catcatgaac 3180

tttttcaaga ccgagattac cctggccaac ggcgagatcc ggaagcggcc tctgatcgag 3240

acaaacggcg aaaccgggga gatcgtgtgg gataagggcc gggattttgc caccgtgcgg 3300

aaagtgctga gcatgcccca agtgaatatc gtgaaaaaga ccgaggtgca gacaggcggc 3360

ttcagcaaag agtctatcct gcccaagagg aacagcgata agctgatcgc cagaaagaag 3420

gactgggacc ctaagaagta cggcggcttc gacagcccca ccgtggccta ttctgtgctg 3480

gtggtggcca aagtggaaaa gggcaagtcc aagaaactga agagtgtgaa agagctgctg 3540

gggatcacca tcatggaaag aagcagcttc gagaagaatc ccatcgactt tctggaagcc 3600

aagggctaca aagaagtgaa aaaggacctg atcatcaagc tgcctaagta ctccctgttc 3660

gagctggaaa acggccggaa gagaatgctg gcctctgccg gcgaactgca gaagggaaac 3720

gaactggccc tgccctccaa atatgtgaac ttcctgtacc tggccagcca ctatgagaag 3780

ctgaagggct cccccgagga taatgagcag aaacagctgt ttgtggaaca gcacaagcac 3840

tacctggacg agatcatcga gcagatcagc gagttctcca agagagtgat cctggccgac 3900

gctaatctgg acaaagtgct gtccgcctac aacaagcacc gggataagcc catcagagag 3960

caggccgaga atatcatcca cctgtttacc ctgaccaatc tgggagcccc tgccgccttc 4020

aagtactttg acaccaccat cgaccggaag aggtacacca gcaccaaaga ggtgctggac 4080

gccaccctga tccaccagag catcaccggc ctgtacgaga cacggatcga cctgtctcag 4140

ctgggaggcg acgatccaaa aaagaagaga aaggtaggcg gctctggcgg cggctccgga 4200

ggctctagcc cggaagataa aattgctcag ctgaaacaaa aaatccaagc gctgaaacag 4260

gaaaaccagc agctggaaga ggaaaacgcc gcactggaat atggttaa 4308

<210> 2
<211> 4308
<212> DNA
<213> Artificial Sequence

<220>
<223> Cas9-P3

<400> 2
atggccccaa agaagaagcg gaaggtcggt atccacggag tcccagcagc cgacaagaag 60

tacagcatcg gcctggacat cggcaccaac tctgtgggct gggccgtgat caccgacgag 120

tacaaggtgc ccagcaagaa attcaaggtg ctgggcaaca ccgaccggca cagcatcaag 180

aagaacctga tcggagccct gctgttcgac agcggcgaaa cagccgaggc cacccggctg 240

aagagaaccg ccagaagaag atacaccaga cggaagaacc ggatctgcta tctgcaagag 300

atcttcagca acgagatggc caaggtggac gacagcttct tccacagact ggaagagtcc 360

ttcctggtgg aagaggataa gaagcacgag cggcacccca tcttcggcaa catcgtggac 420

gaggtggcct accacgagaa gtaccccacc atctaccacc tgagaaagaa actggtggac 480

agcaccgaca aggccgacct gcggctgatc tatctggccc tggcccacat gatcaagttc 540

cggggccact tcctgatcga gggcgacctg aaccccgaca acagcgacgt ggacaagctg 600

ttcatccagc tggtgcagac ctacaaccag ctgttcgagg aaaaccccat caacgccagc 660

ggcgtggacg ccaaggccat cctgtctgcc agactgagca agagcagacg gctggaaaat 720

ctgatcgccc agctgcccgg cgagaagaag aatggcctgt tcggaaacct gattgccctg 780

agcctgggcc tgacccccaa cttcaagagc aacttcgacc tggccgagga tgccaaactg 840

cagctgagca aggacaccta cgacgacgac ctggacaacc tgctggccca gatcggcgac 900

cagtacgccg acctgtttct ggccgccaag aacctgtccg acgccatcct gctgagcgac 960

atcctgagag tgaacaccga gatcaccaag gcccccctga gcgcctctat gatcaagaga 1020

tacgacgagc accaccagga cctgaccctg ctgaaagctc tcgtgcggca gcagctgcct 1080

gagaagtaca aagagatttt cttcgaccag agcaagaacg gctacgccgg ctacattgac 1140

ggcggagcca gccaggaaga gttctacaag ttcatcaagc ccatcctgga aaagatggac 1200

ggcaccgagg aactgctcgt gaagctgaac agagaggacc tgctgcggaa gcagcggacc 1260

ttcgacaacg gcagcatccc ccaccagatc cacctgggag agctgcacgc cattctgcgg 1320

cggcaggaag atttttaccc attcctgaag gacaaccggg aaaagatcga gaagatcctg 1380

accttccgca tcccctacta cgtgggccct ctggccaggg gaaacagcag attcgcctgg 1440

atgaccagaa agagcgagga aaccatcacc ccctggaact tcgaggaagt ggtggacaag 1500

ggcgcttccg cccagagctt catcgagcgg atgaccaact tcgataagaa cctgcccaac 1560

gagaaggtgc tgcccaagca cagcctgctg tacgagtact tcaccgtgta taacgagctg 1620

accaaagtga aatacgtgac cgagggaatg agaaagcccg ccttcctgag cggcgagcag 1680

aaaaaggcca tcgtggacct gctgttcaag accaaccgga aagtgaccgt gaagcagctg 1740

aaagaggact acttcaagaa aatcgagtgc ttcgactccg tggaaatctc cggcgtggaa 1800

gatcggttca acgcctccct gggcacatac cacgatctgc tgaaaattat caaggacaag 1860

gacttcctgg acaatgagga aaacgaggac attctggaag atatcgtgct gaccctgaca 1920

ctgtttgagg acagagagat gatcgaggaa cggctgaaaa cctatgccca cctgttcgac 1980

gacaaagtga tgaagcagct gaagcggcgg agatacaccg gctggggcag gctgagccgg 2040

aagctgatca acggcatccg ggacaagcag tccggcaaga caatcctgga tttcctgaag 2100

tccgacggct tcgccaacag aaacttcatg cagctgatcc acgacgacag cctgaccttt 2160

aaagaggaca tccagaaagc ccaggtgtcc ggccagggcg atagcctgca cgagcacatt 2220

gccaatctgg ccggcagccc cgccattaag aagggcatcc tgcagacagt gaaggtggtg 2280

gacgagctcg tgaaagtgat gggccggcac aagcccgaga acatcgtgat cgaaatggcc 2340

agagagaacc agaccaccca gaagggacag aagaacagcc gcgagagaat gaagcggatc 2400

gaagagggca tcaaagagct gggcagccag atcctgaaag aacaccccgt ggaaaacacc 2460

cagctgcaga acgagaagct gtacctgtac tacctgcaga atgggcggga tatgtacgtg 2520

gaccaggaac tggacatcaa ccggctgtcc gactacgatg tggaccatat cgtgcctcag 2580

agctttctga aggacgactc catcgacaac aaggtgctga ccagaagcga caagaaccgg 2640

ggcaagagcg acaacgtgcc ctccgaagag gtcgtgaaga agatgaagaa ctactggcgg 2700

cagctgctga acgccaagct gattacccag agaaagttcg acaatctgac caaggccgag 2760

agaggcggcc tgagcgaact ggataaggcc ggcttcatca agagacagct ggtggaaacc 2820

cggcagatca caaagcacgt ggcacagatc ctggactccc ggatgaacac taagtacgac 2880

gagaatgaca agctgatccg ggaagtgaaa gtgatcaccc tgaagtccaa gctggtgtcc 2940

gatttccgga aggatttcca gttttacaaa gtgcgcgaga tcaacaacta ccaccacgcc 3000

cacgacgcct acctgaacgc cgtcgtggga accgccctga tcaaaaagta ccctaagctg 3060

gaaagcgagt tcgtgtacgg cgactacaag gtgtacgacg tgcggaagat gatcgccaag 3120

agcgagcagg aaatcggcaa ggctaccgcc aagtacttct tctacagcaa catcatgaac 3180

tttttcaaga ccgagattac cctggccaac ggcgagatcc ggaagcggcc tctgatcgag 3240

acaaacggcg aaaccgggga gatcgtgtgg gataagggcc gggattttgc caccgtgcgg 3300

aaagtgctga gcatgcccca agtgaatatc gtgaaaaaga ccgaggtgca gacaggcggc 3360

ttcagcaaag agtctatcct gcccaagagg aacagcgata agctgatcgc cagaaagaag 3420

gactgggacc ctaagaagta cggcggcttc gacagcccca ccgtggccta ttctgtgctg 3480

gtggtggcca aagtggaaaa gggcaagtcc aagaaactga agagtgtgaa agagctgctg 3540

gggatcacca tcatggaaag aagcagcttc gagaagaatc ccatcgactt tctggaagcc 3600

aagggctaca aagaagtgaa aaaggacctg atcatcaagc tgcctaagta ctccctgttc 3660

gagctggaaa acggccggaa gagaatgctg gcctctgccg gcgaactgca gaagggaaac 3720

gaactggccc tgccctccaa atatgtgaac ttcctgtacc tggccagcca ctatgagaag 3780

ctgaagggct cccccgagga taatgagcag aaacagctgt ttgtggaaca gcacaagcac 3840

tacctggacg agatcatcga gcagatcagc gagttctcca agagagtgat cctggccgac 3900

gctaatctgg acaaagtgct gtccgcctac aacaagcacc gggataagcc catcagagag 3960

caggccgaga atatcatcca cctgtttacc ctgaccaatc tgggagcccc tgccgccttc 4020

aagtactttg acaccaccat cgaccggaag aggtacacca gcaccaaaga ggtgctggac 4080

gccaccctga tccaccagag catcaccggc ctgtacgaga cacggatcga cctgtctcag 4140

ctgggaggcg acgatccaaa aaagaagaga aaggtaggcg gctctggcgg cggctccgga 4200

ggctcttccc cggaagatga gatccagcaa ctggaagaag aaatcgctca gctggaacag 4260

aaaaacgcag cgctgaaaga gaaaaaccag gcgctgaaat acggttaa 4308

<210> 3
<211> 4293
<212> DNA
<213> Artificial Sequence

<220>
<223> Cas9-N5

<400> 3
atggccccaa agaagaagcg gaaggtcggt atccacggag tcccagcagc cgacaagaag 60

tacagcatcg gcctggacat cggcaccaac tctgtgggct gggccgtgat caccgacgag 120

tacaaggtgc ccagcaagaa attcaaggtg ctgggcaaca ccgaccggca cagcatcaag 180

aagaacctga tcggagccct gctgttcgac agcggcgaaa cagccgaggc cacccggctg 240

aagagaaccg ccagaagaag atacaccaga cggaagaacc ggatctgcta tctgcaagag 300

atcttcagca acgagatggc caaggtggac gacagcttct tccacagact ggaagagtcc 360

ttcctggtgg aagaggataa gaagcacgag cggcacccca tcttcggcaa catcgtggac 420

gaggtggcct accacgagaa gtaccccacc atctaccacc tgagaaagaa actggtggac 480

agcaccgaca aggccgacct gcggctgatc tatctggccc tggcccacat gatcaagttc 540

cggggccact tcctgatcga gggcgacctg aaccccgaca acagcgacgt ggacaagctg 600

ttcatccagc tggtgcagac ctacaaccag ctgttcgagg aaaaccccat caacgccagc 660

ggcgtggacg ccaaggccat cctgtctgcc agactgagca agagcagacg gctggaaaat 720

ctgatcgccc agctgcccgg cgagaagaag aatggcctgt tcggaaacct gattgccctg 780

agcctgggcc tgacccccaa cttcaagagc aacttcgacc tggccgagga tgccaaactg 840

cagctgagca aggacaccta cgacgacgac ctggacaacc tgctggccca gatcggcgac 900

cagtacgccg acctgtttct ggccgccaag aacctgtccg acgccatcct gctgagcgac 960

atcctgagag tgaacaccga gatcaccaag gcccccctga gcgcctctat gatcaagaga 1020

tacgacgagc accaccagga cctgaccctg ctgaaagctc tcgtgcggca gcagctgcct 1080

gagaagtaca aagagatttt cttcgaccag agcaagaacg gctacgccgg ctacattgac 1140

ggcggagcca gccaggaaga gttctacaag ttcatcaagc ccatcctgga aaagatggac 1200

ggcaccgagg aactgctcgt gaagctgaac agagaggacc tgctgcggaa gcagcggacc 1260

ttcgacaacg gcagcatccc ccaccagatc cacctgggag agctgcacgc cattctgcgg 1320

cggcaggaag atttttaccc attcctgaag gacaaccggg aaaagatcga gaagatcctg 1380

accttccgca tcccctacta cgtgggccct ctggccaggg gaaacagcag attcgcctgg 1440

atgaccagaa agagcgagga aaccatcacc ccctggaact tcgaggaagt ggtggacaag 1500

ggcgcttccg cccagagctt catcgagcgg atgaccaact tcgataagaa cctgcccaac 1560

gagaaggtgc tgcccaagca cagcctgctg tacgagtact tcaccgtgta taacgagctg 1620

accaaagtga aatacgtgac cgagggaatg agaaagcccg ccttcctgag cggcgagcag 1680

aaaaaggcca tcgtggacct gctgttcaag accaaccgga aagtgaccgt gaagcagctg 1740

aaagaggact acttcaagaa aatcgagtgc ttcgactccg tggaaatctc cggcgtggaa 1800

gatcggttca acgcctccct gggcacatac cacgatctgc tgaaaattat caaggacaag 1860

gacttcctgg acaatgagga aaacgaggac attctggaag atatcgtgct gaccctgaca 1920

ctgtttgagg acagagagat gatcgaggaa cggctgaaaa cctatgccca cctgttcgac 1980

gacaaagtga tgaagcagct gaagcggcgg agatacaccg gctggggcag gctgagccgg 2040

aagctgatca acggcatccg ggacaagcag tccggcaaga caatcctgga tttcctgaag 2100

tccgacggct tcgccaacag aaacttcatg cagctgatcc acgacgacag cctgaccttt 2160

aaagaggaca tccagaaagc ccaggtgtcc ggccagggcg atagcctgca cgagcacatt 2220

gccaatctgg ccggcagccc cgccattaag aagggcatcc tgcagacagt gaaggtggtg 2280

gacgagctcg tgaaagtgat gggccggcac aagcccgaga acatcgtgat cgaaatggcc 2340

agagagaacc agaccaccca gaagggacag aagaacagcc gcgagagaat gaagcggatc 2400

gaagagggca tcaaagagct gggcagccag atcctgaaag aacaccccgt ggaaaacacc 2460

cagctgcaga acgagaagct gtacctgtac tacctgcaga atgggcggga tatgtacgtg 2520

gaccaggaac tggacatcaa ccggctgtcc gactacgatg tggaccatat cgtgcctcag 2580

agctttctga aggacgactc catcgacaac aaggtgctga ccagaagcga caagaaccgg 2640

ggcaagagcg acaacgtgcc ctccgaagag gtcgtgaaga agatgaagaa ctactggcgg 2700

cagctgctga acgccaagct gattacccag agaaagttcg acaatctgac caaggccgag 2760

agaggcggcc tgagcgaact ggataaggcc ggcttcatca agagacagct ggtggaaacc 2820

cggcagatca caaagcacgt ggcacagatc ctggactccc ggatgaacac taagtacgac 2880

gagaatgaca agctgatccg ggaagtgaaa gtgatcaccc tgaagtccaa gctggtgtcc 2940

gatttccgga aggatttcca gttttacaaa gtgcgcgaga tcaacaacta ccaccacgcc 3000

cacgacgcct acctgaacgc cgtcgtggga accgccctga tcaaaaagta ccctaagctg 3060

gaaagcgagt tcgtgtacgg cgactacaag gtgtacgacg tgcggaagat gatcgccaag 3120

agcgagcagg aaatcggcaa ggctaccgcc aagtacttct tctacagcaa catcatgaac 3180

tttttcaaga ccgagattac cctggccaac ggcgagatcc ggaagcggcc tctgatcgag 3240

acaaacggcg aaaccgggga gatcgtgtgg gataagggcc gggattttgc caccgtgcgg 3300

aaagtgctga gcatgcccca agtgaatatc gtgaaaaaga ccgaggtgca gacaggcggc 3360

ttcagcaaag agtctatcct gcccaagagg aacagcgata agctgatcgc cagaaagaag 3420

gactgggacc ctaagaagta cggcggcttc gacagcccca ccgtggccta ttctgtgctg 3480

gtggtggcca aagtggaaaa gggcaagtcc aagaaactga agagtgtgaa agagctgctg 3540

gggatcacca tcatggaaag aagcagcttc gagaagaatc ccatcgactt tctggaagcc 3600

aagggctaca aagaagtgaa aaaggacctg atcatcaagc tgcctaagta ctccctgttc 3660

gagctggaaa acggccggaa gagaatgctg gcctctgccg gcgaactgca gaagggaaac 3720

gaactggccc tgccctccaa atatgtgaac ttcctgtacc tggccagcca ctatgagaag 3780

ctgaagggct cccccgagga taatgagcag aaacagctgt ttgtggaaca gcacaagcac 3840

tacctggacg agatcatcga gcagatcagc gagttctcca agagagtgat cctggccgac 3900

gctaatctgg acaaagtgct gtccgcctac aacaagcacc gggataagcc catcagagag 3960

caggccgaga atatcatcca cctgtttacc ctgaccaatc tgggagcccc tgccgccttc 4020

aagtactttg acaccaccat cgaccggaag aggtacacca gcaccaaaga ggtgctggac 4080

gccaccctga tccaccagag catcaccggc ctgtacgaga cacggatcga cctgtctcag 4140

ctgggaggcg acgatccaaa aaagaagaga aaggtaggcg gctctggcgg cggctccgga 4200

ggctctgaga tcgccgccct ggaggccaag atcgccgccc tgaaggccaa gaacgccgcc 4260

ctgaaggccg agatcgccgc cctggaggcc taa 4293

<210> 4
<211> 4293
<212> DNA
<213> Artificial Sequence

<220>
<223> Cas9-N6

<400> 4
atggccccaa agaagaagcg gaaggtcggt atccacggag tcccagcagc cgacaagaag 60

tacagcatcg gcctggacat cggcaccaac tctgtgggct gggccgtgat caccgacgag 120

tacaaggtgc ccagcaagaa attcaaggtg ctgggcaaca ccgaccggca cagcatcaag 180

aagaacctga tcggagccct gctgttcgac agcggcgaaa cagccgaggc cacccggctg 240

aagagaaccg ccagaagaag atacaccaga cggaagaacc ggatctgcta tctgcaagag 300

atcttcagca acgagatggc caaggtggac gacagcttct tccacagact ggaagagtcc 360

ttcctggtgg aagaggataa gaagcacgag cggcacccca tcttcggcaa catcgtggac 420

gaggtggcct accacgagaa gtaccccacc atctaccacc tgagaaagaa actggtggac 480

agcaccgaca aggccgacct gcggctgatc tatctggccc tggcccacat gatcaagttc 540

cggggccact tcctgatcga gggcgacctg aaccccgaca acagcgacgt ggacaagctg 600
ttcatccagc tggtgcagac ctacaaccag ctgttcgagg aaaaccccat caacgccagc 660
ggcgtggacg ccaaggccat cctgtctgcc agactgagca agagcagacg gctggaaaat 720
ctgatcgccc agctgcccgg cgagaagaag aatggcctgt tcggaaacct gattgccctg 780
agcctgggcc tgacccccaa cttcaagagc aacttcgacc tggccgagga tgccaaactg 840
cagctgagca aggacaccta cgacgacgac ctggacaacc tgctggccca gatcggcgac 900
cagtacgccg acctgtttct ggccgccaag aacctgtccg acgccatcct gctgagcgac 960
atcctgagag tgaacaccga gatcaccaag gcccccctga gcgcctctat gatcaagaga 1020
tacgacgagc accaccagga cctgaccctg ctgaaagctc tcgtgcggca gcagctgcct 1080
gagaagtaca aagagatttt cttcgaccag agcaagaacg gctacgccgg ctacattgac 1140
ggcggagcca gccaggaaga gttctacaag ttcatcaagc ccatcctgga aaagatggac 1200
ggcaccgagg aactgctcgt gaagctgaac agagaggacc tgctgcggaa gcagcggacc 1260
ttcgacaacg gcagcatccc ccaccagatc cacctgggag agctgcacgc cattctgcgg 1320
cggcaggaag atttttaccc attcctgaag gacaaccggg aaaagatcga gaagatcctg 1380
accttccgca tcccctacta cgtgggccct ctggccaggg gaaacagcag attcgcctgg 1440
atgaccagaa agagcgagga aaccatcacc ccctggaact tcgaggaagt ggtggacaag 1500
ggcgcttccg cccagagctt catcgagcgg atgaccaact tcgataagaa cctgcccaac 1560
gagaaggtgc tgcccaagca cagcctgctg tacgagtact tcaccgtgta taacgagctg 1620
accaaagtga aatacgtgac cgagggaatg agaaagcccg ccttcctgag cggcgagcag 1680
aaaaaggcca tcgtggacct gctgttcaag accaaccgga aagtgaccgt gaagcagctg 1740
aaagaggact acttcaagaa aatcgagtgc ttcgactccg tggaaatctc cggcgtggaa 1800
gatcggttca acgcctccct gggcacatac cacgatctgc tgaaaattat caaggacaag 1860
gacttcctgg acaatgagga aaacgaggac attctggaag atatcgtgct gaccctgaca 1920
ctgtttgagg acagagagat gatcgaggaa cggctgaaaa cctatgccca cctgttcgac 1980
gacaaagtga tgaagcagct gaagcggcgg agatacaccg gctggggcag gctgagccgg 2040
aagctgatca acggcatccg ggacaagcag tccggcaaga caatcctgga tttcctgaag 2100
tccgacggct tcgccaacag aaacttcatg cagctgatcc acgacgacag cctgaccttt 2160
aaagaggaca tccagaaagc ccaggtgtcc ggccagggcg atagcctgca cgagcacatt 2220

gccaatctgg ccggcagccc cgccattaag aagggcatcc tgcagacagt gaaggtggtg 2280

gacgagctcg tgaaagtgat gggccggcac aagcccgaga acatcgtgat cgaaatggcc 2340

agagagaacc agaccaccca gaagggacag aagaacagcc gcgagagaat gaagcggatc 2400

gaagagggca tcaaagagct gggcagccag atcctgaaag aacaccccgt ggaaaacacc 2460

cagctgcaga acgagaagct gtacctgtac tacctgcaga atgggcggga tatgtacgtg 2520

gaccaggaac tggacatcaa ccggctgtcc gactacgatg tggaccatat cgtgcctcag 2580

agctttctga aggacgactc catcgacaac aaggtgctga ccagaagcga caagaaccgg 2640

ggcaagagcg acaacgtgcc ctccgaagag gtcgtgaaga agatgaagaa ctactggcgg 2700

cagctgctga acgccaagct gattacccag agaaagttcg acaatctgac caaggccgag 2760

agaggcggcc tgagcgaact ggataaggcc ggcttcatca agagacagct ggtggaaacc 2820

cggcagatca caaagcacgt ggcacagatc ctggactccc ggatgaacac taagtacgac 2880

gagaatgaca agctgatccg ggaagtgaaa gtgatcaccc tgaagtccaa gctggtgtcc 2940

gatttccgga aggatttcca gttttacaaa gtgcgcgaga tcaacaacta ccaccacgcc 3000

cacgacgcct acctgaacgc cgtcgtggga accgccctga tcaaaaagta ccctaagctg 3060

gaaagcgagt tcgtgtacgg cgactacaag gtgtacgacg tgcggaagat gatcgccaag 3120

agcgagcagg aaatcggcaa ggctaccgcc aagtacttct tctacagcaa catcatgaac 3180

tttttcaaga ccgagattac cctggccaac ggcgagatcc ggaagcggcc tctgatcgag 3240

acaaacggcg aaaccgggga gatcgtgtgg gataagggcc gggattttgc caccgtgcgg 3300

aaagtgctga gcatgcccca agtgaatatc gtgaaaaaga ccgaggtgca gacaggcggc 3360

ttcagcaaag agtctatcct gcccaagagg aacagcgata agctgatcgc cagaaagaag 3420

gactgggacc ctaagaagta cggcggcttc gacagcccca ccgtggccta ttctgtgctg 3480

gtggtggcca aagtggaaaa gggcaagtcc aagaaactga agagtgtgaa agagctgctg 3540

gggatcacca tcatggaaag aagcagcttc gagaagaatc ccatcgactt tctggaagcc 3600

aagggctaca aagaagtgaa aaaggacctg atcatcaagc tgcctaagta ctccctgttc 3660

gagctggaaa acggccggaa gagaatgctg gcctctgccg gcgaactgca gaagggaaac 3720

gaactggccc tgccctccaa atatgtgaac ttcctgtacc tggccagcca ctatgagaag 3780

ctgaagggct cccccgagga taatgagcag aaacagctgt ttgtggaaca gcacaagcac 3840

tacctggacg agatcatcga gcagatcagc gagttctcca agagagtgat cctggccgac 3900

gctaatctgg acaaagtgct gtccgcctac aacaagcacc gggataagcc catcagagag 3960

caggccgaga atatcatcca cctgtttacc ctgaccaatc tgggagcccc tgccgccttc 4020

aagtactttg acaccaccat cgaccggaag aggtacacca gcaccaaaga ggtgctggac 4080

gccaccctga tccaccagag catcaccggc ctgtacgaga cacggatcga cctgtctcag 4140

ctgggaggcg acgatccaaa aaagaagaga aaggtaggcg gctctggcgg cggctccgga 4200

ggctctaaga tcgccgccct gaaggccgag atcgccgccc tggaggccga gaacgccgcc 4260

ctggaggcca agatcgccgc cctgaaggcc taa 4293

<210> 5
<211> 4305
<212> DNA
<213> Artificial Sequence

<220>
<223> Cas9-P3S

<400> 5
atggccccaa agaagaagcg gaaggtcggt atccacggag tcccagcagc cgacaagaag 60

tacagcatcg gcctggacat cggcaccaac tctgtgggct gggccgtgat caccgacgag 120

tacaaggtgc ccagcaagaa attcaaggtg ctgggcaaca ccgaccggca cagcatcaag 180

aagaacctga tcggagccct gctgttcgac agcggcgaaa cagccgaggc cacccggctg 240

aagagaaccg ccagaagaag atacaccaga cggaagaacc ggatctgcta tctgcaagag 300

atcttcagca acgagatggc caaggtggac gacagcttct tccacagact ggaagagtcc 360

ttcctggtgg aagaggataa gaagcacgag cggcacccca tcttcggcaa catcgtggac 420

gaggtggcct accacgagaa gtaccccacc atctaccacc tgagaaagaa actggtggac 480

agcaccgaca aggccgacct gcggctgatc tatctggccc tggcccacat gatcaagttc 540

cggggccact tcctgatcga gggcgacctg aaccccgaca acagcgacgt ggacaagctg 600

ttcatccagc tggtgcagac ctacaaccag ctgttcgagg aaaaccccat caacgccagc 660

ggcgtggacg ccaaggccat cctgtctgcc agactgagca agagcagacg gctggaaaat 720

ctgatcgccc agctgcccgg cgagaagaag aatggcctgt tcggaaacct gattgccctg 780

agcctgggcc tgacccccaa cttcaagagc aacttcgacc tggccgagga tgccaaactg 840

cagctgagca aggacaccta cgacgacgac ctggacaacc tgctggccca gatcggcgac 900

cagtacgccg acctgtttct ggccgccaag aacctgtccg acgccatcct gctgagcgac 960

atcctgagag tgaacaccga gatcaccaag gcccccctga gcgcctctat gatcaagaga 1020

tacgacgagc accaccagga cctgaccctg ctgaaagctc tcgtgcggca gcagctgcct 1080

gagaagtaca aagagatttt cttcgaccag agcaagaacg gctacgccgg ctacattgac 1140

ggcggagcca gccaggaaga gttctacaag ttcatcaagc ccatcctgga aaagatggac 1200

ggcaccgagg aactgctcgt gaagctgaac agagaggacc tgctgcggaa gcagcggacc 1260

ttcgacaacg gcagcatccc ccaccagatc cacctgggag agctgcacgc cattctgcgg 1320

cggcaggaag atttttaccc attcctgaag gacaaccggg aaaagatcga gaagatcctg 1380

accttccgca tcccctacta cgtgggccct ctggccaggg gaaacagcag attcgcctgg 1440

atgaccagaa agagcgagga aaccatcacc ccctggaact tcgaggaagt ggtggacaag 1500

ggcgcttccg cccagagctt catcgagcgg atgaccaact tcgataagaa cctgcccaac 1560

gagaaggtgc tgcccaagca cagcctgctg tacgagtact tcaccgtgta taacgagctg 1620

accaaagtga aatacgtgac cgagggaatg agaaagcccg ccttcctgag cggcgagcag 1680

aaaaaggcca tcgtggacct gctgttcaag accaaccgga aagtgaccgt gaagcagctg 1740

aaagaggact acttcaagaa aatcgagtgc ttcgactccg tggaaatctc cggcgtggaa 1800

gatcggttca acgcctccct gggcacatac cacgatctgc tgaaaattat caaggacaag 1860

gacttcctgg acaatgagga aaacgaggac attctggaag atatcgtgct gaccctgaca 1920

ctgtttgagg acagagagat gatcgaggaa cggctgaaaa cctatgccca cctgttcgac 1980

gacaaagtga tgaagcagct gaagcggcgg agatacaccg gctggggcag gctgagccgg 2040

aagctgatca acggcatccg ggacaagcag tccggcaaga caatcctgga tttcctgaag 2100

tccgacggct tcgccaacag aaacttcatg cagctgatcc acgacgacag cctgaccttt 2160

aaagaggaca tccagaaagc ccaggtgtcc ggccagggcg atagcctgca cgagcacatt 2220

gccaatctgg ccggcagccc cgccattaag aagggcatcc tgcagacagt gaaggtggtg 2280

gacgagctcg tgaaagtgat gggccggcac aagcccgaga acatcgtgat cgaaatggcc 2340

agagagaacc agaccaccca gaagggacag aagaacagcc gcgagagaat gaagcggatc 2400

gaagagggca tcaaagagct gggcagccag atcctgaaag aacaccccgt ggaaaacacc 2460

cagctgcaga acgagaagct gtacctgtac tacctgcaga atgggcggga tatgtacgtg 2520

gaccaggaac tggacatcaa ccggctgtcc gactacgatg tggaccatat cgtgcctcag 2580

agctttctga aggacgactc catcgacaac aaggtgctga ccagaagcga caagaaccgg 2640

ggcaagagcg acaacgtgcc ctccgaagag gtcgtgaaga agatgaagaa ctactggcgg 2700

cagctgctga acgccaagct gattacccag agaaagttcg acaatctgac caaggccgag 2760

agaggcggcc tgagcgaact ggataaggcc ggcttcatca agagacagct ggtggaaacc 2820

cggcagatca caaagcacgt ggcacagatc ctggactccc ggatgaacac taagtacgac 2880

gagaatgaca agctgatccg ggaagtgaaa gtgatcaccc tgaagtccaa gctggtgtcc 2940

gatttccgga aggatttcca gttttacaaa gtgcgcgaga tcaacaacta ccaccacgcc 3000

cacgacgcct acctgaacgc cgtcgtggga accgccctga tcaaaaagta ccctaagctg 3060

gaaagcgagt tcgtgtacgg cgactacaag gtgtacgacg tgcggaagat gatcgccaag 3120

agcgagcagg aaatcggcaa ggctaccgcc aagtacttct tctacagcaa catcatgaac 3180

tttttcaaga ccgagattac cctggccaac ggcgagatcc ggaagcggcc tctgatcgag 3240

acaaacggcg aaaccgggga gatcgtgtgg gataagggcc gggattttgc caccgtgcgg 3300

aaagtgctga gcatgcccca agtgaatatc gtgaaaaaga ccgaggtgca gacaggcggc 3360

ttcagcaaag agtctatcct gcccaagagg aacagcgata agctgatcgc cagaaagaag 3420

gactgggacc ctaagaagta cggcggcttc gacagcccca ccgtggccta ttctgtgctg 3480

gtggtggcca aagtggaaaa gggcaagtcc aagaaactga agagtgtgaa agagctgctg 3540

gggatcacca tcatggaaag aagcagcttc gagaagaatc ccatcgactt tctggaagcc 3600

aagggctaca aagaagtgaa aaaggacctg atcatcaagc tgcctaagta ctccctgttc 3660

gagctggaaa acggccggaa gagaatgctg gcctctgccg gcgaactgca gaagggaaac 3720

gaactggccc tgccctccaa atatgtgaac ttcctgtacc tggccagcca ctatgagaag 3780

ctgaagggct cccccgagga taatgagcag aaacagctgt ttgtggaaca gcacaagcac 3840

tacctggacg agatcatcga gcagatcagc gagttctcca agagagtgat cctggccgac 3900

gctaatctgg acaaagtgct gtccgcctac aacaagcacc gggataagcc catcagagag 3960

caggccgaga atatcatcca cctgtttacc ctgaccaatc tgggagcccc tgccgccttc 4020

aagtactttg acaccaccat cgaccggaag aggtacacca gcaccaaaga ggtgctggac 4080

gccaccctga tccaccagag catcaccggc ctgtacgaga cacggatcga cctgtctcag 4140

ctgggaggcg acgatccaaa aaagaagaga aaggtaggcg gctctggcgg cggctccgga 4200

ggctctagcc ccgaggacga gatccagcag ctggaggagg agatcagcca gctggagcag 4260

aagaacagcc agctgaagga gaagaaccag cagctgaagt actaa 4305

<210>  6
<211>  4305
<212>  DNA
<213>  Artificial Sequence

<220>
<223>  Cas9-P4S

<400>  6
atggccccaa agaagaagcg gaaggtcggt atccacggag tcccagcagc cgacaagaag      60

tacagcatcg gcctggacat cggcaccaac tctgtgggct gggccgtgat caccgacgag     120

tacaaggtgc ccagcaagaa attcaaggtg ctgggcaaca ccgaccggca cagcatcaag     180

aagaacctga tcggagccct gctgttcgac agcggcgaaa cagccgaggc cacccggctg     240

aagagaaccg ccagaagaag atacaccaga cggaagaacc ggatctgcta tctgcaagag     300

atcttcagca acgagatggc caaggtggac gacagcttct tccacagact ggaagagtcc     360

ttcctggtgg aagaggataa gaagcacgag cggcacccca tcttcggcaa catcgtggac     420

gaggtggcct accacgagaa gtaccccacc atctaccacc tgagaaagaa actggtggac     480

agcaccgaca aggccgacct gcggctgatc tatctggccc tggcccacat gatcaagttc     540

cggggccact tcctgatcga gggcgacctg aaccccgaca acagcgacgt ggacaagctg     600

ttcatccagc tggtgcagac ctacaaccag ctgttcgagg aaaaccccat caacgccagc     660

ggcgtggacg ccaaggccat cctgtctgcc agactgagca agagcagacg gctggaaaat     720

ctgatcgccc agctgcccgg cgagaagaag aatggcctgt tcggaaacct gattgccctg     780

agcctgggcc tgacccccaa cttcaagagc aacttcgacc tggccgagga tgccaaactg     840

cagctgagca aggacaccta cgacgacgac ctggacaacc tgctggccca gatcggcgac     900

cagtacgccg acctgtttct ggccgccaag aacctgtccg acgccatcct gctgagcgac     960

atcctgagag tgaacaccga gatcaccaag gcccccctga gcgcctctat gatcaagaga    1020

tacgacgagc accaccagga cctgaccctg ctgaaagctc tcgtgcggca gcagctgcct    1080

gagaagtaca aagagatttt cttcgaccag agcaagaacg gctacgccgg ctacattgac    1140

ggcggagcca gccaggaaga gttctacaag ttcatcaagc ccatcctgga aaagatggac    1200

ggcaccgagg aactgctcgt gaagctgaac agagaggacc tgctgcggaa gcagcggacc    1260

ttcgacaacg gcagcatccc ccaccagatc cacctgggag agctgcacgc cattctgcgg    1320

cggcaggaag atttttaccc attcctgaag gacaaccggg aaaagatcga gaagatcctg    1380

accttccgca tcccctacta cgtgggccct ctggccaggg gaaacagcag attcgcctgg 1440

atgaccagaa agagcgagga aaccatcacc ccctggaact tcgaggaagt ggtggacaag 1500

ggcgcttccg cccagagctt catcgagcgg atgaccaact tcgataagaa cctgcccaac 1560

gagaaggtgc tgcccaagca cagcctgctg tacgagtact tcaccgtgta taacgagctg 1620

accaaagtga aatacgtgac cgagggaatg agaaagcccg ccttcctgag cggcgagcag 1680

aaaaaggcca tcgtggacct gctgttcaag accaaccgga aagtgaccgt gaagcagctg 1740

aaagaggact acttcaagaa aatcgagtgc ttcgactccg tggaaatctc cggcgtggaa 1800

gatcggttca acgcctccct gggcacatac cacgatctgc tgaaaattat caaggacaag 1860

gacttcctgg acaatgagga aaacgaggac attctggaag atatcgtgct gaccctgaca 1920

ctgtttgagg acagagagat gatcgaggaa cggctgaaaa cctatgccca cctgttcgac 1980

gacaaagtga tgaagcagct gaagcggcgg agatacaccg gctggggcag gctgagccgg 2040

aagctgatca acggcatccg ggacaagcag tccggcaaga caatcctgga tttcctgaag 2100

tccgacggct tcgccaacag aaacttcatg cagctgatcc acgacgacag cctgaccttt 2160

aaagaggaca tccagaaagc ccaggtgtcc ggccagggcg atagcctgca cgagcacatt 2220

gccaatctgg ccggcagccc cgccattaag aagggcatcc tgcagacagt gaaggtggtg 2280

gacgagctcg tgaaagtgat gggccggcac aagcccgaga acatcgtgat cgaaatggcc 2340

agagagaacc agaccaccca gaagggacag aagaacagcc gcgagagaat gaagcggatc 2400

gaagagggca tcaaagagct gggcagccag atcctgaaag aacaccccgt ggaaaacacc 2460

cagctgcaga acgagaagct gtacctgtac tacctgcaga atgggcggga tatgtacgtg 2520

gaccaggaac tggacatcaa ccggctgtcc gactacgatg tggaccatat cgtgcctcag 2580

agctttctga aggacgactc catcgacaac aaggtgctga ccagaagcga caagaaccgg 2640

ggcaagagcg acaacgtgcc ctccgaagag gtcgtgaaga agatgaagaa ctactggcgg 2700

cagctgctga acgccaagct gattacccag agaaagttcg acaatctgac caaggccgag 2760

agaggcggcc tgagcgaact ggataaggcc ggcttcatca agagacagct ggtggaaacc 2820

cggcagatca caaagcacgt ggcacagatc ctggactccc ggatgaacac taagtacgac 2880

gagaatgaca agctgatccg ggaagtgaaa gtgatcaccc tgaagtccaa gctggtgtcc 2940

gatttccgga aggatttcca gttttacaaa gtgcgcgaga tcaacaacta ccaccacgcc 3000

cacgacgcct acctgaacgc cgtcgtggga accgccctga tcaaaaagta ccctaagctg 3060

gaaagcgagt tcgtgtacgg cgactacaag gtgtacgacg tgcggaagat gatcgccaag 3120

agcgagcagg aaatcggcaa ggctaccgcc aagtacttct tctacagcaa catcatgaac 3180

tttttcaaga ccgagattac cctggccaac ggcgagatcc ggaagcggcc tctgatcgag 3240

acaaacggcg aaaccgggga gatcgtgtgg gataagggcc gggattttgc caccgtgcgg 3300

aaagtgctga gcatgcccca agtgaatatc gtgaaaaaga ccgaggtgca gacaggcggc 3360

ttcagcaaag agtctatcct gcccaagagg aacagcgata agctgatcgc cagaaagaag 3420

gactgggacc ctaagaagta cggcggcttc gacagcccca ccgtggccta ttctgtgctg 3480

gtggtggcca aagtggaaaa gggcaagtcc aagaaactga agagtgtgaa agagctgctg 3540

gggatcacca tcatggaaag aagcagcttc gagaagaatc ccatcgactt tctggaagcc 3600

aagggctaca aagaagtgaa aaaggacctg atcatcaagc tgcctaagta ctccctgttc 3660

gagctggaaa acggccggaa gagaatgctg gcctctgccg gcgaactgca gaagggaaac 3720

gaactggccc tgccctccaa atatgtgaac ttcctgtacc tggccagcca ctatgagaag 3780

ctgaagggct cccccgagga taatgagcag aaacagctgt ttgtggaaca gcacaagcac 3840

tacctggacg agatcatcga gcagatcagc gagttctcca agagagtgat cctggccgac 3900

gctaatctgg acaaagtgct gtccgcctac aacaagcacc gggataagcc catcagagag 3960

caggccgaga atatcatcca cctgtttacc ctgaccaatc tgggagcccc tgccgccttc 4020

aagtactttg acaccaccat cgaccggaag aggtacacca gcaccaaaga ggtgctggac 4080

gccaccctga tccaccagag catcaccggc ctgtacgaga cacggatcga cctgtctcag 4140

ctgggaggcg acgatccaaa aaagaagaga aaggtaggcg gctctggcgg cggctccgga 4200

ggctctagcc ccgaggacaa gatcagccag ctgaagcaga agatccagca gctgaagcag 4260

gagaaccagc agctggagga ggagaacagc cagctggagt actaa 4305

<210> 7
<211> 4305
<212> DNA
<213> Artificial Sequence

<220>
<223> Cas9-AP4

<400> 7
atggccccaa agaagaagcg gaaggtcggt atccacggag tcccagcagc cgacaagaag 60

tacagcatcg gcctggacat cggcaccaac tctgtgggct gggccgtgat caccgacgag 120

tacaaggtgc ccagcaagaa attcaaggtg ctgggcaaca ccgaccggca cagcatcaag 180

aagaacctga tcggagccct gctgttcgac agcggcgaaa cagccgaggc cacccggctg 240

aagagaaccg ccagaagaag atacaccaga cggaagaacc ggatctgcta tctgcaagag 300

atcttcagca acgagatggc caaggtggac gacagcttct tccacagact ggaagagtcc 360

ttcctggtgg aagaggataa gaagcacgag cggcacccca tcttcggcaa catcgtggac 420

gaggtggcct accacgagaa gtaccccacc atctaccacc tgagaaagaa actggtggac 480

agcaccgaca aggccgacct gcggctgatc tatctggccc tggcccacat gatcaagttc 540

cggggccact tcctgatcga gggcgacctg aaccccgaca acagcgacgt ggacaagctg 600

ttcatccagc tggtgcagac ctacaaccag ctgttcgagg aaaaccccat caacgccagc 660

ggcgtggacg ccaaggccat cctgtctgcc agactgagca agagcagacg gctggaaaat 720

ctgatcgccc agctgcccgg cgagaagaag aatggcctgt tcggaaacct gattgccctg 780

agcctgggcc tgacccccaa cttcaagagc aacttcgacc tggccgagga tgccaaactg 840

cagctgagca aggacaccta cgacgacgac ctggacaacc tgctggccca gatcggcgac 900

cagtacgccg acctgtttct ggccgccaag aacctgtccg acgccatcct gctgagcgac 960

atcctgagag tgaacaccga gatcaccaag gcccccctga gcgcctctat gatcaagaga 1020

tacgacgagc accaccagga cctgaccctg ctgaaagctc tcgtgcggca gcagctgcct 1080

gagaagtaca aagagatttt cttcgaccag agcaagaacg gctacgccgg ctacattgac 1140

ggcggagcca gccaggaaga gttctacaag ttcatcaagc ccatcctgga aaagatggac 1200

ggcaccgagg aactgctcgt gaagctgaac agagaggacc tgctgcggaa gcagcggacc 1260

ttcgacaacg gcagcatccc ccaccagatc cacctgggag agctgcacgc cattctgcgg 1320

cggcaggaag atttttaccc attcctgaag gacaaccggg aaaagatcga gaagatcctg 1380

accttccgca tcccctacta cgtgggccct ctggccaggg gaaacagcag attcgcctgg 1440

atgaccagaa agagcgagga aaccatcacc ccctggaact tcgaggaagt ggtggacaag 1500

ggcgcttccg cccagagctt catcgagcgg atgaccaact tcgataagaa cctgcccaac 1560

gagaaggtgc tgcccaagca cagcctgctg tacgagtact tcaccgtgta taacgagctg 1620

accaaagtga aatacgtgac cgagggaatg agaaagcccg ccttcctgag cggcgagcag 1680

aaaaaggcca tcgtggacct gctgttcaag accaaccgga aagtgaccgt gaagcagctg 1740

aaagaggact acttcaagaa aatcgagtgc ttcgactccg tggaaatctc cggcgtggaa 1800

gatcggttca acgcctccct gggcacatac cacgatctgc tgaaaattat caaggacaag 1860

gacttcctgg acaatgagga aaacgaggac attctggaag atatcgtgct gaccctgaca 1920

ctgtttgagg acagagagat gatcgaggaa cggctgaaaa cctatgccca cctgttcgac 1980

gacaaagtga tgaagcagct gaagcggcgg agatacaccg gctggggcag gctgagccgg 2040

aagctgatca acggcatccg ggacaagcag tccggcaaga caatcctgga tttcctgaag 2100

tccgacggct tcgccaacag aaacttcatg cagctgatcc acgacgacag cctgaccttt 2160

aaagaggaca tccagaaagc ccaggtgtcc ggccagggcg atagcctgca cgagcacatt 2220

gccaatctgg ccggcagccc cgccattaag aagggcatcc tgcagacagt gaaggtggtg 2280

gacgagctcg tgaaagtgat gggccggcac aagcccgaga acatcgtgat cgaaatggcc 2340

agagagaacc agaccaccca gaagggacag aagaacagcc gcgagagaat gaagcggatc 2400

gaagagggca tcaaagagct gggcagccag atcctgaaag aacaccccgt ggaaaacacc 2460

cagctgcaga acgagaagct gtacctgtac tacctgcaga atgggcggga tatgtacgtg 2520

gaccaggaac tggacatcaa ccggctgtcc gactacgatg tggaccatat cgtgcctcag 2580

agctttctga aggacgactc catcgacaac aaggtgctga ccagaagcga caagaaccgg 2640

ggcaagagcg acaacgtgcc ctccgaagag gtcgtgaaga agatgaagaa ctactggcgg 2700

cagctgctga acgccaagct gattacccag agaaagttcg acaatctgac caaggccgag 2760

agaggcggcc tgagcgaact ggataaggcc ggcttcatca agagacagct ggtggaaacc 2820

cggcagatca caaagcacgt ggcacagatc ctggactccc ggatgaacac taagtacgac 2880

gagaatgaca agctgatccg ggaagtgaaa gtgatcaccc tgaagtccaa gctggtgtcc 2940

gatttccgga aggatttcca gttttacaaa gtgcgcgaga tcaacaacta ccaccacgcc 3000

cacgacgcct acctgaacgc cgtcgtggga accgccctga tcaaaaagta ccctaagctg 3060

gaaagcgagt tcgtgtacgg cgactacaag gtgtacgacg tgcggaagat gatcgccaag 3120

agcgagcagg aaatcggcaa ggctaccgcc aagtacttct tctacagcaa catcatgaac 3180

tttttcaaga ccgagattac cctggccaac ggcgagatcc ggaagcggcc tctgatcgag 3240

acaaacggcg aaaccgggga gatcgtgtgg gataagggcc gggattttgc caccgtgcgg 3300

aaagtgctga gcatgcccca agtgaatatc gtgaaaaaga ccgaggtgca gacaggcggc 3360

ttcagcaaag agtctatcct gcccaagagg aacagcgata agctgatcgc cagaaagaag 3420

gactgggacc ctaagaagta cggcggcttc gacagcccca ccgtggccta ttctgtgctg 3480

gtggtggcca aagtggaaaa gggcaagtcc aagaaactga agagtgtgaa agagctgctg 3540

gggatcacca tcatggaaag aagcagcttc gagaagaatc ccatcgactt tctggaagcc 3600

aagggctaca aagaagtgaa aaaggacctg atcatcaagc tgcctaagta ctccctgttc 3660

gagctggaaa acggccggaa gagaatgctg gcctctgccg gcgaactgca gaagggaaac 3720

gaactggccc tgccctccaa atatgtgaac ttcctgtacc tggccagcca ctatgagaag 3780

ctgaagggct cccccgagga taatgagcag aaacagctgt ttgtggaaca gcacaagcac 3840

tacctggacg agatcatcga gcagatcagc gagttctcca agagagtgat cctggccgac 3900

gctaatctgg acaaagtgct gtccgcctac aacaagcacc gggataagcc catcagagag 3960

caggccgaga atatcatcca cctgtttacc ctgaccaatc tgggagcccc tgccgccttc 4020

aagtactttg acaccaccat cgaccggaag aggtacacca gcaccaaaga ggtgctggac 4080

gccaccctga tccaccagag catcaccggc ctgtacgaga cacggatcga cctgtctcag 4140

ctgggaggcg acgatccaaa aaagaagaga aaggtaggcg gctctggcgg cggctccgga 4200

ggctctagcc ccgaggacga gctggccgcc aacgaggagg agctgcagca gaacgagcag 4260

aagctggccc agatcaagca gaagctgcag gccatcaagt actaa 4305

<210> 8
<211> 957
<212> DNA
<213> Artificial Sequence

<220>
<223> P4-EXOIII

<400> 8
agcccggaag ataaaattgc tcagctgaaa caaaaaatcc aagcgctgaa acaggaaaac 60

cagcagctgg aagaggaaaa cgccgcactg gaatatggtg gcggctctgg cggcggctcc 120

ggaggctctg atccaaaaaa gaagagaaag gtaaaatttg tctcttttaa tatcaacggc 180

ctgcgcgcca gacctcacca gcttgaagcc atcgtcgaaa agcaccaacc ggatgtgatt 240

ggcctgcagg agacaaaagt tcatgacgat atgtttccgc tcgaagaggt ggcgaagctc 300

ggctacaacg tgttttatca cgggcagaaa ggccattatg gcgtggcgct gctgaccaaa 360

gagacgccga ttgccgtgcg tcgcggcttt cccggtgacg acgaagaggc gcagcggcgg 420

attattatgg cggaaatccc ctcactgctg ggtaatgtca ccgtgatcaa cggttacttc 480

ccgcagggtg aaagccgcga ccatccgata aaattcccgg caaaagcgca gttttatcag 540

aatctgcaaa actacctgga aaccgaactc aaacgtgata atccggtact gattatgggc 600

gatatgaata tcagccctac agatctggac atcggcattg gcgaagaaaa ccgtaagcgc 660

tggctgcgta ccggtaaatg ctctttcctg ccggaagagc gcgaatggat ggacaggctg 720

atgagctggg ggttggtcga taccttccgc catgcgaatc cgcaaacagc agatcgtttc 780

tcatggtttg attaccgctc aaaaggtttt gacgataacc gtggtctgcg catcgacctg 840

ctgctcgcca gccaaccgct ggcagaatgt tgcgtagaaa ccggcatcga ctatgaaatc 900

cgcagcatgg aaaaaccgtc cgatcacgcc cccgtctggg cgaccttccg ccgctaa 957

<210> 9
<211> 957
<212> DNA
<213> Artificial Sequence

<220>
<223> P3-EXOIII

<400> 9
tccccggaag atgagatcca gcaactggaa gaagaaatcg ctcagctgga acagaaaaac 60

gcagcgctga aagagaaaaa ccaggcgctg aaatacggtg gcggctctgg cggcggctcc 120

ggaggctctg atccaaaaaa gaagagaaag gtaaaatttg tctcttttaa tatcaacggc 180

ctgcgcgcca gacctcacca gcttgaagcc atcgtcgaaa agcaccaacc ggatgtgatt 240

ggcctgcagg agacaaaagt tcatgacgat atgtttccgc tcgaagaggt ggcgaagctc 300

ggctacaacg tgttttatca cgggcagaaa ggccattatg gcgtggcgct gctgaccaaa 360

gagacgccga ttgccgtgcg tcgcggcttt cccggtgacg acgaagaggc gcagcggcgg 420

attattatgg cggaaatccc ctcactgctg ggtaatgtca ccgtgatcaa cggttacttc 480

ccgcagggtg aaagccgcga ccatccgata aaattcccgg caaaagcgca gttttatcag 540

aatctgcaaa actacctgga aaccgaactc aaacgtgata atccggtact gattatgggc 600

gatatgaata tcagccctac agatctggac atcggcattg gcgaagaaaa ccgtaagcgc 660

tggctgcgta ccggtaaatg ctctttcctg ccggaagagc gcgaatggat ggacaggctg 720

atgagctggg ggttggtcga taccttccgc catgcgaatc cgcaaacagc agatcgtttc 780

tcatggtttg attaccgctc aaaaggtttt gacgataacc gtggtctgcg catcgacctg 840

ctgctcgcca gccaaccgct ggcagaatgt tgcgtagaaa ccggcatcga ctatgaaatc 900

cgcagcatgg aaaaaccgtc cgatcacgcc cccgtctggg cgaccttccg ccgctaa 957

<210>  10
<211>  939
<212>  DNA
<213>  Artificial Sequence

<220>
<223>  N5-EXOIII

<400>  10
gagatcgccg ccctggaggc caagatcgcc gccctgaagg ccaagaacgc cgccctgaag      60

gccgagatcg ccgccctgga ggccggcggc tctggcggcg gctccggagg ctctgatcca     120

aaaaagaaga gaaaggtaaa atttgtctct tttaatatca acggcctgcg cgccagacct     180

caccagcttg aagccatcgt cgaaaagcac caaccggatg tgattggcct gcaggagaca     240

aaagttcatg acgatatgtt tccgctcgaa gaggtggcga agctcggcta caacgtgttt     300

tatcacgggc agaaaggcca ttatggcgtg gcgctgctga ccaaagagac gccgattgcc     360

gtgcgtcgcg gctttcccgg tgacgacgaa gaggcgcagc ggcggattat tatggcggaa     420

atcccctcac tgctgggtaa tgtcaccgtg atcaacggtt acttcccgca gggtgaaagc     480

cgcgaccatc cgataaaatt cccggcaaaa gcgcagtttt atcagaatct gcaaaactac     540

ctggaaaccg aactcaaacg tgataatccg gtactgatta tgggcgatat gaatatcagc     600

cctacagatc tggacatcgg cattggcgaa gaaaaccgta agcgctggct gcgtaccggt     660

aaatgctctt tcctgccgga agagcgcgaa tggatggaca ggctgatgag ctgggggttg     720

gtcgatacct tccgccatgc gaatccgcaa acagcagatc gtttctcatg gtttgattac     780

cgctcaaaag gttttgacga taaccgtggt ctgcgcatcg acctgctgct cgccagccaa     840

ccgctggcag aatgttgcgt agaaaccggc atcgactatg aaatccgcag catggaaaaa     900

ccgtccgatc acgcccccgt ctgggcgacc ttccgccgc                            939


<210>  11
<211>  939
<212>  DNA
<213>  Artificial Sequence

<220>
<223>  N6-EXOIII

<400>  11
aagatcgccg ccctgaaggc cgagatcgcc gccctggagg ccgagaacgc cgccctggag      60

gccaagatcg ccgccctgaa ggccggcggc tctggcggcg gctccggagg ctctgatcca     120

aaaaagaaga gaaaggtaaa atttgtctct tttaatatca acggcctgcg cgccagacct 180

caccagcttg aagccatcgt cgaaaagcac caaccggatg tgattggcct gcaggagaca 240

aaagttcatg acgatatgtt tccgctcgaa gaggtggcga agctcggcta caacgtgttt 300

tatcacgggc agaaaggcca ttatggcgtg gcgctgctga ccaaagagac gccgattgcc 360

gtgcgtcgcg gctttcccgg tgacgacgaa gaggcgcagc ggcggattat tatggcggaa 420

atcccctcac tgctgggtaa tgtcaccgtg atcaacggtt acttcccgca gggtgaaagc 480

cgcgaccatc cgataaaatt cccggcaaaa gcgcagtttt atcagaatct gcaaaactac 540

ctggaaaccg aactcaaacg tgataatccg gtactgatta tgggcgatat gaatatcagc 600

cctacagatc tggacatcgg cattggcgaa gaaaaccgta agcgctggct gcgtaccggt 660

aaatgctctt tcctgccgga agagcgcgaa tggatggaca ggctgatgag ctgggggttg 720

gtcgatacct tccgccatgc gaatccgcaa acagcagatc gtttctcatg gtttgattac 780

cgctcaaaag gttttgacga taaccgtggt ctgcgcatcg acctgctgct cgccagccaa 840

ccgctggcag aatgttgcgt agaaaccggc atcgactatg aaatccgcag catggaaaaa 900

ccgtccgatc acgcccccgt ctgggcgacc ttccgccgc 939

<210> 12
<211> 951
<212> DNA
<213> Artificial Sequence

<220>
<223> P3S-EXOIII

<400> 12
agccccgagg acgagatcca gcagctggag gaggagatca gccagctgga gcagaagaac 60

agccagctga aggagaagaa ccagcagctg aagtacggcg gctctggcgg cggctccgga 120

ggctctgatc caaaaaagaa gagaaaggta aaatttgtct cttttaatat caacggcctg 180

cgcgccagac ctcaccagct tgaagccatc gtcgaaaagc accaaccgga tgtgattggc 240

ctgcaggaga caaaagttca tgacgatatg tttccgctcg aagaggtggc gaagctcggc 300

tacaacgtgt tttatcacgg gcagaaaggc cattatggcg tggcgctgct gaccaaagag 360

acgccgattg ccgtgcgtcg cggctttccc ggtgacgacg aagaggcgca gcggcggatt 420

attatggcgg aaatcccctc actgctgggt aatgtcaccg tgatcaacgg ttacttcccg 480

cagggtgaaa gccgcgacca tccgataaaa ttcccggcaa aagcgcagtt ttatcagaat 540

ctgcaaaact acctggaaac cgaactcaaa cgtgataatc cggtactgat tatgggcgat 600

atgaatatca gccctacaga tctggacatc ggcattggcg aagaaaaccg taagcgctgg 660

ctgcgtaccg gtaaatgctc tttcctgccg gaagagcgcg aatggatgga caggctgatg 720

agctgggggt tggtcgatac cttccgccat gcgaatccgc aaacagcaga tcgtttctca 780

tggtttgatt accgctcaaa aggttttgac gataaccgtg gtctgcgcat cgacctgctg 840

ctcgccagcc aaccgctggc agaatgttgc gtagaaaccg gcatcgacta tgaaatccgc 900

agcatggaaa aaccgtccga tcacgccccc gtctgggcga ccttccgccg c 951

<210> 13
<211> 951
<212> DNA
<213> Artificial Sequence

<220>
<223> P4S-EXOIII

<400> 13
agccccgagg acaagatcag ccagctgaag cagaagatcc agcagctgaa gcaggagaac 60

cagcagctgg aggaggagaa cagccagctg gagtacggcg gctctggcgg cggctccgga 120

ggctctgatc caaaaaagaa gagaaaggta aaatttgtct cttttaatat caacggcctg 180

cgcgccagac ctcaccagct tgaagccatc gtcgaaaagc accaaccgga tgtgattggc 240

ctgcaggaga caaaagttca tgacgatatg tttccgctcg aagaggtggc gaagctcggc 300

tacaacgtgt tttatcacgg gcagaaaggc cattatggcg tggcgctgct gaccaaagag 360

acgccgattg ccgtgcgtcg cggctttccc ggtgacgacg aagaggcgca gcggcggatt 420

attatggcgg aaatcccctc actgctgggt aatgtcaccg tgatcaacgg ttacttcccg 480

cagggtgaaa gccgcgacca tccgataaaa ttcccggcaa aagcgcagtt ttatcagaat 540

ctgcaaaact acctggaaac cgaactcaaa cgtgataatc cggtactgat tatgggcgat 600

atgaatatca gccctacaga tctggacatc ggcattggcg aagaaaaccg taagcgctgg 660

ctgcgtaccg gtaaatgctc tttcctgccg gaagagcgcg aatggatgga caggctgatg 720

agctgggggt tggtcgatac cttccgccat gcgaatccgc aaacagcaga tcgtttctca 780

tggtttgatt accgctcaaa aggttttgac gataaccgtg gtctgcgcat cgacctgctg 840

ctcgccagcc aaccgctggc agaatgttgc gtagaaaccg gcatcgacta tgaaatccgc 900

agcatggaaa aaccgtccga tcacgccccc gtctgggcga ccttccgccg c 951

<210>  14
<211>  951
<212>  DNA
<213>  Artificial Sequence

<220>
<223>  AP4-EXOIII

<400>  14
agccccgagg acgagctggc cgccaacgag gaggagctgc agcagaacga gcagaagctg      60

gcccagatca agcagaagct gcaggccatc aagtacggcg gctctggcgg cggctccgga     120

ggctctgatc caaaaaagaa gagaaaggta aaatttgtct cttttaatat caacggcctg     180

cgcgccagac ctcaccagct tgaagccatc gtcgaaaagc accaaccgga tgtgattggc     240

ctgcaggaga caaaagttca tgacgatatg tttccgctcg aagaggtggc gaagctcggc     300

tacaacgtgt tttatcacgg gcagaaaggc cattatggcg tggcgctgct gaccaaagag     360

acgccgattg ccgtgcgtcg cggctttccc ggtgacgacg aagaggcgca gcggcggatt     420

attatggcgg aaatcccctc actgctgggt aatgtcaccg tgatcaacgg ttacttcccg     480

cagggtgaaa gccgcgacca tccgataaaa ttcccggcaa aagcgcagtt ttatcagaat     540

ctgcaaaact acctggaaac cgaactcaaa cgtgataatc cggtactgat tatgggcgat     600

atgaatatca gccctacaga tctggacatc ggcattggcg aagaaaaccg taagcgctgg     660

ctgcgtaccg gtaaatgctc tttcctgccg gaagagcgcg aatggatgga caggctgatg     720

agctgggggt tggtcgatac cttccgccat gcgaatccgc aaacagcaga tcgtttctca     780

tggtttgatt accgctcaaa aggttttgac gataaccgtg gtctgcgcat cgacctgctg     840

ctcgccagcc aaccgctggc agaatgttgc gtagaaaccg gcatcgacta tgaaatccgc     900

agcatggaaa aaccgtccga tcacgccccc gtctgggcga ccttccgccg c              951


<210>  15
<211>  840
<212>  DNA
<213>  Artificial Sequence

<220>
<223>  P3-TREX2

<400>  15
tccccggaag atgagatcca gcaactggaa gaagaaatcg ctcagctgga acagaaaaac      60

gcagcgctga aagagaaaaa ccaggcgctg aaatacggtg gcggctctgg cggcggctcc     120

ggaggctcta tgtctgagcc acctcgggct gagacctttg tattcctgga cctagaagcc 180

actgggctcc caaacatgga ccctgagatt gcagagatat ccctttttgc tgttcaccgc 240

tcttccctgg agaacccaga acgggatgat tctggttcct tggtgctgcc ccgtgttctg 300

gacaagctca cactgtgcat gtgcccggag cgccccttta ctgccaaggc cagtgagatt 360

actggtttga gcagcgaaag cctgatgcac tgcgggaagg ctggtttcaa tggcgctgtg 420

gtaaggacac tgcagggctt cctaagccgc caggagggcc ccatctgcct tgtggcccac 480

aatggcttcg attatgactt cccactgctg tgcacggagc tacaacgtct gggtgcccat 540

ctgccccaag acactgtctg cctggacaca ctgcctgcat tgcggggcct ggaccgtgct 600

cacagccacg gcaccagggc tcaaggccgc aaaagctaca gcctggccag tctcttccac 660

cgctacttcc aggctgaacc cagtgctgcc cattcagcag aaggtgatgt gcacaccctg 720

cttctgatct tcctgcatcg tgctcctgag ctgctcgcct gggcagatga gcaggcccgc 780

agctgggctc atattgagcc catgtacgtg ccacctgatg gtccaagcct cgaagcctga 840

<210> 16
<211> 837
<212> DNA
<213> Artificial Sequence

<220>
<223> P4-TREX2

<400> 16
agcccggaag ataaaattgc tcagctgaaa caaaaaatcc aagcgctgaa acaggaaaac 60

cagcagctgg aagaggaaaa cgccgcactg gaatatggtg gcggctctgg cggcggctcc 120

ggaggctctt ctgagccacc tcgggctgag acctttgtat tcctggacct agaagccact 180

gggctcccaa acatggaccc tgagattgca gagatatccc tttttgctgt tcaccgctct 240

tccctggaga acccagaacg ggatgattct ggttccttgg tgctgccccg tgttctggac 300

aagctcacac tgtgcatgtg cccggagcgc ccctttactg ccaaggccag tgagattact 360

ggtttgagca gcgaaagcct gatgcactgc gggaaggctg gtttcaatgg cgctgtggta 420

aggacactgc agggcttcct aagccgccag gagggcccca tctgccttgt ggcccacaat 480

ggcttcgatt atgacttccc actgctgtgc acggagctac aacgtctggg tgcccatctg 540

ccccaagaca ctgtctgcct ggacacactg cctgcattgc ggggcctgga ccgtgctcac 600

agccacggca ccagggctca aggccgcaaa agctacagcc tggccagtct cttccaccgc 660

tacttccagg ctgaacccag tgctgcccat tcagcagaag gtgatgtgca caccctgctt 720

ctgatcttcc tgcatcgtgc tcctgagctg ctcgcctggg cagatgagca ggcccgcagc 780

tgggctcata ttgagcccat gtacgtgcca cctgatggtc caagcctcga agcctga 837

<210> 17
<211> 2668
<212> DNA
<213> Artificial Sequence

<220>
<223> P3-EXO1

<400> 17
tccccggaag atgagatcca gcaactggaa gaagaaatcg ctcagctgga acagaaaaac 60

gcagcgctga aagagaaaaa ccaggcgctg aaatacggtg gcggctctgg cggcggctcc 120

ggaggctctg cgatcgccat ggggatacag ggattgctac aatttatcaa agaagcttca 180

gaacccatcc atgtgaggaa gtataaaggg caggtagtag ctgtggatac atattgctgg 240

cttcacaaag gagctattgc ttgtgctgaa aaactagcca aaggtgaacc tactgatagg 300

tatgtaggat tttgtatgaa atttgtaaat atgttactat ctcatgggat caagcctatt 360

ctcgtatttg atggatgtac tttaccttct aaaaaggaag tagagagatc tagaagagaa 420

agacgacaag ccaatcttct taagggaaag caacttcttc gtgaggggaa agtctcggaa 480

gctcgagagt gtttcacccg gtctatcaat atcacacatg ccatggccca caaagtaatt 540

aaagctgccc ggtctcaggg ggtagattgc ctcgtggctc cctatgaagc tgatgcgcag 600

ttggcctatc ttaacaaagc gggaattgtg caagccataa ttacagagga ctcggatctc 660

ctagcttttg gctgtaaaaa ggtaatttta aagatggacc agtttggaaa tggacttgaa 720

attgatcaag ctcggctagg aatgtgcaga cagcttgggg atgtattcac ggaagagaag 780

tttcgttaca tgtgtattct ttcaggttgt gactacctgt catcactgcg tgggattgga 840

ttagcaaagg catgcaaagt cctaagacta gccaataatc cagatatagt aaaggttatc 900

aagaaaattg gacattatct caagatgaat atcacggtac cagaggatta catcaacggg 960

tttattcggg ccaacaatac cttcctctat cagctagttt ttgatcccat caaaaggaaa 1020

cttattcctc tgaacgccta tgaagatgat gttgatcctg aaacactaag ctacgctggg 1080

caatatgttg atgattccat agctcttcaa atagcacttg gaaataaaga tataaatact 1140

tttgaacaga tcgatgacta caatccagac actgctatgc ctgcccattc aagaagtcat 1200

agttgggatg acaaaacatg tcaaaagtca gctaatgtta gcagcatttg gcataggaat 1260

tactctccca gaccagagtc gggtactgtt tcagatgccc cacaattgaa ggaaaatcca 1320

agtactgtgg gagtggaacg agtgattagt actaaagggt taaatctccc aaggaaatca 1380

tccattgtga aaagaccaag aagtgcagag ctgtcagaag atgacctgtt gagtcagtat 1440

tctctttcat ttacgaagaa gaccaagaaa aatagctctg aaggcaataa atcattgagc 1500

ttttctgaag tgtttgtgcc tgacctggta aatggaccta ctaacaaaaa gagtgtaagc 1560

actccaccta ggacgagaaa taaatttgca acatttttac aaaggaaaaa tgaagaaagt 1620

ggtgcagttg tggttccagg gaccagaagc aggttttttt gcagttcaga ttctactgac 1680

tgtgtatcaa acaaagtgag catccagcct ctggatgaaa ctgctgtcac agataaagag 1740

aacaatctgc atgaatcaga gtatggagac caagaaggca agagactggt tgacacagat 1800

gtagcacgta attcaagtga tgacattccg aataatcata ttccaggtga tcatattcca 1860

gacaaggcaa cagtgtttac agatgaagag tcctactctt ttgagagcag caaatttaca 1920

aggaccattt caccacccac tttgggaaca ctaagaagtt gttttagttg gtctggaggt 1980

cttggagatt tttcaagaac gccgagcccc tctccaagca cagcattgca gcagttccga 2040

agaaagagcg attcccccac ctctttgcct gagaataata tgtctgatgt gtcgcagtta 2100

aagagcgagg agtccagtga cgatgagtct catcccttac gagaaggggc atgttcttca 2160

cagtcccagg aaagtggaga attctcactg cagagttcaa atgcatcaaa gctttctcag 2220

tgctctagta aggactctga ttcagaggaa tctgattgca atattaagtt acttgacagt 2280

caaagtgacc agacctccaa gctatgttta tctcatttct caaaaaaaga cacacctcta 2340

aggaacaagg ttcctgggct atataagtcc agttctgcag actctctttc tacaaccaag 2400

atcaaacctc taggacctgc cagagccagt gggctgagca agaagccggc aagcatccag 2460

aagagaaagc atcataatgc cgagaacaag ccggggttac agatcaaact caatgagctc 2520

tggaaaaact ttggatttaa aaaagattct gaaaagcttc ctccttgtaa gaaacccctg 2580

tccccagtca gagataacat ccaactaact ccagaagcgg aagaggatat atttaacaaa 2640

cctgaatgtg gccgtgttca aagagcaa 2668

<210> 18
<211> 2668
<212> DNA
<213> Artificial Sequence

<220>
<223>  P4-EXO1

<400>  18
   agcccggaag ataaaattgc tcagctgaaa caaaaaatcc aagcgctgaa acaggaaaac      60
   cagcagctgg aagaggaaaa cgccgcactg gaatatggtg gcggctctgg cggcggctcc     120
   ggaggctctg cgatcgccat ggggatacag ggattgctac aatttatcaa agaagcttca     180
   gaacccatcc atgtgaggaa gtataaaggg caggtagtag ctgtggatac atattgctgg     240
   cttcacaaag gagctattgc ttgtgctgaa aaactagcca aaggtgaacc tactgatagg     300
   tatgtaggat tttgtatgaa atttgtaaat atgttactat ctcatgggat caagcctatt     360
   ctcgtatttg atggatgtac tttaccttct aaaaaggaag tagagagatc tagaagagaa     420
   agacgacaag ccaatcttct taagggaaag caacttcttc gtgaggggaa agtctcggaa     480
   gctcgagagt gtttcacccg gtctatcaat atcacacatg ccatggccca caaagtaatt     540
   aaagctgccc ggtctcaggg ggtagattgc ctcgtggctc cctatgaagc tgatgcgcag     600
   ttggcctatc ttaacaaagc gggaattgtg caagccataa ttacagagga ctcggatctc     660
   ctagcttttg gctgtaaaaa ggtaatttta aagatggacc agtttggaaa tggacttgaa     720
   attgatcaag ctcggctagg aatgtgcaga cagcttgggg atgtattcac ggaagagaag     780
   tttcgttaca tgtgtattct ttcaggttgt gactacctgt catcactgcg tgggattgga     840
   ttagcaaagg catgcaaagt cctaagacta gccaataatc cagatatagt aaaggttatc     900
   aagaaaattg gacattatct caagatgaat atcacggtac cagaggatta catcaacggg     960
   tttattcggg ccaacaatac cttcctctat cagctagttt ttgatcccat caaaaggaaa    1020
   cttattcctc tgaacgccta tgaagatgat gttgatcctg aaacactaag ctacgctggg    1080
   caatatgttg atgattccat agctcttcaa atagcacttg gaaataaaga tataaatact    1140
   tttgaacaga tcgatgacta caatccagac actgctatgc ctgcccattc aagaagtcat    1200
   agttgggatg acaaaacatg tcaaaagtca gctaatgtta gcagcatttg gcataggaat    1260
   tactctccca gaccagagtc gggtactgtt tcagatgccc cacaattgaa ggaaaatcca    1320
   agtactgtgg gagtggaacg agtgattagt actaaagggt taaatctccc aaggaaatca    1380
   tccattgtga aaagaccaag aagtgcagag ctgtcagaag atgacctgtt gagtcagtat    1440
   tctctttcat ttacgaagaa gaccaagaaa aatagctctg aaggcaataa atcattgagc    1500
   ttttctgaag tgtttgtgcc tgacctggta aatggaccta ctaacaaaaa gagtgtaagc    1560
   actccaccta ggacgagaaa taaatttgca acatttttac aaaggaaaaa tgaagaaagt 1620
   ggtgcagttg tggttccagg gaccagaagc aggttttttt gcagttcaga ttctactgac 1680
   tgtgtatcaa acaaagtgag catccagcct ctggatgaaa ctgctgtcac agataaagag 1740
   aacaatctgc atgaatcaga gtatggagac caagaaggca agagactggt tgacacagat 1800
   gtagcacgta attcaagtga tgacattccg aataatcata ttccaggtga tcatattcca 1860
   gacaaggcaa cagtgtttac agatgaagag tcctactctt ttgagagcag caaatttaca 1920
   aggaccattt caccacccac tttgggaaca ctaagaagtt gttttagttg gtctggaggt 1980
   cttggagatt tttcaagaac gccgagcccc tctccaagca cagcattgca gcagttccga 2040
   agaaagagcg attcccccac ctctttgcct gagaataata tgtctgatgt gtcgcagtta 2100
   aagagcgagg agtccagtga cgatgagtct catcccttac gagaaggggc atgttcttca 2160
   cagtcccagg aaagtggaga attctcactg cagagttcaa atgcatcaaa gctttctcag 2220
   tgctctagta aggactctga ttcagaggaa tctgattgca atattaagtt acttgacagt 2280
   caaagtgacc agacctccaa gctatgttta tctcatttct caaaaaaaga cacacctcta 2340
   aggaacaagg ttcctgggct atataagtcc agttctgcag actctctttc tacaaccaag 2400
   atcaaacctc taggacctgc cagagccagt gggctgagca agaagccggc aagcatccag 2460
   aagagaaagc atcataatgc cgagaacaag ccggggttac agatcaaact caatgagctc 2520
   tggaaaaact ttggatttaa aaaagattct gaaaagcttc ctccttgtaa gaaacccctg 2580
   tccccagtca gagataacat ccaactaact ccagaagcgg aagaggatat atttaacaaa 2640
   cctgaatgtg gccgtgttca aagagcaa 2668

SEQUENCE LISTING

<110>  National Institute of Chemistry

<120>  Coiled-coil mediated tethering of CRISPR/CAS and exonucleases for
       enhanced genome editing

<130>  69116P EP

<160>  18

<170>  PatentIn version 3.5

<210>  1
<211>  4308
<212>  DNA
<213>  Artificial Sequence

<220>
<223>  Cas9-P4

<400>  1
atggccccaa agaagaagcg gaaggtcggt atccacggag tcccagcagc cgacaagaag      60

tacagcatcg gcctggacat cggcaccaac tctgtgggct gggccgtgat caccgacgag     120

tacaaggtgc ccagcaagaa attcaaggtg ctgggcaaca ccgaccggca cagcatcaag     180

aagaacctga tcggagccct gctgttcgac agcggcgaaa cagccgaggc cacccggctg     240

aagagaaccg ccagaagaag atacaccaga cggaagaacc ggatctgcta tctgcaagag     300

atcttcagca acgagatggc caaggtggac gacagcttct tccacagact ggaagagtcc     360

ttcctggtgg aagaggataa gaagcacgag cggcacccca tcttcggcaa catcgtggac     420

gaggtggcct accacgagaa gtaccccacc atctaccacc tgagaaagaa actggtggac     480

agcaccgaca aggccgacct gcggctgatc tatctggccc tggcccacat gatcaagttc     540

cggggccact tcctgatcga gggcgacctg aaccccgaca acagcgacgt ggacaagctg     600

ttcatccagc tggtgcagac ctacaaccag ctgttcgagg aaaaccccat caacgccagc     660

ggcgtggacg ccaaggccat cctgtctgcc agactgagca agagcagacg gctggaaaat     720

ctgatcgccc agctgcccgg cgagaagaag aatggcctgt tcggaaacct gattgccctg     780

agcctgggcc tgacccccaa cttcaagagc aacttcgacc tggccgagga tgccaaactg     840

cagctgagca aggacaccta cgacgacgac ctggacaacc tgctggccca gatcggcgac     900

cagtacgccg acctgtttct ggccgccaag aacctgtccg acgccatcct gctgagcgac     960

atcctgagag tgaacaccga gatcaccaag gcccccctga gcgcctctat gatcaagaga    1020

tacgacgagc accaccagga cctgaccctg ctgaaagctc tcgtgcggca gcagctgcct    1080

gagaagtaca aagagatttt cttcgaccag agcaagaacg gctacgccgg ctacattgac    1140

ggcggagcca gccaggaaga gttctacaag ttcatcaagc ccatcctgga aaagatggac    1200

ggcaccgagg aactgctcgt gaagctgaac agagaggacc tgctgcggaa gcagcggacc    1260

ttcgacaacg gcagcatccc ccaccagatc cacctgggag agctgcacgc cattctgcgg 1320

cggcaggaag atttttaccc attcctgaag gacaaccggg aaaagatcga gaagatcctg 1380

accttccgca tcccctacta cgtgggccct ctggccaggg gaaacagcag attcgcctgg 1440

atgaccagaa agagcgagga aaccatcacc ccctggaact tcgaggaagt ggtggacaag 1500

ggcgcttccg cccagagctt catcgagcgg atgaccaact tcgataagaa cctgcccaac 1560

gagaaggtgc tgcccaagca cagcctgctg tacgagtact tcaccgtgta taacgagctg 1620

accaaagtga aatacgtgac cgagggaatg agaaagcccg ccttcctgag cggcgagcag 1680

aaaaaggcca tcgtggacct gctgttcaag accaaccgga aagtgaccgt gaagcagctg 1740

aaagaggact acttcaagaa aatcgagtgc ttcgactccg tggaaatctc cggcgtggaa 1800

gatcggttca acgcctccct gggcacatac cacgatctgc tgaaaattat caaggacaag 1860

gacttcctgg acaatgagga aaacgaggac attctggaag atatcgtgct gaccctgaca 1920

ctgtttgagg acagagagat gatcgaggaa cggctgaaaa cctatgccca cctgttcgac 1980

gacaaagtga tgaagcagct gaagcggcgg agatacaccg gctggggcag gctgagccgg 2040

aagctgatca acggcatccg ggacaagcag tccggcaaga caatcctgga tttcctgaag 2100

tccgacggct tcgccaacag aaacttcatg cagctgatcc acgacgacag cctgaccttt 2160

aaagaggaca tccagaaagc ccaggtgtcc ggccagggcg atagcctgca cgagcacatt 2220

gccaatctgg ccggcagccc cgccattaag aagggcatcc tgcagacagt gaaggtggtg 2280

gacgagctcg tgaaagtgat gggccggcac aagcccgaga acatcgtgat cgaaatggcc 2340

agagagaacc agaccaccca gaagggacag aagaacagcc gcgagagaat gaagcggatc 2400

gaagagggca tcaaagagct gggcagccag atcctgaaag aacaccccgt ggaaaacacc 2460

cagctgcaga acgagaagct gtacctgtac tacctgcaga atgggcggga tatgtacgtg 2520

gaccaggaac tggacatcaa ccggctgtcc gactacgatg tggaccatat cgtgcctcag 2580

agctttctga aggacgactc catcgacaac aaggtgctga ccagaagcga caagaaccgg 2640

ggcaagagcg acaacgtgcc ctccgaagag gtcgtgaaga agatgaagaa ctactggcgg 2700

cagctgctga acgccaagct gattacccag agaaagttcg acaatctgac caaggccgag 2760

agaggcggcc tgagcgaact ggataaggcc ggcttcatca agagacagct ggtggaaacc 2820

cggcagatca caaagcacgt ggcacagatc ctggactccc ggatgaacac taagtacgac 2880

gagaatgaca agctgatccg ggaagtgaaa gtgatcaccc tgaagtccaa gctggtgtcc 2940

gatttccgga aggatttcca gttttacaaa gtgcgcgaga tcaacaacta ccaccacgcc 3000

cacgacgcct acctgaacgc cgtcgtggga accgccctga tcaaaaagta ccctaagctg 3060

gaaagcgagt tcgtgtacgg cgactacaag gtgtacgacg tgcggaagat gatcgccaag 3120

agcgagcagg aaatcggcaa ggctaccgcc aagtacttct tctacagcaa catcatgaac 3180

tttttcaaga ccgagattac cctggccaac ggcgagatcc ggaagcggcc tctgatcgag 3240

acaaacggcg aaaccgggga gatcgtgtgg gataagggcc gggattttgc caccgtgcgg 3300

aaagtgctga gcatgcccca agtgaatatc gtgaaaaaga ccgaggtgca gacaggcggc 3360

ttcagcaaag agtctatcct gcccaagagg aacagcgata agctgatcgc cagaaagaag 3420

gactgggacc ctaagaagta cggcggcttc gacagcccca ccgtggccta ttctgtgctg 3480

gtggtggcca aagtggaaaa gggcaagtcc aagaaactga agagtgtgaa agagctgctg 3540

gggatcacca tcatggaaag aagcagcttc gagaagaatc ccatcgactt tctggaagcc 3600

aagggctaca aagaagtgaa aaaggacctg atcatcaagc tgcctaagta ctccctgttc 3660

gagctggaaa acggccggaa gagaatgctg gcctctgccg gcgaactgca gaagggaaac 3720

gaactggccc tgccctccaa atatgtgaac ttcctgtacc tggccagcca ctatgagaag 3780

ctgaagggct cccccgagga taatgagcag aaacagctgt ttgtggaaca gcacaagcac 3840

tacctggacg agatcatcga gcagatcagc gagttctcca agagagtgat cctggccgac 3900

gctaatctgg acaaagtgct gtccgcctac aacaagcacc gggataagcc catcagagag 3960

caggccgaga atatcatcca cctgtttacc ctgaccaatc tgggagcccc tgccgccttc 4020

aagtactttg acaccaccat cgaccggaag aggtacacca gcaccaaaga ggtgctggac 4080

gccaccctga tccaccagag catcaccggc ctgtacgaga cacggatcga cctgtctcag 4140

ctgggaggcg acgatccaaa aaagaagaga aaggtaggcg gctctggcgg cggctccgga 4200

ggctctagcc cggaagataa aattgctcag ctgaaacaaa aaatccaagc gctgaaacag 4260

gaaaaccagc agctggaaga ggaaaacgcc gcactggaat atggttaa 4308

<210> 2
<211> 4308
<212> DNA
<213> Artificial Sequence

<220>
<223> Cas9-P3

<400> 2
atggccccaa agaagaagcg gaaggtcggt atccacggag tcccagcagc cgacaagaag 60

tacagcatcg gcctggacat cggcaccaac tctgtgggct gggccgtgat caccgacgag 120

tacaaggtgc ccagcaagaa attcaaggtg ctgggcaaca ccgaccggca cagcatcaag 180

aagaacctga tcggagccct gctgttcgac agcggcgaaa cagccgaggc cacccggctg 240

aagagaaccg ccagaagaag atacaccaga cggaagaacc ggatctgcta tctgcaagag 300

atcttcagca acgagatggc caaggtggac gacagcttct tccacagact ggaagagtcc 360

ttcctggtgg aagaggataa gaagcacgag cggcacccca tcttcggcaa catcgtggac 420

gaggtggcct accacgagaa gtaccccacc atctaccacc tgagaaagaa actggtggac 480

agcaccgaca aggccgacct gcggctgatc tatctggccc tggcccacat gatcaagttc 540

cggggccact tcctgatcga gggcgacctg aaccccgaca acagcgacgt ggacaagctg 600

ttcatccagc tggtgcagac ctacaaccag ctgttcgagg aaaaccccat caacgccagc 660

ggcgtggacg ccaaggccat cctgtctgcc agactgagca agagcagacg gctggaaaat 720

ctgatcgccc agctgcccgg cgagaagaag aatggcctgt tcggaaacct gattgccctg 780

agcctgggcc tgacccccaa cttcaagagc aacttcgacc tggccgagga tgccaaactg 840

cagctgagca aggacaccta cgacgacgac ctggacaacc tgctggccca gatcggcgac 900

cagtacgccg acctgtttct ggccgccaag aacctgtccg acgccatcct gctgagcgac 960

atcctgagag tgaacaccga gatcaccaag gcccccctga gcgcctctat gatcaagaga 1020

tacgacgagc accaccagga cctgaccctg ctgaaagctc tcgtgcggca gcagctgcct 1080

gagaagtaca aagagatttt cttcgaccag agcaagaacg gctacgccgg ctacattgac 1140

ggcggagcca gccaggaaga gttctacaag ttcatcaagc ccatcctgga aaagatggac 1200

ggcaccgagg aactgctcgt gaagctgaac agagaggacc tgctgcggaa gcagcggacc 1260

ttcgacaacg gcagcatccc ccaccagatc cacctgggag agctgcacgc cattctgcgg 1320

cggcaggaag atttttaccc attcctgaag gacaaccggg aaaagatcga gaagatcctg 1380

accttccgca tcccctacta cgtgggccct ctggccaggg gaaacagcag attcgcctgg 1440

atgaccagaa agagcgagga aaccatcacc ccctggaact tcgaggaagt ggtggacaag 1500

ggcgcttccg cccagagctt catcgagcgg atgaccaact tcgataagaa cctgcccaac 1560

gagaaggtgc tgcccaagca cagcctgctg tacgagtact tcaccgtgta taacgagctg 1620

accaaagtga aatacgtgac cgagggaatg agaaagcccg ccttcctgag cggcgagcag 1680

aaaaaggcca tcgtggacct gctgttcaag accaaccgga aagtgaccgt gaagcagctg 1740

aaagaggact acttcaagaa aatcgagtgc ttcgactccg tggaaatctc cggcgtggaa 1800

gatcggttca acgcctccct gggcacatac cacgatctgc tgaaaattat caaggacaag 1860

gacttcctgg acaatgagga aaacgaggac attctggaag atatcgtgct gaccctgaca 1920

ctgtttgagg acagagagat gatcgaggaa cggctgaaaa cctatgccca cctgttcgac 1980

gacaaagtga tgaagcagct gaagcggcgg agatacaccg gctggggcag gctgagccgg 2040

aagctgatca acggcatccg ggacaagcag tccggcaaga caatcctgga tttcctgaag 2100

tccgacggct tcgccaacag aaacttcatg cagctgatcc acgacgacag cctgaccttt 2160

aaagaggaca tccagaaagc ccaggtgtcc ggccagggcg atagcctgca cgagcacatt 2220

gccaatctgg ccggcagccc cgccattaag aagggcatcc tgcagacagt gaaggtggtg 2280

gacgagctcg tgaaagtgat gggccggcac aagcccgaga acatcgtgat cgaaatggcc 2340
agagagaacc agaccaccca gaagggacag aagaacagcc gcgagagaat gaagcggatc 2400
gaagagggca tcaaagagct gggcagccag atcctgaaag aacaccccgt ggaaaacacc 2460
cagctgcaga acgagaagct gtacctgtac tacctgcaga atgggcggga tatgtacgtg 2520
gaccaggaac tggacatcaa ccggctgtcc gactacgatg tggaccatat cgtgcctcag 2580
agctttctga aggacgactc catcgacaac aaggtgctga ccagaagcga caagaaccgg 2640
ggcaagagcg acaacgtgcc ctccgaagag gtcgtgaaga agatgaagaa ctactggcgg 2700
cagctgctga acgccaagct gattacccag agaaagttcg acaatctgac caaggccgag 2760
agaggcggcc tgagcgaact ggataaggcc ggcttcatca agagacagct ggtggaaacc 2820
cggcagatca caaagcacgt ggcacagatc ctggactccc ggatgaacac taagtacgac 2880
gagaatgaca agctgatccg ggaagtgaaa gtgatcaccc tgaagtccaa gctggtgtcc 2940
gatttccgga aggatttcca gttttacaaa gtgcgcgaga tcaacaacta ccaccacgcc 3000
cacgacgcct acctgaacgc cgtcgtggga accgccctga tcaaaaagta ccctaagctg 3060
gaaagcgagt tcgtgtacgg cgactacaag gtgtacgacg tgcggaagat gatcgccaag 3120
agcgagcagg aaatcggcaa ggctaccgcc aagtacttct tctacagcaa catcatgaac 3180
tttttcaaga ccgagattac cctggccaac ggcgagatcc ggaagcggcc tctgatcgag 3240
acaaacggcg aaaccgggga gatcgtgtgg gataagggcc gggattttgc caccgtgcgg 3300
aaagtgctga gcatgcccca agtgaatatc gtgaaaaaga ccgaggtgca gacaggcggc 3360
ttcagcaaag agtctatcct gcccaagagg aacagcgata agctgatcgc cagaaagaag 3420
gactgggacc ctaagaagta cggcggcttc gacagcccca ccgtggccta ttctgtgctg 3480
gtggtggcca aagtggaaaa gggcaagtcc aagaaactga agagtgtgaa agagctgctg 3540
gggatcacca tcatggaaag aagcagcttc gagaagaatc ccatcgactt tctggaagcc 3600
aagggctaca aagaagtgaa aaaggacctg atcatcaagc tgcctaagta ctccctgttc 3660
gagctggaaa acggccggaa gagaatgctg gcctctgccg gcgaactgca gaagggaaac 3720
gaactggccc tgccctccaa atatgtgaac ttcctgtacc tggccagcca ctatgagaag 3780
ctgaagggct cccccgagga taatgagcag aaacagctgt ttgtggaaca gcacaagcac 3840
tacctggacg agatcatcga gcagatcagc gagttctcca agagagtgat cctggccgac 3900
gctaatctgg acaaagtgct gtccgcctac aacaagcacc gggataagcc catcagagag 3960
caggccgaga atatcatcca cctgtttacc ctgaccaatc tgggagcccc tgccgccttc 4020
aagtactttg acaccaccat cgaccggaag aggtacacca gcaccaaaga ggtgctggac 4080
gccaccctga tccaccagag catcaccggc ctgtacgaga cacggatcga cctgtctcag 4140
ctgggaggcg acgatccaaa aaagaagaga aaggtaggcg gctctggcgg cggctccgga 4200

ggctcttccc cggaagatga gatccagcaa ctggaagaag aaatcgctca gctggaacag 4260

aaaaacgcag cgctgaaaga gaaaaaccag gcgctgaaat acggttaa 4308

<210> 3
<211> 4293
<212> DNA
<213> Artificial Sequence

<220>
<223> Cas9-N5

<400> 3
atggccccaa agaagaagcg gaaggtcggt atccacggag tcccagcagc cgacaagaag 60

tacagcatcg gcctggacat cggcaccaac tctgtgggct gggccgtgat caccgacgag 120

tacaaggtgc ccagcaagaa attcaaggtg ctgggcaaca ccgaccggca cagcatcaag 180

aagaacctga tcggagccct gctgttcgac agcggcgaaa cagccgaggc cacccggctg 240

aagagaaccg ccagaagaag atacaccaga cggaagaacc ggatctgcta tctgcaagag 300

atcttcagca acgagatggc caaggtggac gacagcttct tccacagact ggaagagtcc 360

ttcctggtgg aagaggataa gaagcacgag cggcacccca tcttcggcaa catcgtggac 420

gaggtggcct accacgagaa gtaccccacc atctaccacc tgagaaagaa actggtggac 480

agcaccgaca aggccgacct gcggctgatc tatctggccc tggcccacat gatcaagttc 540

cggggccact tcctgatcga gggcgacctg aaccccgaca acagcgacgt ggacaagctg 600

ttcatccagc tggtgcagac ctacaaccag ctgttcgagg aaaaccccat caacgccagc 660

ggcgtggacg ccaaggccat cctgtctgcc agactgagca agagcagacg gctggaaaat 720

ctgatcgccc agctgcccgg cgagaagaag aatggcctgt tcggaaacct gattgccctg 780

agcctgggcc tgacccccaa cttcaagagc aacttcgacc tggccgagga tgccaaactg 840

cagctgagca aggacaccta cgacgacgac ctggacaacc tgctggccca gatcggcgac 900

cagtacgccg acctgtttct ggccgccaag aacctgtccg acgccatcct gctgagcgac 960

atcctgagag tgaacaccga gatcaccaag gcccccctga gcgcctctat gatcaagaga 1020

tacgacgagc accaccagga cctgaccctg ctgaaagctc tcgtgcggca gcagctgcct 1080

gagaagtaca aagagatttt cttcgaccag agcaagaacg gctacgccgg ctacattgac 1140

ggcggagcca gccaggaaga gttctacaag ttcatcaagc ccatcctgga aaagatggac 1200

ggcaccgagg aactgctcgt gaagctgaac agagaggacc tgctgcggaa gcagcggacc 1260

ttcgacaacg gcagcatccc ccaccagatc cacctgggag agctgcacgc cattctgcgg 1320

cggcaggaag atttttaccc attcctgaag gacaaccggg aaaagatcga gaagatcctg 1380

accttccgca tcccctacta cgtgggccct ctggccaggg gaaacagcag attcgcctgg 1440

atgaccagaa agagcgagga aaccatcacc ccctggaact tcgaggaagt ggtggacaag 1500

ggcgcttccg cccagagctt catcgagcgg atgaccaact tcgataagaa cctgcccaac 1560

gagaaggtgc tgcccaagca cagcctgctg tacgagtact tcaccgtgta taacgagctg 1620

accaaagtga aatacgtgac cgagggaatg agaaagcccg ccttcctgag cggcgagcag 1680

aaaaaggcca tcgtggacct gctgttcaag accaaccgga aagtgaccgt gaagcagctg 1740

aaagaggact acttcaagaa aatcgagtgc ttcgactccg tggaaatctc cggcgtggaa 1800

gatcggttca acgcctccct gggcacatac cacgatctgc tgaaaattat caaggacaag 1860

gacttcctgg acaatgagga aaacgaggac attctggaag atatcgtgct gaccctgaca 1920

ctgtttgagg acagagagat gatcgaggaa cggctgaaaa cctatgccca cctgttcgac 1980

gacaaagtga tgaagcagct gaagcggcgg agatacaccg gctggggcag gctgagccgg 2040

aagctgatca acggcatccg ggacaagcag tccggcaaga caatcctgga tttcctgaag 2100

tccgacggct tcgccaacag aaacttcatg cagctgatcc acgacgacag cctgaccttt 2160

aaagaggaca tccagaaagc ccaggtgtcc ggccagggcg atagcctgca cgagcacatt 2220

gccaatctgg ccggcagccc cgccattaag aagggcatcc tgcagacagt gaaggtggtg 2280

gacgagctcg tgaaagtgat gggccggcac aagcccgaga acatcgtgat cgaaatggcc 2340

agagagaacc agaccaccca gaagggacag aagaacagcc gcgagagaat gaagcggatc 2400

gaagagggca tcaaagagct gggcagccag atcctgaaag aacaccccgt ggaaaacacc 2460

cagctgcaga acgagaagct gtacctgtac tacctgcaga atgggcggga tatgtacgtg 2520

gaccaggaac tggacatcaa ccggctgtcc gactacgatg tggaccatat cgtgcctcag 2580

agctttctga aggacgactc catcgacaac aaggtgctga ccagaagcga caagaaccgg 2640

ggcaagagcg acaacgtgcc ctccgaagag gtcgtgaaga agatgaagaa ctactggcgg 2700

cagctgctga acgccaagct gattacccag agaaagttcg acaatctgac caaggccgag 2760

agaggcggcc tgagcgaact ggataaggcc ggcttcatca agagacagct ggtggaaacc 2820

cggcagatca caaagcacgt ggcacagatc ctggactccc ggatgaacac taagtacgac 2880

gagaatgaca agctgatccg ggaagtgaaa gtgatcaccc tgaagtccaa gctggtgtcc 2940

gatttccgga aggatttcca gttttacaaa gtgcgcgaga tcaacaacta ccaccacgcc 3000

cacgacgcct acctgaacgc cgtcgtggga accgccctga tcaaaaagta ccctaagctg 3060

gaaagcgagt tcgtgtacgg cgactacaag gtgtacgacg tgcggaagat gatcgccaag 3120

agcgagcagg aaatcggcaa ggctaccgcc aagtacttct tctacagcaa catcatgaac 3180

tttttcaaga ccgagattac cctggccaac ggcgagatcc ggaagcggcc tctgatcgag 3240

acaaacggcg aaaccgggga gatcgtgtgg gataagggcc gggattttgc caccgtgcgg 3300

aaagtgctga gcatgcccca agtgaatatc gtgaaaaaga ccgaggtgca gacaggcggc 3360

ttcagcaaag agtctatcct gcccaagagg aacagcgata agctgatcgc cagaaagaag 3420

gactgggacc ctaagaagta cggcggcttc gacagcccca ccgtggccta ttctgtgctg 3480

gtggtggcca aagtggaaaa gggcaagtcc aagaaactga agagtgtgaa agagctgctg 3540

gggatcacca tcatggaaag aagcagcttc gagaagaatc ccatcgactt tctggaagcc 3600

aagggctaca aagaagtgaa aaaggacctg atcatcaagc tgcctaagta ctccctgttc 3660

gagctggaaa acggccggaa gagaatgctg gcctctgccg gcgaactgca gaagggaaac 3720

gaactggccc tgccctccaa atatgtgaac ttcctgtacc tggccagcca ctatgagaag 3780

ctgaagggct cccccgagga taatgagcag aaacagctgt ttgtggaaca gcacaagcac 3840

tacctggacg agatcatcga gcagatcagc gagttctcca agagagtgat cctggccgac 3900

gctaatctgg acaaagtgct gtccgcctac aacaagcacc gggataagcc catcagagag 3960

caggccgaga atatcatcca cctgtttacc ctgaccaatc tgggagcccc tgccgccttc 4020

aagtactttg acaccaccat cgaccggaag aggtacacca gcaccaaaga ggtgctggac 4080

gccaccctga tccaccagag catcaccggc ctgtacgaga cacggatcga cctgtctcag 4140

ctgggaggcg acgatccaaa aaagaagaga aaggtaggcg gctctggcgg cggctccgga 4200

ggctctgaga tcgccgccct ggaggccaag atcgccgccc tgaaggccaa gaacgccgcc 4260

ctgaaggccg agatcgccgc cctggaggcc taa 4293

<210> 4
<211> 4293
<212> DNA
<213> Artificial Sequence

<220>
<223> Cas9-N6

<400> 4
atggccccaa agaagaagcg gaaggtcggt atccacggag tcccagcagc cgacaagaag 60

tacagcatcg gcctggacat cggcaccaac tctgtgggct gggccgtgat caccgacgag 120

tacaaggtgc ccagcaagaa attcaaggtg ctgggcaaca ccgaccggca cagcatcaag 180

aagaacctga tcggagccct gctgttcgac agcggcgaaa cagccgaggc cacccggctg 240

aagagaaccg ccagaagaag atacaccaga cggaagaacc ggatctgcta tctgcaagag 300

atcttcagca acgagatggc caaggtggac gacagcttct tccacagact ggaagagtcc 360

ttcctggtgg aagaggataa gaagcacgag cggcacccca tcttcggcaa catcgtggac 420

gaggtggcct accacgagaa gtaccccacc atctaccacc tgagaaagaa actggtggac 480

agcaccgaca aggccgacct gcggctgatc tatctggccc tggcccacat gatcaagttc 540

cggggccact tcctgatcga gggcgacctg aaccccgaca acagcgacgt ggacaagctg 600

ttcatccagc tggtgcagac ctacaaccag ctgttcgagg aaaaccccat caacgccagc 660

ggcgtggacg ccaaggccat cctgtctgcc agactgagca agagcagacg gctggaaaat 720

ctgatcgccc agctgcccgg cgagaagaag aatggcctgt tcggaaacct gattgccctg 780

agcctgggcc tgacccccaa cttcaagagc aacttcgacc tggccgagga tgccaaactg 840

cagctgagca aggacaccta cgacgacgac ctggacaacc tgctggccca gatcggcgac 900

cagtacgccg acctgtttct ggccgccaag aacctgtccg acgccatcct gctgagcgac 960

atcctgagag tgaacaccga gatcaccaag gcccccctga gcgcctctat gatcaagaga 1020

tacgacgagc accaccagga cctgaccctg ctgaaagctc tcgtgcggca gcagctgcct 1080

gagaagtaca aagagatttt cttcgaccag agcaagaacg gctacgccgg ctacattgac 1140

ggcggagcca gccaggaaga gttctacaag ttcatcaagc ccatcctgga aaagatggac 1200

ggcaccgagg aactgctcgt gaagctgaac agagaggacc tgctgcggaa gcagcggacc 1260

ttcgacaacg gcagcatccc ccaccagatc cacctgggag agctgcacgc cattctgcgg 1320

cggcaggaag atttttaccc attcctgaag gacaaccggg aaaagatcga gaagatcctg 1380

accttccgca tcccctacta cgtgggccct ctggccaggg gaaacagcag attcgcctgg 1440

atgaccagaa agagcgagga aaccatcacc ccctggaact tcgaggaagt ggtggacaag 1500

ggcgcttccg cccagagctt catcgagcgg atgaccaact tcgataagaa cctgcccaac 1560

gagaaggtgc tgcccaagca cagcctgctg tacgagtact tcaccgtgta taacgagctg 1620

accaaagtga aatacgtgac cgagggaatg agaaagcccg ccttcctgag cggcgagcag 1680

aaaaaggcca tcgtggacct gctgttcaag accaaccgga aagtgaccgt gaagcagctg 1740

aaagaggact acttcaagaa aatcgagtgc ttcgactccg tggaaatctc cggcgtggaa 1800

gatcggttca acgcctccct gggcacatac cacgatctgc tgaaaattat caaggacaag 1860

gacttcctgg acaatgagga aaacgaggac attctggaag atatcgtgct gaccctgaca 1920

ctgtttgagg acagagagat gatcgaggaa cggctgaaaa cctatgccca cctgttcgac 1980

gacaaagtga tgaagcagct gaagcggcgg agatacaccg gctggggcag gctgagccgg 2040

aagctgatca acggcatccg ggacaagcag tccggcaaga caatcctgga tttcctgaag 2100

tccgacggct tcgccaacag aaacttcatg cagctgatcc acgacgacag cctgaccttt 2160

aaagaggaca tccagaaagc ccaggtgtcc ggccagggcg atagcctgca cgagcacatt 2220

gccaatctgg ccggcagccc cgccattaag aagggcatcc tgcagacagt gaaggtggtg 2280

gacgagctcg tgaaagtgat gggccggcac aagcccgaga acatcgtgat cgaaatggcc 2340

agagagaacc agaccaccca gaagggacag aagaacagcc gcgagagaat gaagcggatc 2400

gaagagggca tcaaagagct gggcagccag atcctgaaag aacaccccgt ggaaaacacc 2460

cagctgcaga acgagaagct gtacctgtac tacctgcaga atgggcggga tatgtacgtg 2520

gaccaggaac tggacatcaa ccggctgtcc gactacgatg tggaccatat cgtgcctcag 2580

agctttctga aggacgactc catcgacaac aaggtgctga ccagaagcga caagaaccgg 2640

ggcaagagcg acaacgtgcc ctccgaagag gtcgtgaaga agatgaagaa ctactggcgg 2700

cagctgctga acgccaagct gattacccag agaaagttcg acaatctgac caaggccgag 2760

agaggcggcc tgagcgaact ggataaggcc ggcttcatca agagacagct ggtggaaacc 2820

cggcagatca caaagcacgt ggcacagatc ctggactccc ggatgaacac taagtacgac 2880

gagaatgaca agctgatccg ggaagtgaaa gtgatcaccc tgaagtccaa gctggtgtcc 2940

gatttccgga aggatttcca gttttacaaa gtgcgcgaga tcaacaacta ccaccacgcc 3000

cacgacgcct acctgaacgc cgtcgtggga accgccctga tcaaaaagta ccctaagctg 3060

gaaagcgagt tcgtgtacgg cgactacaag gtgtacgacg tgcggaagat gatcgccaag 3120

agcgagcagg aaatcggcaa ggctaccgcc aagtacttct tctacagcaa catcatgaac 3180

tttttcaaga ccgagattac cctggccaac ggcgagatcc ggaagcggcc tctgatcgag 3240

acaaacggcg aaaccgggga gatcgtgtgg gataagggcc gggattttgc caccgtgcgg 3300

aaagtgctga gcatgcccca agtgaatatc gtgaaaaaga ccgaggtgca gacaggcggc 3360

ttcagcaaag agtctatcct gcccaagagg aacagcgata agctgatcgc cagaaagaag 3420

gactgggacc ctaagaagta cggcggcttc gacagcccca ccgtggccta ttctgtgctg 3480

gtggtggcca aagtggaaaa gggcaagtcc aagaaactga agagtgtgaa agagctgctg 3540

gggatcacca tcatggaaag aagcagcttc gagaagaatc ccatcgactt tctggaagcc 3600

aagggctaca aagaagtgaa aaaggacctg atcatcaagc tgcctaagta ctccctgttc 3660

gagctggaaa acggccggaa gagaatgctg gcctctgccg gcgaactgca gaagggaaac 3720

gaactggccc tgccctccaa atatgtgaac ttcctgtacc tggccagcca ctatgagaag 3780

ctgaagggct cccccgagga taatgagcag aaacagctgt ttgtggaaca gcacaagcac 3840

tacctggacg agatcatcga gcagatcagc gagttctcca agagagtgat cctggccgac 3900

gctaatctgg acaaagtgct gtccgcctac aacaagcacc gggataagcc catcagagag 3960

caggccgaga atatcatcca cctgtttacc ctgaccaatc tgggagcccc tgccgccttc 4020

aagtactttg acaccaccat cgaccggaag aggtacacca gcaccaaaga ggtgctggac 4080

gccaccctga tccaccagag catcaccggc ctgtacgaga cacggatcga cctgtctcag 4140

ctgggaggcg acgatccaaa aaagaagaga aaggtaggcg gctctggcgg cggctccgga 4200

ggctctaaga tcgccgccct gaaggccgag atcgccgccc tggaggccga gaacgccgcc 4260

ctggaggcca agatcgccgc cctgaaggcc taa 4293

<210> 5

<211>  4305
<212>  DNA
<213>  Artificial Sequence

<220>
<223>  Cas9-P3S

<400>  5
atggccccaa agaagaagcg gaaggtcggt atccacggag tcccagcagc cgacaagaag      60

tacagcatcg gcctggacat cggcaccaac tctgtgggct gggccgtgat caccgacgag     120

tacaaggtgc ccagcaagaa attcaaggtg ctgggcaaca ccgaccggca cagcatcaag     180

aagaacctga tcggagccct gctgttcgac agcggcgaaa cagccgaggc cacccggctg     240

aagagaaccg ccagaagaag atacaccaga cggaagaacc ggatctgcta tctgcaagag     300

atcttcagca acgagatggc caaggtggac gacagcttct tccacagact ggaagagtcc     360

ttcctggtgg aagaggataa gaagcacgag cggcacccca tcttcggcaa catcgtggac     420

gaggtggcct accacgagaa gtaccccacc atctaccacc tgagaaagaa actggtggac     480

agcaccgaca aggccgacct gcggctgatc tatctggccc tggcccacat gatcaagttc     540

cggggccact tcctgatcga gggcgacctg aaccccgaca acagcgacgt ggacaagctg     600

ttcatccagc tggtgcagac ctacaaccag ctgttcgagg aaaaccccat caacgccagc     660

ggcgtggacg ccaaggccat cctgtctgcc agactgagca agagcagacg gctggaaaat     720

ctgatcgccc agctgcccgg cgagaagaag aatggcctgt tcggaaacct gattgccctg     780

agcctgggcc tgacccccaa cttcaagagc aacttcgacc tggccgagga tgccaaactg     840

cagctgagca aggacaccta cgacgacgac ctggacaacc tgctggccca gatcggcgac     900

cagtacgccg acctgtttct ggccgccaag aacctgtccg acgccatcct gctgagcgac     960

atcctgagag tgaacaccga gatcaccaag gcccccctga gcgcctctat gatcaagaga    1020

tacgacgagc accaccagga cctgaccctg ctgaaagctc tcgtgcggca gcagctgcct    1080

gagaagtaca aagagatttt cttcgaccag agcaagaacg gctacgccgg ctacattgac    1140

ggcggagcca gccaggaaga gttctacaag ttcatcaagc ccatcctgga aaagatggac    1200

ggcaccgagg aactgctcgt gaagctgaac agagaggacc tgctgcggaa gcagcggacc    1260

ttcgacaacg gcagcatccc ccaccagatc cacctgggag agctgcacgc cattctgcgg    1320

cggcaggaag atttttaccc attcctgaag gacaaccggg aaaagatcga gaagatcctg    1380

accttccgca tcccctacta cgtgggccct ctggccaggg gaaacagcag attcgcctgg    1440

atgaccagaa agagcgagga aaccatcacc ccctggaact tcgaggaagt ggtggacaag    1500

ggcgcttccg cccagagctt catcgagcgg atgaccaact tcgataagaa cctgcccaac    1560

gagaaggtgc tgcccaagca cagcctgctg tacgagtact tcaccgtgta taacgagctg    1620

accaaagtga aatacgtgac cgagggaatg agaaagcccg ccttcctgag cggcgagcag    1680

aaaaaggcca tcgtggacct gctgttcaag accaaccgga aagtgaccgt gaagcagctg 1740

aaagaggact acttcaagaa aatcgagtgc ttcgactccg tggaaatctc cggcgtggaa 1800

gatcggttca acgcctccct gggcacatac cacgatctgc tgaaaattat caaggacaag 1860

gacttcctgg acaatgagga aaacgaggac attctggaag atatcgtgct gaccctgaca 1920

ctgtttgagg acagagagat gatcgaggaa cggctgaaaa cctatgccca cctgttcgac 1980

gacaaagtga tgaagcagct gaagcggcgg agatacaccg gctggggcag gctgagccgg 2040

aagctgatca acggcatccg ggacaagcag tccggcaaga caatcctgga tttcctgaag 2100

tccgacggct tcgccaacag aaacttcatg cagctgatcc acgacgacag cctgaccttt 2160

aaagaggaca tccagaaagc ccaggtgtcc ggccagggcg atagcctgca cgagcacatt 2220

gccaatctgg ccggcagccc cgccattaag aagggcatcc tgcagacagt gaaggtggtg 2280

gacgagctcg tgaaagtgat gggccggcac aagcccgaga acatcgtgat cgaaatggcc 2340

agagagaacc agaccaccca gaagggacag aagaacagcc gcgagagaat gaagcggatc 2400

gaagagggca tcaaagagct gggcagccag atcctgaaag aacaccccgt ggaaaacacc 2460

cagctgcaga acgagaagct gtacctgtac tacctgcaga atgggcggga tatgtacgtg 2520

gaccaggaac tggacatcaa ccggctgtcc gactacgatg tggaccatat cgtgcctcag 2580

agctttctga aggacgactc catcgacaac aaggtgctga ccagaagcga caagaaccgg 2640

ggcaagagcg acaacgtgcc ctccgaagag gtcgtgaaga agatgaagaa ctactggcgg 2700

cagctgctga acgccaagct gattacccag agaaagttcg acaatctgac caaggccgag 2760

agaggcggcc tgagcgaact ggataaggcc ggcttcatca agagacagct ggtggaaacc 2820

cggcagatca caaagcacgt ggcacagatc ctggactccc ggatgaacac taagtacgac 2880

gagaatgaca agctgatccg ggaagtgaaa gtgatcaccc tgaagtccaa gctggtgtcc 2940

gatttccgga aggatttcca gttttacaaa gtgcgcgaga tcaacaacta ccaccacgcc 3000

cacgacgcct acctgaacgc cgtcgtggga accgccctga tcaaaaagta ccctaagctg 3060

gaaagcgagt tcgtgtacgg cgactacaag gtgtacgacg tgcggaagat gatcgccaag 3120

agcgagcagg aaatcggcaa ggctaccgcc aagtacttct tctacagcaa catcatgaac 3180

tttttcaaga ccgagattac cctggccaac ggcgagatcc ggaagcggcc tctgatcgag 3240

acaaacggcg aaaccgggga gatcgtgtgg gataagggcc gggattttgc caccgtgcgg 3300

aaagtgctga gcatgcccca agtgaatatc gtgaaaaaga ccgaggtgca gacaggcggc 3360

ttcagcaaag agtctatcct gcccaagagg aacagcgata agctgatcgc cagaaagaag 3420

gactgggacc ctaagaagta cggcggcttc gacagcccca ccgtggccta ttctgtgctg 3480

gtggtggcca aagtggaaaa gggcaagtcc aagaaactga agagtgtgaa agagctgctg 3540

gggatcacca tcatggaaag aagcagcttc gagaagaatc ccatcgactt tctggaagcc 3600

aagggctaca aagaagtgaa aaaggacctg atcatcaagc tgcctaagta ctccctgttc 3660

gagctggaaa acggccggaa gagaatgctg gcctctgccg gcgaactgca gaagggaaac 3720

gaactggccc tgccctccaa atatgtgaac ttcctgtacc tggccagcca ctatgagaag 3780

ctgaagggct cccccgagga taatgagcag aaacagctgt ttgtggaaca gcacaagcac 3840

tacctggacg agatcatcga gcagatcagc gagttctcca agagagtgat cctggccgac 3900

gctaatctgg acaaagtgct gtccgcctac aacaagcacc gggataagcc catcagagag 3960

caggccgaga atatcatcca cctgtttacc ctgaccaatc tgggagcccc tgccgccttc 4020

aagtactttg acaccaccat cgaccggaag aggtacacca gcaccaaaga ggtgctggac 4080

gccaccctga tccaccagag catcaccggc ctgtacgaga cacggatcga cctgtctcag 4140

ctgggaggcg acgatccaaa aaagaagaga aaggtaggcg gctctggcgg cggctccgga 4200

ggctctagcc ccgaggacga gatccagcag ctggaggagg agatcagcca gctggagcag 4260

aagaacagcc agctgaagga gaagaaccag cagctgaagt actaa 4305

<210> 6
<211> 4305
<212> DNA
<213> Artificial Sequence

<220>
<223> Cas9-P4S

<400> 6
atggccccaa agaagaagcg gaaggtcggt atccacggag tcccagcagc cgacaagaag 60

tacagcatcg gcctggacat cggcaccaac tctgtgggct gggccgtgat caccgacgag 120

tacaaggtgc ccagcaagaa attcaaggtg ctgggcaaca ccgaccggca cagcatcaag 180

aagaacctga tcggagccct gctgttcgac agcggcgaaa cagccgaggc cacccggctg 240

aagagaaccg ccagaagaag atacaccaga cggaagaacc ggatctgcta tctgcaagag 300

atcttcagca acgagatggc caaggtggac gacagcttct tccacagact ggaagagtcc 360

ttcctggtgg aagaggataa gaagcacgag cggcacccca tcttcggcaa catcgtggac 420

gaggtggcct accacgagaa gtaccccacc atctaccacc tgagaaagaa actggtggac 480

agcaccgaca aggccgacct gcggctgatc tatctggccc tggcccacat gatcaagttc 540

cggggccact tcctgatcga gggcgacctg aaccccgaca acagcgacgt ggacaagctg 600

ttcatccagc tggtgcagac ctacaaccag ctgttcgagg aaaaccccat caacgccagc 660

ggcgtggacg ccaaggccat cctgtctgcc agactgagca agagcagacg gctggaaaat 720

ctgatcgccc agctgcccgg cgagaagaag aatggcctgt tcggaaacct gattgccctg 780

agcctgggcc tgacccccaa cttcaagagc aacttcgacc tggccgagga tgccaaactg 840

cagctgagca aggacaccta cgacgacgac ctggacaacc tgctggccca gatcggcgac 900

cagtacgccg acctgtttct ggccgccaag aacctgtccg acgccatcct gctgagcgac 960

atcctgagag tgaacaccga gatcaccaag gcccccctga gcgcctctat gatcaagaga 1020

tacgacgagc accaccagga cctgaccctg ctgaaagctc tcgtgcggca gcagctgcct 1080

gagaagtaca aagagatttt cttcgaccag agcaagaacg gctacgccgg ctacattgac 1140

ggcggagcca gccaggaaga gttctacaag ttcatcaagc ccatcctgga aaagatggac 1200

ggcaccgagg aactgctcgt gaagctgaac agagaggacc tgctgcggaa gcagcggacc 1260

ttcgacaacg gcagcatccc ccaccagatc cacctgggag agctgcacgc cattctgcgg 1320

cggcaggaag atttttaccc attcctgaag gacaaccggg aaaagatcga gaagatcctg 1380

accttccgca tcccctacta cgtgggccct ctggccaggg gaaacagcag attcgcctgg 1440

atgaccagaa agagcgagga aaccatcacc ccctggaact tcgaggaagt ggtggacaag 1500

ggcgcttccg cccagagctt catcgagcgg atgaccaact tcgataagaa cctgcccaac 1560

gagaaggtgc tgcccaagca cagcctgctg tacgagtact tcaccgtgta taacgagctg 1620

accaaagtga aatacgtgac cgagggaatg agaaagcccg ccttcctgag cggcgagcag 1680

aaaaaggcca tcgtggacct gctgttcaag accaaccgga aagtgaccgt gaagcagctg 1740

aaagaggact acttcaagaa aatcgagtgc ttcgactccg tggaaatctc cggcgtggaa 1800

gatcggttca acgcctccct gggcacatac cacgatctgc tgaaaattat caaggacaag 1860

gacttcctgg acaatgagga aaacgaggac attctggaag atatcgtgct gaccctgaca 1920

ctgtttgagg acagagagat gatcgaggaa cggctgaaaa cctatgccca cctgttcgac 1980

gacaaagtga tgaagcagct gaagcggcgg agatacaccg gctggggcag gctgagccgg 2040

aagctgatca acggcatccg ggacaagcag tccggcaaga caatcctgga tttcctgaag 2100

tccgacggct tcgccaacag aaacttcatg cagctgatcc acgacgacag cctgaccttt 2160

aaagaggaca tccagaaagc ccaggtgtcc ggccagggcg atagcctgca cgagcacatt 2220

gccaatctgg ccggcagccc cgccattaag aagggcatcc tgcagacagt gaaggtggtg 2280

gacgagctcg tgaaagtgat gggccggcac aagcccgaga acatcgtgat cgaaatggcc 2340

agagagaacc agaccaccca gaagggacag aagaacagcc gcgagagaat gaagcggatc 2400

gaagagggca tcaaagagct gggcagccag atcctgaaag aacaccccgt ggaaaacacc 2460

cagctgcaga acgagaagct gtacctgtac tacctgcaga atgggcggga tatgtacgtg 2520

gaccaggaac tggacatcaa ccggctgtcc gactacgatg tggaccatat cgtgcctcag 2580

agctttctga aggacgactc catcgacaac aaggtgctga ccagaagcga caagaaccgg 2640

ggcaagagcg acaacgtgcc ctccgaagag gtcgtgaaga agatgaagaa ctactggcgg 2700

cagctgctga acgccaagct gattacccag agaaagttcg acaatctgac caaggccgag 2760

agaggcggcc tgagcgaact ggataaggcc ggcttcatca agagacagct ggtggaaacc 2820

cggcagatca caaagcacgt ggcacagatc ctggactccc ggatgaacac taagtacgac 2880

gagaatgaca agctgatccg ggaagtgaaa gtgatcaccc tgaagtccaa gctggtgtcc 2940

gatttccgga aggatttcca gttttacaaa gtgcgcgaga tcaacaacta ccaccacgcc 3000

cacgacgcct acctgaacgc cgtcgtggga accgccctga tcaaaaagta ccctaagctg 3060

gaaagcgagt tcgtgtacgg cgactacaag gtgtacgacg tgcggaagat gatcgccaag 3120

agcgagcagg aaatcggcaa ggctaccgcc aagtacttct tctacagcaa catcatgaac 3180

tttttcaaga ccgagattac cctggccaac ggcgagatcc ggaagcggcc tctgatcgag 3240

acaaacggcg aaaccgggga gatcgtgtgg gataagggcc gggattttgc caccgtgcgg 3300

aaagtgctga gcatgcccca agtgaatatc gtgaaaaaga ccgaggtgca gacaggcggc 3360

ttcagcaaag agtctatcct gcccaagagg aacagcgata agctgatcgc cagaaagaag 3420

gactgggacc ctaagaagta cggcggcttc gacagcccca ccgtggccta ttctgtgctg 3480

gtggtggcca aagtggaaaa gggcaagtcc aagaaactga agagtgtgaa agagctgctg 3540

gggatcacca tcatggaaag aagcagcttc gagaagaatc ccatcgactt tctggaagcc 3600

aagggctaca aagaagtgaa aaaggacctg atcatcaagc tgcctaagta ctccctgttc 3660

gagctggaaa acggccggaa gagaatgctg gcctctgccg gcgaactgca gaagggaaac 3720

gaactggccc tgccctccaa atatgtgaac ttcctgtacc tggccagcca ctatgagaag 3780

ctgaagggct cccccgagga taatgagcag aaacagctgt ttgtggaaca gcacaagcac 3840

tacctggacg agatcatcga gcagatcagc gagttctcca agagagtgat cctggccgac 3900

gctaatctgg acaaagtgct gtccgcctac aacaagcacc gggataagcc catcagagag 3960

caggccgaga atatcatcca cctgtttacc ctgaccaatc tgggagcccc tgccgccttc 4020

aagtactttg acaccaccat cgaccggaag aggtacacca gcaccaaaga ggtgctggac 4080

gccaccctga tccaccagag catcaccggc ctgtacgaga cacggatcga cctgtctcag 4140

ctgggaggcg acgatccaaa aaagaagaga aaggtaggcg gctctggcgg cggctccgga 4200

ggctctagcc ccgaggacaa gatcagccag ctgaagcaga agatccagca gctgaagcag 4260

gagaaccagc agctggagga ggagaacagc cagctggagt actaa 4305

<210> 7
<211> 4305
<212> DNA
<213> Artificial Sequence

<220>
<223> Cas9-AP4

<400> 7
atggccccaa agaagaagcg gaaggtcggt atccacggag tcccagcagc cgacaagaag 60

tacagcatcg gcctggacat cggcaccaac tctgtgggct gggccgtgat caccgacgag 120

tacaaggtgc ccagcaagaa attcaaggtg ctgggcaaca ccgaccggca cagcatcaag 180

aagaacctga tcggagccct gctgttcgac agcggcgaaa cagccgaggc cacccggctg 240

aagagaaccg ccagaagaag atacaccaga cggaagaacc ggatctgcta tctgcaagag 300

atcttcagca acgagatggc caaggtggac gacagcttct tccacagact ggaagagtcc 360

ttcctggtgg aagaggataa gaagcacgag cggcacccca tcttcggcaa catcgtggac 420

gaggtggcct accacgagaa gtaccccacc atctaccacc tgagaaagaa actggtggac 480

agcaccgaca aggccgacct gcggctgatc tatctggccc tggcccacat gatcaagttc 540

cggggccact tcctgatcga gggcgacctg aaccccgaca acagcgacgt ggacaagctg 600

ttcatccagc tggtgcagac ctacaaccag ctgttcgagg aaaaccccat caacgccagc 660

ggcgtggacg ccaaggccat cctgtctgcc agactgagca agagcagacg gctggaaaat 720

ctgatcgccc agctgcccgg cgagaagaag aatggcctgt tcggaaacct gattgccctg 780

agcctgggcc tgacccccaa cttcaagagc aacttcgacc tggccgagga tgccaaactg 840

cagctgagca aggacaccta cgacgacgac ctggacaacc tgctggccca gatcggcgac 900

cagtacgccg acctgtttct ggccgccaag aacctgtccg acgccatcct gctgagcgac 960

atcctgagag tgaacaccga gatcaccaag gcccccctga gcgcctctat gatcaagaga 1020

tacgacgagc accaccagga cctgaccctg ctgaaagctc tcgtgcggca gcagctgcct 1080

gagaagtaca aagagatttt cttcgaccag agcaagaacg gctacgccgg ctacattgac 1140

ggcggagcca gccaggaaga gttctacaag ttcatcaagc ccatcctgga aaagatggac 1200

ggcaccgagg aactgctcgt gaagctgaac agagaggacc tgctgcggaa gcagcggacc 1260

ttcgacaacg gcagcatccc ccaccagatc cacctgggag agctgcacgc cattctgcgg 1320

cggcaggaag atttttaccc attcctgaag gacaaccggg aaaagatcga gaagatcctg 1380

accttccgca tcccctacta cgtgggccct ctggccaggg gaaacagcag attcgcctgg 1440

atgaccagaa agagcgagga aaccatcacc ccctggaact tcgaggaagt ggtggacaag 1500

ggcgcttccg cccagagctt catcgagcgg atgaccaact tcgataagaa cctgcccaac 1560

gagaaggtgc tgcccaagca cagcctgctg tacgagtact tcaccgtgta taacgagctg 1620

accaaagtga aatacgtgac cgagggaatg agaaagcccg ccttcctgag cggcgagcag 1680

aaaaaggcca tcgtggacct gctgttcaag accaaccgga aagtgaccgt gaagcagctg 1740

aaagaggact acttcaagaa aatcgagtgc ttcgactccg tggaaatctc cggcgtggaa 1800

gatcggttca acgcctccct gggcacatac cacgatctgc tgaaaattat caaggacaag 1860

gacttcctgg acaatgagga aaacgaggac attctggaag atatcgtgct gaccctgaca 1920
ctgtttgagg acagagagat gatcgaggaa cggctgaaaa cctatgccca cctgttcgac 1980
gacaaagtga tgaagcagct gaagcggcgg agatacaccg gctggggcag gctgagccgg 2040
aagctgatca acggcatccg ggacaagcag tccggcaaga caatcctgga tttcctgaag 2100
tccgacggct tcgccaacag aaacttcatg cagctgatcc acgacgacag cctgaccttt 2160
aaagaggaca tccagaaagc ccaggtgtcc ggccagggcg atagcctgca cgagcacatt 2220
gccaatctgg ccggcagccc cgccattaag aagggcatcc tgcagacagt gaaggtggtg 2280
gacgagctcg tgaaagtgat gggccggcac aagcccgaga acatcgtgat cgaaatggcc 2340
agagagaacc agaccaccca gaagggacag aagaacagcc gcgagagaat gaagcggatc 2400
gaagagggca tcaaagagct gggcagccag atcctgaaag aacaccccgt ggaaaacacc 2460
cagctgcaga acgagaagct gtacctgtac tacctgcaga atgggcggga tatgtacgtg 2520
gaccaggaac tggacatcaa ccggctgtcc gactacgatg tggaccatat cgtgcctcag 2580
agctttctga aggacgactc catcgacaac aaggtgctga ccagaagcga caagaaccgg 2640
ggcaagagcg acaacgtgcc ctccgaagag gtcgtgaaga agatgaagaa ctactggcgg 2700
cagctgctga acgccaagct gattacccag agaaagttcg acaatctgac caaggccgag 2760
agaggcggcc tgagcgaact ggataaggcc ggcttcatca agagacagct ggtggaaacc 2820
cggcagatca caaagcacgt ggcacagatc ctggactccc ggatgaacac taagtacgac 2880
gagaatgaca agctgatccg ggaagtgaaa gtgatcaccc tgaagtccaa gctggtgtcc 2940
gatttccgga aggatttcca gttttacaaa gtgcgcgaga tcaacaacta ccaccacgcc 3000
cacgacgcct acctgaacgc cgtcgtggga accgccctga tcaaaaagta ccctaagctg 3060
gaaagcgagt tcgtgtacgg cgactacaag gtgtacgacg tgcggaagat gatcgccaag 3120
agcgagcagg aaatcggcaa ggctaccgcc aagtacttct tctacagcaa catcatgaac 3180
tttttcaaga ccgagattac cctggccaac ggcgagatcc ggaagcggcc tctgatcgag 3240
acaaacggcg aaaccgggga gatcgtgtgg gataagggcc gggattttgc caccgtgcgg 3300
aaagtgctga gcatgcccca agtgaatatc gtgaaaaaga ccgaggtgca gacaggcggc 3360
ttcagcaaag agtctatcct gcccaagagg aacagcgata agctgatcgc cagaaagaag 3420
gactgggacc ctaagaagta cggcggcttc gacagcccca ccgtggccta ttctgtgctg 3480
gtggtggcca aagtggaaaa gggcaagtcc aagaaactga agagtgtgaa agagctgctg 3540
gggatcacca tcatggaaag aagcagcttc gagaagaatc ccatcgactt tctggaagcc 3600
aagggctaca aagaagtgaa aaaggacctg atcatcaagc tgcctaagta ctccctgttc 3660
gagctggaaa acggccggaa gagaatgctg gcctctgccg gcgaactgca gaagggaaac 3720
gaactggccc tgccctccaa atatgtgaac ttcctgtacc tggccagcca ctatgagaag 3780

ctgaagggct cccccgagga taatgagcag aaacagctgt ttgtggaaca gcacaagcac 3840

tacctggacg agatcatcga gcagatcagc gagttctcca agagagtgat cctggccgac 3900

gctaatctgg acaaagtgct gtccgcctac aacaagcacc gggataagcc catcagagag 3960

caggccgaga atatcatcca cctgtttacc ctgaccaatc tgggagcccc tgccgccttc 4020

aagtactttg acaccaccat cgaccggaag aggtacacca gcaccaaaga ggtgctggac 4080

gccaccctga tccaccagag catcaccggc ctgtacgaga cacggatcga cctgtctcag 4140

ctgggaggcg acgatccaaa aaagaagaga aaggtaggcg gctctggcgg cggctccgga 4200

ggctctagcc ccgaggacga gctggccgcc aacgaggagg agctgcagca gaacgagcag 4260

aagctggccc agatcaagca gaagctgcag gccatcaagt actaa 4305

<210> 8
<211> 957
<212> DNA
<213> Artificial Sequence

<220>
<223> P4-EXOIII

<400> 8
agcccggaag ataaaattgc tcagctgaaa caaaaaatcc aagcgctgaa acaggaaaac 60

cagcagctgg aagaggaaaa cgccgcactg gaatatggtg gcggctctgg cggcggctcc 120

ggaggctctg atccaaaaaa gaagagaaag gtaaaatttg tctcttttaa tatcaacggc 180

ctgcgcgcca gacctcacca gcttgaagcc atcgtcgaaa agcaccaacc ggatgtgatt 240

ggcctgcagg agacaaaagt tcatgacgat atgtttccgc tcgaagaggt ggcgaagctc 300

ggctacaacg tgttttatca cgggcagaaa ggccattatg gcgtggcgct gctgaccaaa 360

gagacgccga ttgccgtgcg tcgcggcttt cccggtgacg acgaagaggc gcagcggcgg 420

attattatgg cggaaatccc ctcactgctg ggtaatgtca ccgtgatcaa cggttacttc 480

ccgcagggtg aaagccgcga ccatccgata aaattcccgg caaaagcgca gttttatcag 540

aatctgcaaa actacctgga aaccgaactc aaacgtgata atccggtact gattatgggc 600

gatatgaata tcagccctac agatctggac atcggcattg gcgaagaaaa ccgtaagcgc 660

tggctgcgta ccggtaaatg ctctttcctg ccggaagagc gcgaatggat ggacaggctg 720

atgagctggg ggttggtcga taccttccgc catgcgaatc cgcaaacagc agatcgtttc 780

tcatggtttg attaccgctc aaaaggtttt gacgataacc gtggtctgcg catcgacctg 840

ctgctcgcca gccaaccgct ggcagaatgt tgcgtagaaa ccggcatcga ctatgaaatc 900

cgcagcatgg aaaaaccgtc cgatcacgcc cccgtctggg cgaccttccg ccgctaa 957

<210> 9

<211>  957
<212>  DNA
<213>  Artificial Sequence

<220>
<223>  P3-EXOIII

<400>  9
tccccggaag atgagatcca gcaactggaa gaagaaatcg ctcagctgga acagaaaaac      60

gcagcgctga aagagaaaaa ccaggcgctg aaatacggtg gcggctctgg cggcggctcc     120

ggaggctctg atccaaaaaa gaagagaaag gtaaaatttg tctcttttaa tatcaacggc     180

ctgcgcgcca gacctcacca gcttgaagcc atcgtcgaaa agcaccaacc ggatgtgatt     240

ggcctgcagg agacaaaagt tcatgacgat atgtttccgc tcgaagaggt ggcgaagctc     300

ggctacaacg tgttttatca cgggcagaaa ggccattatg gcgtggcgct gctgaccaaa     360

gagacgccga ttgccgtgcg tcgcggcttt cccggtgacg acgaagaggc gcagcggcgg     420

attattatgg cggaaatccc ctcactgctg ggtaatgtca ccgtgatcaa cggttacttc     480

ccgcagggtg aaagccgcga ccatccgata aaattcccgg caaaagcgca gttttatcag     540

aatctgcaaa actacctgga aaccgaactc aaacgtgata atccggtact gattatgggc     600

gatatgaata tcagccctac agatctggac atcggcattg gcgaagaaaa ccgtaagcgc     660

tggctgcgta ccggtaaatg ctctttcctg ccggaagagc gcgaatggat ggacaggctg     720

atgagctggg ggttggtcga taccttccgc catgcgaatc cgcaaacagc agatcgtttc     780

tcatggtttg attaccgctc aaaaggtttt gacgataacc gtggtctgcg catcgacctg     840

ctgctcgcca gccaaccgct ggcagaatgt tgcgtagaaa ccggcatcga ctatgaaatc     900

cgcagcatgg aaaaaccgtc cgatcacgcc cccgtctggg cgaccttccg ccgctaa        957

<210>  10
<211>  939
<212>  DNA
<213>  Artificial Sequence

<220>
<223>  N5-EXOIII

<400>  10
gagatcgccg ccctggaggc caagatcgcc gccctgaagg ccaagaacgc cgccctgaag      60

gccgagatcg ccgccctgga ggccggcggc tctggcggcg gctccggagg ctctgatcca     120

aaaaagaaga gaaaggtaaa atttgtctct tttaatatca acggcctgcg cgccagacct     180

caccagcttg aagccatcgt cgaaaagcac caaccggatg tgattggcct gcaggagaca     240

aaagttcatg acgatatgtt tccgctcgaa gaggtggcga agctcggcta caacgtgttt     300

tatcacgggc agaaaggcca ttatggcgtg gcgctgctga ccaaagagac gccgattgcc     360

gtgcgtcgcg gctttcccgg tgacgacgaa gaggcgcagc ggcggattat tatggcggaa     420

atcccctcac tgctgggtaa tgtcaccgtg atcaacggtt acttcccgca gggtgaaagc 480

cgcgaccatc cgataaaatt cccggcaaaa gcgcagtttt atcagaatct gcaaaactac 540

ctggaaaccg aactcaaacg tgataatccg gtactgatta tgggcgatat gaatatcagc 600

cctacagatc tggacatcgg cattggcgaa gaaaaccgta agcgctggct gcgtaccggt 660

aaatgctctt tcctgccgga agagcgcgaa tggatggaca ggctgatgag ctgggggttg 720

gtcgatacct tccgccatgc gaatccgcaa acagcagatc gtttctcatg gtttgattac 780

cgctcaaaag gttttgacga taaccgtggt ctgcgcatcg acctgctgct cgccagccaa 840

ccgctggcag aatgttgcgt agaaaccggc atcgactatg aaatccgcag catggaaaaa 900

ccgtccgatc acgcccccgt ctgggcgacc ttccgccgc 939

<210> 11
<211> 939
<212> DNA
<213> Artificial Sequence

<220>
<223> N6-EXOIII

<400> 11
aagatcgccg ccctgaaggc cgagatcgcc gccctggagg ccgagaacgc cgccctggag 60

gccaagatcg ccgccctgaa ggccggcggc tctggcggcg gctccggagg ctctgatcca 120

aaaaagaaga gaaaggtaaa atttgtctct tttaatatca acggcctgcg cgccagacct 180

caccagcttg aagccatcgt cgaaaagcac caaccggatg tgattggcct gcaggagaca 240

aaagttcatg acgatatgtt tccgctcgaa gaggtggcga agctcggcta caacgtgttt 300

tatcacgggc agaaaggcca ttatggcgtg gcgctgctga ccaaagagac gccgattgcc 360

gtgcgtcgcg gctttcccgg tgacgacgaa gaggcgcagc ggcggattat tatggcggaa 420

atcccctcac tgctgggtaa tgtcaccgtg atcaacggtt acttcccgca gggtgaaagc 480

cgcgaccatc cgataaaatt cccggcaaaa gcgcagtttt atcagaatct gcaaaactac 540

ctggaaaccg aactcaaacg tgataatccg gtactgatta tgggcgatat gaatatcagc 600

cctacagatc tggacatcgg cattggcgaa gaaaaccgta agcgctggct gcgtaccggt 660

aaatgctctt tcctgccgga agagcgcgaa tggatggaca ggctgatgag ctgggggttg 720

gtcgatacct tccgccatgc gaatccgcaa acagcagatc gtttctcatg gtttgattac 780

cgctcaaaag gttttgacga taaccgtggt ctgcgcatcg acctgctgct cgccagccaa 840

ccgctggcag aatgttgcgt agaaaccggc atcgactatg aaatccgcag catggaaaaa 900

ccgtccgatc acgcccccgt ctgggcgacc ttccgccgc 939

<210> 12

<211>  951
<212>  DNA
<213>  Artificial Sequence

<220>
<223>  P3S-EXOIII

<400>  12
agccccgagg acgagatcca gcagctggag gaggagatca gccagctgga gcagaagaac      60

agccagctga aggagaagaa ccagcagctg aagtacggcg gctctggcgg cggctccgga     120

ggctctgatc caaaaaagaa gagaaaggta aaatttgtct cttttaatat caacggcctg     180

cgcgccagac ctcaccagct tgaagccatc gtcgaaaagc accaaccgga tgtgattggc     240

ctgcaggaga caaaagttca tgacgatatg tttccgctcg aagaggtggc gaagctcggc     300

tacaacgtgt tttatcacgg gcagaaaggc cattatggcg tggcgctgct gaccaaagag     360

acgccgattg ccgtgcgtcg cggctttccc ggtgacgacg aagaggcgca gcggcggatt     420

attatggcgg aaatcccctc actgctgggt aatgtcaccg tgatcaacgg ttacttcccg     480

cagggtgaaa gccgcgacca tccgataaaa ttcccggcaa aagcgcagtt ttatcagaat     540

ctgcaaaact acctggaaac cgaactcaaa cgtgataatc cggtactgat tatgggcgat     600

atgaatatca gccctacaga tctggacatc ggcattggcg aagaaaaccg taagcgctgg     660

ctgcgtaccg gtaaatgctc tttcctgccg gaagagcgcg aatggatgga caggctgatg     720

agctgggggt tggtcgatac cttccgccat gcgaatccgc aaacagcaga tcgtttctca     780

tggtttgatt accgctcaaa aggttttgac gataaccgtg gtctgcgcat cgacctgctg     840

ctcgccagcc aaccgctggc agaatgttgc gtagaaaccg gcatcgacta tgaaatccgc     900

agcatggaaa aaccgtccga tcacgccccc gtctgggcga ccttccgccg c              951


<210>  13
<211>  951
<212>  DNA
<213>  Artificial Sequence

<220>
<223>  P4S-EXOIII

<400>  13
agccccgagg acaagatcag ccagctgaag cagaagatcc agcagctgaa gcaggagaac      60

cagcagctgg aggaggagaa cagccagctg gagtacggcg gctctggcgg cggctccgga     120

ggctctgatc caaaaaagaa gagaaaggta aaatttgtct cttttaatat caacggcctg     180

cgcgccagac ctcaccagct tgaagccatc gtcgaaaagc accaaccgga tgtgattggc     240

ctgcaggaga caaaagttca tgacgatatg tttccgctcg aagaggtggc gaagctcggc     300

tacaacgtgt tttatcacgg gcagaaaggc cattatggcg tggcgctgct gaccaaagag     360

acgccgattg ccgtgcgtcg cggctttccc ggtgacgacg aagaggcgca gcggcggatt     420

attatggcgg aaatcccctc actgctgggt aatgtcaccg tgatcaacgg ttacttcccg 480

cagggtgaaa gccgcgacca tccgataaaa ttcccggcaa aagcgcagtt ttatcagaat 540

ctgcaaaact acctggaaac cgaactcaaa cgtgataatc cggtactgat tatgggcgat 600

atgaatatca gccctacaga tctggacatc ggcattggcg aagaaaaccg taagcgctgg 660

ctgcgtaccg gtaaatgctc tttcctgccg gaagagcgcg aatggatgga caggctgatg 720

agctgggggt tggtcgatac cttccgccat gcgaatccgc aaacagcaga tcgtttctca 780

tggtttgatt accgctcaaa aggttttgac gataaccgtg gtctgcgcat cgacctgctg 840

ctcgccagcc aaccgctggc agaatgttgc gtagaaaccg gcatcgacta tgaaatccgc 900

agcatggaaa aaccgtccga tcacgccccc gtctgggcga ccttccgccg c 951

<210> 14
<211> 951
<212> DNA
<213> Artificial Sequence

<220>
<223> AP4-EXOIII

<400> 14
agccccgagg acgagctggc cgccaacgag gaggagctgc agcagaacga gcagaagctg 60

gcccagatca agcagaagct gcaggccatc aagtacggcg gctctggcgg cggctccgga 120

ggctctgatc caaaaaagaa gagaaaggta aaatttgtct cttttaatat caacggcctg 180

cgcgccagac ctcaccagct tgaagccatc gtcgaaaagc accaaccgga tgtgattggc 240

ctgcaggaga caaaagttca tgacgatatg tttccgctcg aagaggtggc gaagctcggc 300

tacaacgtgt tttatcacgg gcagaaaggc cattatggcg tggcgctgct gaccaaagag 360

acgccgattg ccgtgcgtcg cggctttccc ggtgacgacg aagaggcgca gcggcggatt 420

attatggcgg aaatcccctc actgctgggt aatgtcaccg tgatcaacgg ttacttcccg 480

cagggtgaaa gccgcgacca tccgataaaa ttcccggcaa aagcgcagtt ttatcagaat 540

ctgcaaaact acctggaaac cgaactcaaa cgtgataatc cggtactgat tatgggcgat 600

atgaatatca gccctacaga tctggacatc ggcattggcg aagaaaaccg taagcgctgg 660

ctgcgtaccg gtaaatgctc tttcctgccg gaagagcgcg aatggatgga caggctgatg 720

agctgggggt tggtcgatac cttccgccat gcgaatccgc aaacagcaga tcgtttctca 780

tggtttgatt accgctcaaa aggttttgac gataaccgtg gtctgcgcat cgacctgctg 840

ctcgccagcc aaccgctggc agaatgttgc gtagaaaccg gcatcgacta tgaaatccgc 900

agcatggaaa aaccgtccga tcacgccccc gtctgggcga ccttccgccg c 951

<210> 15

<211>  840
<212>  DNA
<213>  Artificial Sequence

<220>
<223>  P3-TREX2

<400>  15
tccccggaag atgagatcca gcaactggaa gaagaaatcg ctcagctgga acagaaaaac      60

gcagcgctga aagagaaaaa ccaggcgctg aaatacggtg gcggctctgg cggcggctcc     120

ggaggctcta tgtctgagcc acctcgggct gagacctttg tattcctgga cctagaagcc     180

actgggctcc caaacatgga ccctgagatt gcagagatat ccctttttgc tgttcaccgc     240

tcttccctgg agaacccaga acgggatgat tctggttcct tggtgctgcc ccgtgttctg     300

gacaagctca cactgtgcat gtgcccggag cgccccttta ctgccaaggc cagtgagatt     360

actggtttga gcagcgaaag cctgatgcac tgcgggaagg ctggtttcaa tggcgctgtg     420

gtaaggacac tgcagggctt cctaagccgc caggagggcc ccatctgcct tgtggcccac     480

aatggcttcg attatgactt cccactgctg tgcacggagc tacaacgtct gggtgcccat     540

ctgccccaag acactgtctg cctggacaca ctgcctgcat tgcggggcct ggaccgtgct     600

cacagccacg gcaccagggc tcaaggccgc aaaagctaca gcctggccag tctcttccac     660

cgctacttcc aggctgaacc cagtgctgcc cattcagcag aaggtgatgt gcacaccctg     720

cttctgatct tcctgcatcg tgctcctgag ctgctcgcct gggcagatga gcaggcccgc     780

agctgggctc atattgagcc catgtacgtg ccacctgatg gtccaagcct cgaagcctga     840


<210>  16
<211>  837
<212>  DNA
<213>  Artificial Sequence

<220>
<223>  P4-TREX2

<400>  16
agcccggaag ataaaattgc tcagctgaaa caaaaaatcc aagcgctgaa acaggaaaac      60

cagcagctgg aagaggaaaa cgccgcactg gaatatggtg gcggctctgg cggcggctcc     120

ggaggctctt ctgagccacc tcgggctgag acctttgtat tcctggacct agaagccact     180

gggctcccaa acatggaccc tgagattgca gagatatccc tttttgctgt tcaccgctct     240

tccctggaga acccagaacg ggatgattct ggttccttgg tgctgccccg tgttctggac     300

aagctcacac tgtgcatgtg cccggagcgc ccctttactg ccaaggccag tgagattact     360

ggtttgagca gcgaaagcct gatgcactgc gggaaggctg gtttcaatgg cgctgtggta     420

aggacactgc agggcttcct aagccgccag gagggcccca tctgccttgt ggcccacaat     480

ggcttcgatt atgacttccc actgctgtgc acggagctac aacgtctggg tgcccatctg     540

ccccaagaca ctgtctgcct ggacacactg cctgcattgc ggggcctgga ccgtgctcac 600

agccacggca ccagggctca aggccgcaaa agctacagcc tggccagtct cttccaccgc 660

tacttccagg ctgaacccag tgctgcccat tcagcagaag gtgatgtgca caccctgctt 720

ctgatcttcc tgcatcgtgc tcctgagctg ctcgcctggg cagatgagca ggcccgcagc 780

tgggctcata ttgagcccat gtacgtgcca cctgatggtc caagcctcga agcctga 837

<210> 17
<211> 2668
<212> DNA
<213> Artificial Sequence

<220>
<223> P3-EXO1

<400> 17
tccccggaag atgagatcca gcaactggaa gaagaaatcg ctcagctgga acagaaaaac 60

gcagcgctga aagagaaaaa ccaggcgctg aaatacggtg gcggctctgg cggcggctcc 120

ggaggctctg cgatcgccat ggggatacag ggattgctac aatttatcaa agaagcttca 180

gaacccatcc atgtgaggaa gtataaaggg caggtagtag ctgtggatac atattgctgg 240

cttcacaaag gagctattgc ttgtgctgaa aaactagcca aaggtgaacc tactgatagg 300

tatgtaggat tttgtatgaa atttgtaaat atgttactat ctcatgggat caagcctatt 360

ctcgtatttg atggatgtac tttaccttct aaaaaggaag tagagagatc tagaagagaa 420

agacgacaag ccaatcttct taagggaaag caacttcttc gtgaggggaa agtctcggaa 480

gctcgagagt gtttcacccg gtctatcaat atcacacatg ccatggccca caaagtaatt 540

aaagctgccc ggtctcaggg ggtagattgc ctcgtggctc cctatgaagc tgatgcgcag 600

ttggcctatc ttaacaaagc gggaattgtg caagccataa ttacagagga ctcggatctc 660

ctagcttttg gctgtaaaaa ggtaatttta aagatggacc agtttggaaa tggacttgaa 720

attgatcaag ctcggctagg aatgtgcaga cagcttgggg atgtattcac ggaagagaag 780

tttcgttaca tgtgtattct ttcaggttgt gactacctgt catcactgcg tgggattgga 840

ttagcaaagg catgcaaagt cctaagacta gccaataatc cagatatagt aaaggttatc 900

aagaaaattg gacattatct caagatgaat atcacggtac cagaggatta catcaacggg 960

tttattcggg ccaacaatac cttcctctat cagctagttt ttgatcccat caaaaggaaa 1020

cttattcctc tgaacgccta tgaagatgat gttgatcctg aaacactaag ctacgctggg 1080

caatatgttg atgattccat agctcttcaa atagcacttg gaaataaaga tataaatact 1140

tttgaacaga tcgatgacta caatccagac actgctatgc ctgcccattc aagaagtcat 1200

agttgggatg acaaaacatg tcaaaagtca gctaatgtta gcagcatttg gcataggaat 1260

tactctccca gaccagagtc gggtactgtt tcagatgccc cacaattgaa ggaaaatcca 1320

agtactgtgg gagtggaacg agtgattagt actaaagggt taaatctccc aaggaaatca 1380

tccattgtga aaagaccaag aagtgcagag ctgtcagaag atgacctgtt gagtcagtat 1440

tctctttcat ttacgaagaa gaccaagaaa aatagctctg aaggcaataa atcattgagc 1500

ttttctgaag tgtttgtgcc tgacctggta aatggaccta ctaacaaaaa gagtgtaagc 1560

actccaccta ggacgagaaa taaatttgca acatttttac aaaggaaaaa tgaagaaagt 1620

ggtgcagttg tggttccagg gaccagaagc aggttttttt gcagttcaga ttctactgac 1680

tgtgtatcaa acaaagtgag catccagcct ctggatgaaa ctgctgtcac agataaagag 1740

aacaatctgc atgaatcaga gtatggagac caagaaggca agagactggt tgacacagat 1800

gtagcacgta attcaagtga tgacattccg aataatcata ttccaggtga tcatattcca 1860

gacaaggcaa cagtgtttac agatgaagag tcctactctt ttgagagcag caaatttaca 1920

aggaccattt caccacccac tttgggaaca ctaagaagtt gttttagttg gtctggaggt 1980

cttggagatt tttcaagaac gccgagcccc tctccaagca cagcattgca gcagttccga 2040

agaaagagcg attcccccac ctctttgcct gagaataata tgtctgatgt gtcgcagtta 2100

aagagcgagg agtccagtga cgatgagtct catcccttac gagaaggggc atgttcttca 2160

cagtcccagg aaagtggaga attctcactg cagagttcaa atgcatcaaa gctttctcag 2220

tgctctagta aggactctga ttcagaggaa tctgattgca atattaagtt acttgacagt 2280

caaagtgacc agacctccaa gctatgttta tctcatttct caaaaaaaga cacacctcta 2340

aggaacaagg ttcctgggct atataagtcc agttctgcag actctctttc tacaaccaag 2400

atcaaacctc taggacctgc cagagccagt gggctgagca agaagccggc aagcatccag 2460

aagagaaagc atcataatgc cgagaacaag ccggggttac agatcaaact caatgagctc 2520

tggaaaaact ttggatttaa aaaagattct gaaaagcttc ctccttgtaa gaaacccctg 2580

tccccagtca gagataacat ccaactaact ccagaagcgg aagaggatat atttaacaaa 2640

cctgaatgtg gccgtgttca aagagcaa 2668

<210> 18
<211> 2668
<212> DNA
<213> Artificial Sequence

<220>
<223> P4-EXO1

<400> 18
agcccggaag ataaaattgc tcagctgaaa caaaaaatcc aagcgctgaa acaggaaaac 60

cagcagctgg aagaggaaaa cgccgcactg gaatatggtg gcggctctgg cggcggctcc 120

ggaggctctg cgatcgccat ggggatacag ggattgctac aatttatcaa agaagcttca 180

gaacccatcc atgtgaggaa gtataaaggg caggtagtag ctgtggatac atattgctgg 240

cttcacaaag gagctattgc ttgtgctgaa aaactagcca aaggtgaacc tactgatagg 300

tatgtaggat tttgtatgaa atttgtaaat atgttactat ctcatgggat caagcctatt 360

ctcgtatttg atggatgtac tttaccttct aaaaaggaag tagagagatc tagaagagaa 420

agacgacaag ccaatcttct taagggaaag caacttcttc gtgaggggaa agtctcggaa 480

gctcgagagt gtttcacccg gtctatcaat atcacacatg ccatggccca caaagtaatt 540

aaagctgccc ggtctcaggg ggtagattgc ctcgtggctc cctatgaagc tgatgcgcag 600

ttggcctatc ttaacaaagc gggaattgtg caagccataa ttacagagga ctcggatctc 660

ctagcttttg gctgtaaaaa ggtaatttta aagatggacc agtttggaaa tggacttgaa 720

attgatcaag ctcggctagg aatgtgcaga cagcttgggg atgtattcac ggaagagaag 780

tttcgttaca tgtgtattct ttcaggttgt gactacctgt catcactgcg tgggattgga 840

ttagcaaagg catgcaaagt cctaagacta gccaataatc cagatatagt aaaggttatc 900

aagaaaattg gacattatct caagatgaat atcacggtac cagaggatta catcaacggg 960

tttattcggg ccaacaatac cttcctctat cagctagttt ttgatcccat caaaaggaaa 1020

cttattcctc tgaacgccta tgaagatgat gttgatcctg aaacactaag ctacgctggg 1080

caatatgttg atgattccat agctcttcaa atagcacttg gaaataaaga tataaatact 1140

tttgaacaga tcgatgacta caatccagac actgctatgc ctgcccattc aagaagtcat 1200

agttgggatg acaaaacatg tcaaaagtca gctaatgtta gcagcatttg gcataggaat 1260

tactctccca gaccagagtc gggtactgtt tcagatgccc cacaattgaa ggaaaatcca 1320

agtactgtgg gagtggaacg agtgattagt actaaagggt taaatctccc aaggaaatca 1380

tccattgtga aaagaccaag aagtgcagag ctgtcagaag atgacctgtt gagtcagtat 1440

tctctttcat ttacgaagaa gaccaagaaa aatagctctg aaggcaataa atcattgagc 1500

ttttctgaag tgtttgtgcc tgacctggta aatggaccta ctaacaaaaa gagtgtaagc 1560

actccaccta ggacgagaaa taaatttgca acatttttac aaaggaaaaa tgaagaaagt 1620

ggtgcagttg tggttccagg gaccagaagc aggttttttt gcagttcaga ttctactgac 1680

tgtgtatcaa acaaagtgag catccagcct ctggatgaaa ctgctgtcac agataaagag 1740

aacaatctgc atgaatcaga gtatggagac caagaaggca agagactggt tgacacagat 1800

gtagcacgta attcaagtga tgacattccg aataatcata ttccaggtga tcatattcca 1860

gacaaggcaa cagtgtttac agatgaagag tcctactctt ttgagagcag caaatttaca 1920

aggaccattt caccacccac tttgggaaca ctaagaagtt gttttagttg gtctggaggt 1980

cttggagatt tttcaagaac gccgagcccc tctccaagca cagcattgca gcagttccga 2040

agaaagagcg attcccccac ctctttgcct gagaataata tgtctgatgt gtcgcagtta 2100

aagagcgagg agtccagtga cgatgagtct catcccttac gagaaggggc atgttcttca 2160

cagtcccagg aaagtggaga attctcactg cagagttcaa atgcatcaaa gctttctcag 2220

tgctctagta aggactctga ttcagaggaa tctgattgca atattaagtt acttgacagt 2280

caaagtgacc agacctccaa gctatgttta tctcatttct caaaaaaaga cacacctcta 2340

aggaacaagg ttcctgggct atataagtcc agttctgcag actctctttc tacaaccaag 2400

atcaaacctc taggacctgc cagagccagt gggctgagca agaagccggc aagcatccag 2460

aagagaaagc atcataatgc cgagaacaag ccggggttac agatcaaact caatgagctc 2520

tggaaaaact ttggatttaa aaaagattct gaaaagcttc ctccttgtaa gaaacccctg 2580

tccccagtca gagataacat ccaactaact ccagaagcgg aagaggatat atttaacaaa 2640

cctgaatgtg gccgtgttca aagagcaa 2668